# EUROPEAN PATENT APPLICATION

(11) **EP 4 582 413 A1**
(43) Date of publication of application: **09.07.2025**
(21) Application number: 23860540.6
(22) Date of filing: 01.09.2023
(51) Int. Cl.: C07D 211/84, A01N 43/40, A01N 43/54, A01N 43/56, A01N 43/58, A01N 43/60, A01N 43/836, A01N 53/12, A01P 5/00, A01P 7/04, A61K 31/4418, A61K 31/4427, A61K 31/443, A61K 31/4436, A61K 31/4439, A61K 31/444, A61K 31/497, A61K 31/501, A61K 31/506, A61P 33/00, A61P 33/02

(54) **ARYLTETRAHYDROPYRIDINE COMPOUND AND PEST CONTROL AGENT**

(30) Priority: 01.09.2022 JP 2022139441; 21.11.2022 JP 2022185884; 11.01.2023 JP 2023002272; 24.03.2023 JP 2023049089; 30.06.2023 JP 2023108955
(71) Applicant: Nissan Chemical Corporation, Tokyo 103-6119 (JP)
(72) Inventor: TSUJI, Keisuke, Funabashi-shi, Chiba 274-8507 (JP); KOBAYASHI, Chisato, Funabashi-shi, Chiba 274-8507 (JP); TAJIMA, Yuki, Funabashi-shi, Chiba 274-8507 (JP); MIZOYAMA, Yasunori, Shiraoka-shi, Saitama 349-0294 (JP)
(74) Representative: Hoffmann Eitle
(86) International application number: PCT/JP2023/032082
(87) International publication number: WO 2024/048778

(57) **Abstract**

The present invention relates to an aryltetrahydropyridine compound or a salt thereof, the aryltetrahydropyridine compound represented by a formula (1): 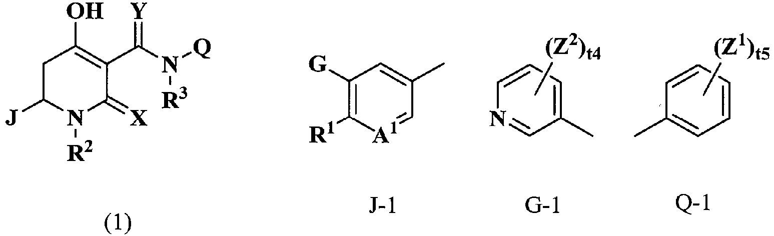 wherein Q represents, for example, Q-1, J represents, for example, J-1, G represents, for example, G-1, R¹ represents, for example, halo (C₁-C₆) alkyl, R² represents, for example, a hydrogen atom, R³ represents, for example, a hydrogen atom, X represents, for example, an oxygen atom, Y represents, for example, an oxygen atom, and A¹ represents, for example, a nitrogen atom.

## Description

### Technical Field

The present invention relates to novel aryltetrahydropyridine compounds and salts thereof, production intermediates therefor, and a pesticide, an agrochemical, a control agent, an insecticide or acaricide, an agent for seed treatment, and an agent for soil treatment each containing such a compound and/or a salt thereof as an active ingredient.

### Background Art

A certain class of aryltetrahydropyridine compounds is known to exhibit pesticidal activity (for example, Patent Literatures 1, 2).

### Document List

### Patent Literatures

Patent Literature 1: International Publication No. WO 2021/261562
Patent Literature 2: International Publication No. WO 2023/127806

### Summary of Invention

### Technical Problem

However, use of chemicals for many years may cause the outbreak of noxious insects and pests that have acquired resistance to existing insecticides. Accordingly, development of a novel chemical having a superior pesticidal effect is always demanded.

### Solution to Problem

The present inventors have diligently studied for solving the problem to find that novel aryltetrahydropyridine compounds according to the present invention, which are represented by a formula (1) shown below, have superior pesticidal activity, in particular, superior activity for killing insects and killing acarians, and completed the present invention.

Specifically, the present invention relates to an aryltetrahydropyridine compound represented by the following formula (1) or a salt thereof.
A formula (1): 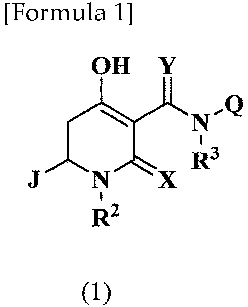 wherein
Q represents Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, Q-37, Q-38, Q-39, Q-40, Q-41, Q-42, Q-43, Q-44, Q-45, Q-46, Q-47, Q-48, Q-49, Q-50, Q-51, Q-52, Q-53, Q-54, Q-55 or Q-56, 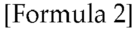 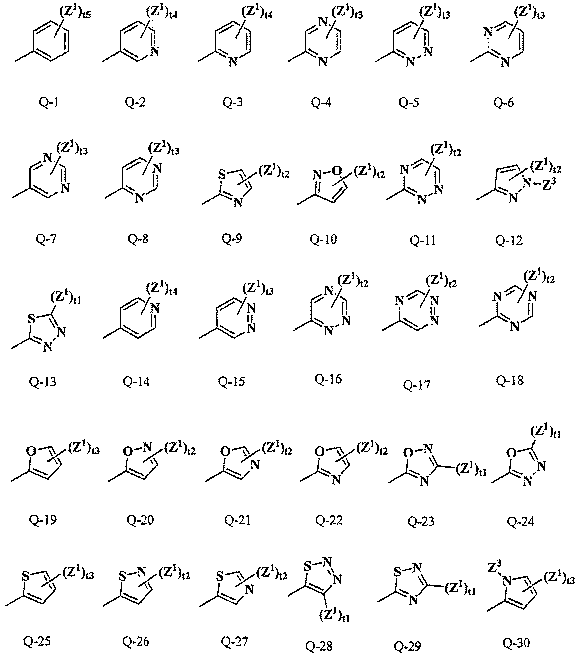 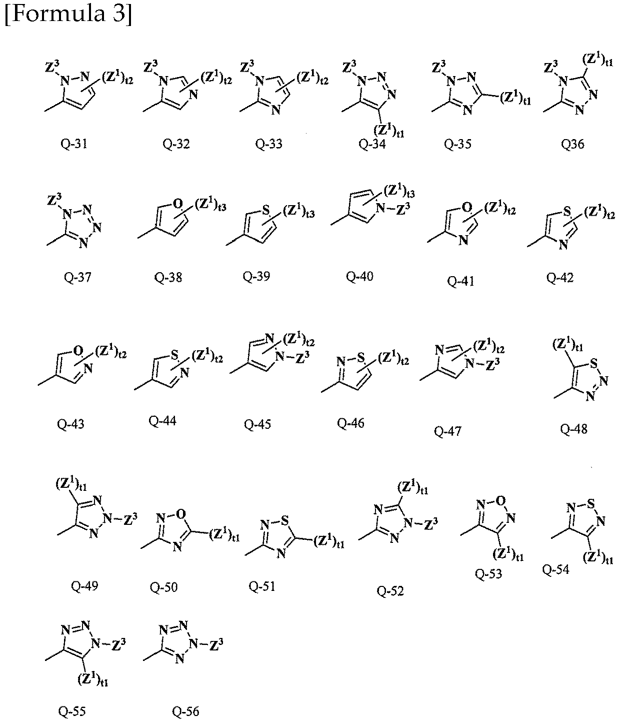
Z¹ represents a halogen atom, C₁-C₆ alkyl, C₂-C₆ alkenyl, C₂-C₆ alkynyl, halo (C₁-C₆) alkyl, halo (C₂-C₆) alkenyl, halo (C₂-C₆) alkynyl, C₁-C₆ alkoxy, halo (C₁-C₆) alkoxy, C₂-C₆ alkoxy (C₁-C₆) alkoxy, C₂-C₆ alkenyloxy, halo (C₂-C₆) alkenyloxy, C₂-C₆ alkynyloxy, halo (C₂-C₆) alkynyloxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, C₂-C₆ alkenylthio, C₂-C₆ alkenylsulfinyl, C₂-C₆ alkenylsulfonyl, halo (C₂-C₆) alkenylthio, halo (C₂-C₆) alkenylsulfinyl, halo (C₂-C₆) alkenylsulfonyl, C₂-C₆ alkynylthio, C₂-C₆ alkynylsulfinyl, C₂-C₆ alkynylsulfonyl, halo (C₂-C₆) alkynylthio, halo (C₂-C₆) alkynylsulfinyl, halo (C₂-C₆) alkynylsulfonyl, -NH₂, C₁-C₆ alkylamino, di (C₁-C₆) alkylamino, halo (C₁-C₆) alkylamino, dihalo (C₁-C₆) alkylamino, formyl, carboxy, C₁-C₆ alkylcarbonyl, halo (C₁-C₆) alkylcarbonyl, C₁-C₆ alkoxycarbonyl, halo (C₁-C₆) alkoxycarbonyl, C₁-C₆ alkylaminocarbonyl, halo (C₁-C₆) alkylaminocarbonyl, di (C₁-C₆) alkylaminocarbonyl, hydroxy, C₁-C₆ alkylcarbonyloxy, C₁-C₆ alkoxycarbonyloxy, C₁-C₆ alkylsulfonyloxy, halo (C₁-C₆) alkylsulfonyloxy, -SO₃H, C₁-C₆ alkylaminosulfonyl, di (C₁-C₆) alkylaminosulfonyl, -C(=NOR¹²)R¹³, cyano or nitro,
R¹² represents a hydrogen atom or C₁-C₆ alkyl,
R¹³ represents a hydrogen atom or C₁-C₆ alkyl,
Z³ represents a hydrogen atom or C₁-C₆ alkyl,
J represents J-1 or J-2, 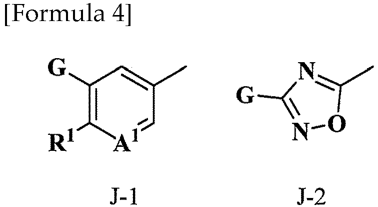
A¹ represents a nitrogen atom or CH,
G represents G-1, G-2, G-3, G-4, G-5, G-6, G-7, G-8, G-9, G-10, G-11, G-12, G-13, G-14, G-15, G-16, G-17, G-18, G-19, G-20, G-21, G-22, G-23, G-24, G-25, G-26, G-27, G-28, G-29, G-30, G-31, G-32, G-33, G-34, G-35, G-36, G-37, G-38, G-39, G-40, G-41, G-42, G-43, G-44, G-45, G-46, G-47, G-48, G-49, G-50, G-51, G-52, G-53, G-54, G-55, G-56, G-57, G-58, G-59, G-60 or G-61, 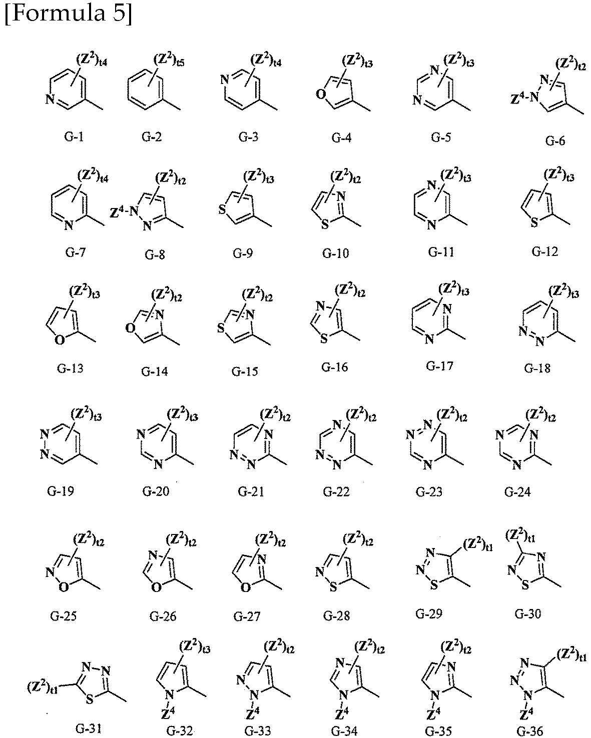 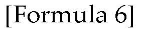 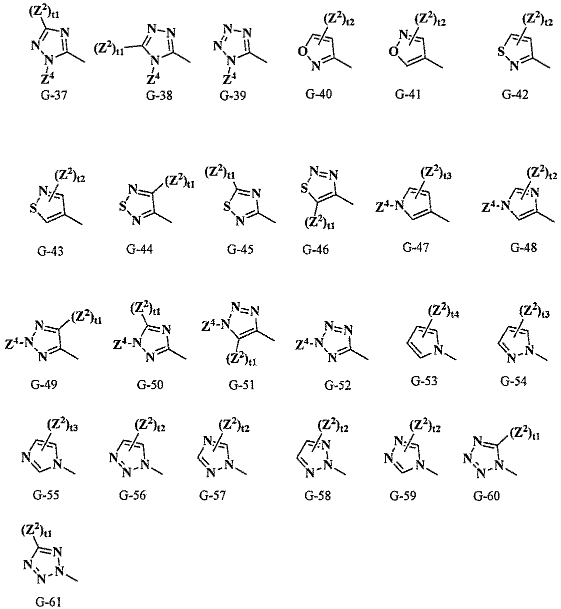
Z² represents a halogen atom, C₁-C₆ alkyl, C₂-C₆ alkenyl, C₂-C₆ alkynyl, halo (C₁-C₆) alkyl, halo (C₂-C₆) alkenyl, halo (C₂-C₆) alkynyl, C₁-C₆ alkoxy, halo (C₁-C₆) alkoxy, C₁-C₆ alkoxy (C₁-C₆) alkoxy, C₂-C₆ alkenyloxy, halo (C₂-C₆) alkenyloxy, C₂-C₆ alkynyloxy, halo (C₂-C₆) alkynyloxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, C₂-C₆ alkenylthio, C₂-C₆ alkenylsulfinyl, C₂-C₆ alkenylsulfonyl, halo (C₂-C₆) alkenylthio, halo (C₂-C₆) alkenylsulfinyl, halo (C₂-C₆) alkenylsulfonyl, C₂-C₆ alkynylthio, C₂-C₆ alkynylsulfinyl, C₂-C₆ alkynylsulfonyl, halo (C₂-C₆) alkynylthio, halo (C₂-C₆) alkynylsulfinyl, halo (C₂-C₆) alkynylsulfonyl, -NH₂, C₁-C₆ alkylamino, di (C₁-C₆) alkylamino, halo (C₁-C₆) alkylamino, dihalo (C₁-C₆) alkylamino, formyl, carboxy, C₁-C₆ alkylcarbonyl, halo (C₁-C₆) alkylcarbonyl, C₁-C₆ alkoxycarbonyl, halo (C₁-C₆) alkoxycarbonyl, C₁-C₆ alkylaminocarbonyl, halo (C₁-C₆) alkylaminocarbonyl, di (C₁-C₆) alkylaminocarbonyl, hydroxy, C₁-C₆ alkylcarbonyloxy, C₁-C₆ alkoxycarbonyloxy, C₁-C₆ alkylsulfonyloxy, halo (C₁-C₆) alkylsulfonyloxy, -SO₃H, C₁-C₆ alkylaminosulfonyl, di (C₁-C₆) alkylaminosulfonyl, -C(=NOR¹⁴)R¹⁵, nitro or cyano,
R¹⁴ represents a hydrogen atom or C₁-C₆ alkyl,
R¹⁵ represents a hydrogen atom or C₁-C₆ alkyl,
Z⁴ represents C₁-C₆ alkyl,
R¹ represents a hydrogen atom, a halogen atom, halo (C₁-C₆) alkyl, C₁-C₆ alkyl or cyano,
R² represents a hydrogen atom, C₁-C₆ alkyl, halo (C₁-C₆) alkyl, C₂-C₆ alkenyl, C₂-C₆ alkynyl, C₃-C₆ cycloalkyl, -C(O)R⁴, -C(O)OR⁵, D-1, D-2 or D-3, 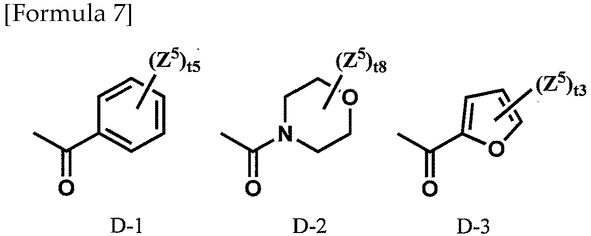
R⁴ represents C₁-C₆ alkyl, C₂-C₆ alkenyl, C₂-C₆ alkynyl, C₃-C₆ cycloalkyl, C₁-C₆ alkylcarbonyl, di (C₁-C₆) alkylamino or (C₁-C₆) alkyl substituted with R⁶,
R⁶ represents a halogen atom, C₁-C₆ alkoxy, cyano, nitro, phenyl or phenyl substituted with R⁸,
Z⁵ represents a halogen atom, C₁-C₆ alkyl, C₁-C₆ alkoxy, cyano or nitro,
R⁸ represents a halogen atom, C₁-C₆ alkyl, C₁-C₆ alkoxy, cyano or nitro,
R⁵ represents C₁-C₆ alkyl, C₂-C₆ alkenyl, C₂-C₆ alkynyl, C₃-C₆ cycloalkyl, (C₁-C₆) alkyl substituted with R⁹, phenyl or phenyl substituted with R¹⁰,
R⁹ represents a halogen atom, C₁-C₆ alkoxy, cyano, nitro, phenyl or phenyl substituted with R¹¹,
R¹⁰ represents a halogen atom, C₁-C₆ alkyl, C₁-C₆ alkoxy, cyano or nitro,
R¹¹ represents a halogen atom, C₁-C₆ alkyl, C₁-C₆ alkoxy, cyano or nitro,
R³ represents a hydrogen atom, C₁-C₆ alkyl, halo (C₁-C₆) alkyl, C₂-C₆ alkenyl, C₂-C₆ alkynyl, C₃-C₆ cycloalkyl or C₁-C₆ alkylcarbonyl,
X represents an oxygen atom or a sulfur atom,
Y represents an oxygen atom or a sulfur atom,
t1 represents an integer of 0 or 1,
t2 represents an integer of 0, 1 or 2,
t3 represents an integer of 0, 1, 2 or 3,
t4 represents an integer of 0, 1, 2, 3 or 4,
t5 represents an integer of 0, 1, 2, 3, 4 or 5, and
t8 represents an integer of 0, 1, 2, 3 or 4.

### Effects of Invention

Compounds according to the present invention, which are represented by the formula (1), exert superior insecticidal and acaricidal activity against many noxious insects in agriculture, spider mites, and internal or external parasites in mammals or birds. Accordingly, the present invention can provide a novel aryltetrahydropyridine compound or a salt thereof useful as a pesticide, in particular, as an insecticide.

### Description of Embodiments

For compounds according to the present invention (hereinafter, also referred to as "the presently invented compound"), which are represented by the formula (1), the existence of tautomers represented by a formula shown below are expected, and the definition of the presently invented compound represented by the formula (1) includes all of the tautomers and mixtures containing the tautomers at any ratio.

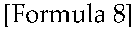

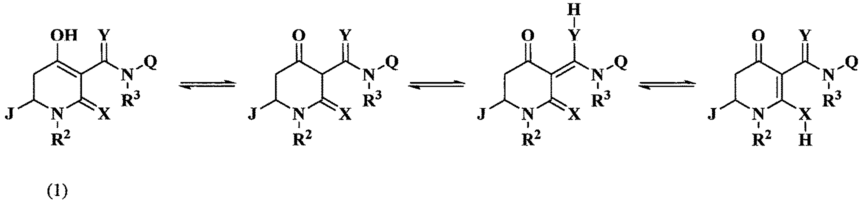

There may be geometric isomers of E-form and Z-form for the presently invented compound for some kinds of substituent, and the definition of the presently invented compound includes all of the E-form and the Z-form, and mixtures containing the E-form and the Z-form at any ratio.

There may exist optically active forms due to the presence of one or two or more asymmetric carbon atoms or asymmetric sulfur atoms for the presently invented compound, and the definition of the presently invented compound includes all of the optically active forms and racemates.

There may exist tautomers for the presently invented compound for some kinds of substituent, and the definition of the presently invented compound includes all of the tautomers and mixtures containing the tautomers at any ratio.

There may exist one or two or more rotational isomers for the presently invented compound because of limited bond rotation caused by steric hindrance between substituents, and the definition of the presently invented compound includes all of the rotational isomers and mixtures containing diastereomers at any ratio.

Salts that can be formed from the presently invented compound according to a conventional method include: salts of hydrohalic acid such as hydrofluoric acid, hydrochloric acid, hydrobromic acid, and hydroiodic acid; salts of inorganic acid such as nitric acid, sulfuric acid, phosphoric acid, chloric acid, and perchloric acid; salts of sulfonic acid such as methanesulfonic acid, ethanesulfonic acid, trifluoromethanesulfonic acid, benzenesulfonic acid, and p-toluenesulfonic acid; salts of carboxylic acid such as formic acid, acetic acid, propionic acid, trifluoroacetic acid, fumaric acid, tartaric acid, oxalic acid, maleic acid, malic acid, succinic acid, benzoic acid, mandelic acid, ascorbic acid, lactic acid, gluconic acid, and citric acid; salts of amino acid such as glutamic acid and aspartic acid; salts of alkali metal such as lithium, sodium, and potassium; salts of alkaline earth metal such as calcium, barium, and magnesium; a salt of aluminum; and quaternary ammonium salts such as a tetramethylammonium salt, a tetrabutylammonium salt, and a benzyltrimethylammonium salt.

Next, specific examples of the substituents shown in the present specification will be shown in the following. There, n-, i-, s-, and tert- mean normal, iso, secondary, and tertiary, respectively.

Examples of "halogen atom" in the present specification include a fluorine atom, a chlorine atom, bromine atom, and an iodine atom. The term "halo" in the present specification indicates such a halogen atom.

The expression "Cₐ-C_{b} alkyl" in the present specification indicates a linear or branched hydrocarbon group having a to b carbon atoms, specific examples include methyl, ethyl, n-propyl, i-propyl, n-butyl, i-butyl, s-butyl, tert-butyl, n-pentyl, 1,1-dimethylpropyl, and n-hexyl, and appropriate one according to the specified numbers of carbon atoms is selected therefrom.

The expression "halo (Cₐ-C_{b}) alkyl" in the present specification indicates a linear or branched hydrocarbon group having a to b carbon atoms in which hydrogen atoms bonding to carbon atoms are arbitrarily substituted by a halogen atom, wherein if the alkyl group is substituted with two or more halogen atoms, the halogen atoms may be same or different from each other. Specific examples include fluoromethyl, chloromethyl, bromomethyl, iodomethyl, difluoromethyl, dichloromethyl, trifluoromethyl, chlorodifluoromethyl, trichloromethyl, bromodifluoromethyl, 1-fluoroethyl, 2-fluoroethyl, 2-chloroethyl, 2-bromoethyl, 2,2-difluoroethyl, 2,2,2-trifluoroethyl, 2-chloro-2,2-difluoroethyl, 2,2,2-trichloroethyl, 2-bromo-2,2-difluoroethyl, 1,1,2,2-tetrafluoroethyl, 2-chloro-1,1,2-trifluoroethyl, 2-chloro-1,1,2,2-tetrafluoroethyl, pentafluoroethyl, 2,2-difluoropropyl, 3,3,3-trifluoropropyl, 3-bromo-3,3-difluoropropyl, 2,2,3,3-tetrafluoropropyl, 2,2,3,3,3-pentafluoropropyl, 1,1,2,3,3,3-hexafluoropropyl, heptafluoropropyl, 2,2,2-trifluoro-1-(methyl)ethyl, 2,2,2-trifluoro-1-(trifluoromethyl)ethyl, 1,2,2,2-tetrafluoro-1-(trifluoromethyl)ethyl, 2,2,3,4,4,4-hexafluorobutyl, 2,2,3,3,4,4,4-heptafluorobutyl, and nonafluorobutyl, and appropriate one according to the specified numbers of carbon atoms is selected therefrom.

The expression "Cₐ-C_{b} alkenyl" in the present specification indicates a linear or branched unsaturated hydrocarbon group having a to b carbon atoms and having one or two or more double bonds in the molecule, specific examples include vinyl, 1-propenyl, 2-propenyl, 1-methylethenyl, 2-butenyl, 2-methyl-2-propenyl, 3-methyl-2-butenyl, and 1,1-dimethyl-2-propenyl, and appropriate one according to the specified numbers of carbon atoms is selected therefrom.

The expression "halo (Cₐ-C_{b}) alkenyl" in the present specification indicates a linear or branched unsaturated hydrocarbon group having a to b carbon atoms in which hydrogen atoms bonding to carbon atoms are arbitrarily substituted by a halogen atom, and having one or two or more double bonds in the molecule; wherein if the alkenyl group is substituted with two or more halogen atoms, the halogen atoms may be same or different from each other. Specific examples include 2,2-dichlorovinyl, 2-fluoro-2-propenyl, 2-chloro-2-propenyl, 3-chloro-2-propenyl, 2-bromo-2-propenyl, 3,3-difluoro-2-propenyl, 2,3-dichloro-2-propenyl, 3,3-dichloro-2-propenyl, 2,3,3-trifluoro-2-propenyl, 2,3,3-trichloro-2-propenyl, 1-(trifluoromethyl)ethenyl, 4,4-difluoro-3-butenyl, 3,4,4-trifluoro-3-butenyl, and 3-chloro-4,4,4-trifluoro-2-butenyl, and appropriate one according to the specified numbers of carbon atoms is selected therefrom.

The expression "Cₐ-C_{b} alkynyl" in the present specification indicates a linear or branched unsaturated hydrocarbon group having a to b carbon atoms and having one or two or more triple bonds, specific examples include ethynyl, propargyl, 2-butynyl, 3-butynyl, 1-pentynyl, and 1-hexynyl, and appropriate one according to the specified numbers of carbon atoms is selected therefrom.

The expression "halo (Cₐ-C_{b}) alkynyl" in the present specification indicates a linear or branched unsaturated hydrocarbon group having a to b carbon atoms in which hydrogen atoms bonding to carbon atoms are arbitrarily substituted by a halogen atom, and having one or two or more triple bonds in the molecule; wherein if the alkynyl group is substituted with two or more halogen atoms, the halogen atoms may be same or different from each other. Specific examples include 2-chloroethynyl, 2-bromoethynyl, 2-iodoethynyl, 3-chloro-2-propynyl, 3-bromo-2-propynyl, and 3-iodo-2-propynyl, and appropriate one according to the specified numbers of carbon atoms is selected therefrom.

The expression "Cₐ-C_{b} alkoxy" in the present specification indicates an alkyl-O- group, wherein the alkyl has a to b carbon atoms and has the above meaning, specific examples include methoxy, ethoxy, n-propyloxy, i-propyloxy, n-butyloxy, i-butyloxy, s-butyloxy, tert-butyloxy, and 2-ethylhexyloxy, and appropriate one according to the specified numbers of carbon atoms is selected therefrom.

The expression "halo (Cₐ-C_{b}) alkoxy" in the present specification indicates a haloalkyl-O- group, wherein the haloalkyl has a to b carbon atoms and has the above meaning, specific examples include difluoromethoxy, trifluoromethoxy, chlorodifluoromethoxy, bromodifluoromethoxy, 2-fluoroethoxy, 2-chloroethoxy, 2,2,2-trifluoroethoxy, 1,1,2,2,-tetrafluoroethoxy, 2-chloro-1,1,2-trifluoroethoxy, and 1,1,2,3,3,3-hexafluoropropyloxy, and appropriate one according to the specified numbers of carbon atoms is selected therefrom.

The expression "Cₐ-C_{b} alkoxy (C_{d}-Cₑ) alkoxy" in the present specification indicates an alkoxy group having d to e carbon atoms, wherein the alkoxy group has the above meaning, in which hydrogen atoms bonding to carbon atoms are arbitrarily substituted by any Cₐ-C_{b} alkoxy group having the above meaning, specific examples include methoxymethoxy and 2-methoxyethoxy, and appropriate one according to the specified numbers of carbon atoms is selected therefrom.

The expression "Cₐ-C_{b} alkenyloxy" in the present specification indicates an alkenyl-O- group, wherein the alkenyl has a to b carbon atoms and has the above meaning, specific examples include 2-propenyloxy, 2-butenyloxy, 2-methyl-2-propenyloxy, and 3-methyl-2-butenyloxy, and appropriate one according to the specified numbers of carbon atoms is selected therefrom.

The expression "halo (Cₐ-C_{b}) alkenyloxy" in the present specification indicates a haloalkenyl-O- group, wherein the haloalkenyl has a to b carbon atoms and has the above meaning, specific examples include 3,3-difluoroallyloxy and 3,3-dichloroallyloxy, and appropriate one according to the specified numbers of carbon atoms is selected therefrom.

The expression "Cₐ-C_{b} alkynyloxy" in the present specification indicates an alkynyl-O- group, wherein the alkynyl has a to b carbon atoms and has the above meaning, specific examples include ethynyloxy, propargyloxy, 2-butynyloxy, 1-pentynyloxy, and 1-hexynyloxy, and appropriate one according to the specified numbers of carbon atoms is selected therefrom.

The expression "halo (Cₐ-C_{b}) alkynyloxy" in the present specification indicates a haloalkynyl-O- group, wherein the haloalkynyl has a to b carbon atoms and has the above meaning, specific examples include 3-chloro-2-propynyloxy, 3-bromo-2-propynyloxy, and 3-iodo-2-propynyloxy, and appropriate one according to the specified numbers of carbon atoms is selected therefrom.

The expression "Cₐ-C_{b} alkylthio" in the present specification indicates an alkyl-S- group, wherein the alkyl has a to b carbon atoms and has the above meaning, specific examples include methylthio, ethylthio, n-propylthio, i-propylthio, n-butylthio, i-butylthio, s-butylthio, and tert-butylthio, and appropriate one according to the specified numbers of carbon atoms is selected therefrom.

The expression "halo (Cₐ-C_{b}) alkylthio" in the present specification indicates a haloalkyl-S- group, wherein the haloalkyl has a to b carbon atoms and has the above meaning, specific examples include difluoromethylthio, trifluoromethylthio, chlorodifluoromethylthio, bromodifluoromethylthio, 2,2,2-trifluoroethylthio, 1,1,2,2-tetrafluoroethylthio, 2-chloro-1,1,2-trifluoroethylthio, pentafluoroethylthio, 1,1,2,3,3,3-hexafluoropropylthio, heptafluoropropylthio, 1,2,2,2-tetrafluoro-1-(trifluoromethyl)ethylthio, and nonafluorobutylthio, and appropriate one according to the specified numbers of carbon atoms is selected therefrom.

The expression "Cₐ-C_{b} alkenylthio" in the present specification indicates an alkenyl-S- group, wherein the alkenyl has a to b carbon atoms and has the above meaning, specific examples include 2-propenylthio, 2-butenylthio, 2-methyl-2-propenylthio, and 3-methyl-2-butenylthio, and appropriate one according to the specified numbers of carbon atoms is selected therefrom.

The expression "halo (Cₐ-C_{b}) alkenylthio" in the present specification indicates a haloalkenyl-S- group, wherein the haloalkenyl has a to b carbon atoms and has the above meaning, specific examples include 2-fluoro-2-propenylthio, 2-chloro-2-propenylthio, 3,3-difluoro-2-propenylthio, 3,3-dichloro-2-propenylthio, 2,3,3-trifluoro-2-propenylthio, 4,4-difluoro-3-butenylthio, and 3,4,4-trifluoro-3-butenylthio, and appropriate one according to the specified numbers of carbon atoms is selected therefrom.

The expression "Cₐ-C_{b} alkynylthio" in the present specification indicates an alkynyl-S- group, wherein the alkynyl has a to b carbon atoms and has the above meaning, specific examples include propynylthio, butynylthio, pentynylthio, and hexynylthio, and appropriate one according to the specified numbers of carbon atoms is selected therefrom.

The expression "halo (Cₐ-C_{b}) alkynylthio" in the present specification indicates a haloalkynyl-S- group, wherein the haloalkynyl has a to b carbon atoms and has the above meaning, specific examples include 3-chloro-2-propynylthio, 3-bromo-2-propynylthio, and 3-iodo-2-propynylthio, and appropriate one according to the specified numbers of carbon atoms is selected therefrom.

The expression "Cₐ-C_{b} alkylsulfinyl" in the present specification indicates an alkyl-S(O)- group, wherein the alkyl has a to b carbon atoms and has the above meaning, specific examples include methylsulfinyl, ethylsulfinyl, n-propylsulfinyl, i-propylsulfinyl, n-butylsulfinyl, i-butylsulfinyl, S-butylsulfinyl, and tert-butylsulfinyl, and appropriate one according to the specified numbers of carbon atoms is selected therefrom.

The expression "halo (Cₐ-C_{b}) alkylsulfinyl" in the present specification indicates a haloalkyl-S(O)- group, wherein the haloalkyl has a to b carbon atoms and has the above meaning, specific examples include difluoromethylsulfinyl, trifluoromethylsulfinyl, chlorodifluoromethylsulfinyl, bromodifluoromethylsulfinyl, 2,2,2-trifluoroethylsulfinyl, 1,2,2,2-tetrafluoro-1-(trifluoromethyl)ethylsulfinyl, and nonafluorobutylsulfinyl, and appropriate one according to the specified numbers of carbon atoms is selected therefrom.

The expression "Cₐ-C_{b} alkenylsulfinyl" in the present specification indicates an alkenyl-S(O)- group, wherein the alkenyl has a to b carbon atoms and has the above meaning, specific examples include 2-propenylsulfinyl, 2-butenylsulfinyl, 2-methyl-2-propenylsulfinyl, and 3-methyl-2-butenylsulfinyl, and appropriate one according to the specified numbers of carbon atoms is selected therefrom.

The expression "halo (Cₐ-C_{b}) alkenylsulfinyl" in the present specification indicates a haloalkenyl-S(O)- group, wherein the haloalkenyl has a to b carbon atoms and has the above meaning, specific examples include 2-fluoro-2-propenylsulfinyl, 2-chloro-2-propenylsulfinyl, 3,3-difluoro-2-propenylsulfinyl, 3,3-dichloro-2-propenylsulfinyl, 4,4-difluoro-3-butenylsulfinyl, and 3,4,4-trifluoro-3-butenylsulfinyl, and appropriate one according to the specified numbers of carbon atoms is selected therefrom.

The expression "Cₐ-C_{b} alkynylsulfinyl" in the present specification indicates an alkynyl-S(O)- group, wherein the alkynyl has a to b carbon atoms and has the above meaning, specific examples include 2-propynylsulfinyl and 2-butynylsulfinyl, and appropriate one according to the specified numbers of carbon atoms is selected therefrom.

The expression "halo (Cₐ-C_{b}) alkynylsulfinyl" in the present specification indicates a haloalkynyl-S(O)- group, wherein the haloalkynyl has a to b carbon atoms and has the above meaning, specific examples include 3-chloro-2-propynylsulfinyl, 3-bromo-2-propynylsulfinyl, and 3-iodo-2-propynylsulfinyl, and appropriate one according to the specified numbers of carbon atoms is selected therefrom.

The expression "Cₐ-C_{b} alkylsulfonyl" in the present specification indicates an alkyl-SO₂- group, wherein the alkyl has a to b carbon atoms and has the above meaning, specific examples include methylsulfonyl, ethylsulfonyl, n-propylsulfonyl, i-propylsulfonyl, n-butylsulfonyl, i-butylsulfonyl, s-butylsulfonyl, and tert-butylsulfonyl, and appropriate one according to the specified numbers of carbon atoms is selected therefrom.

The expression "halo (Cₐ-C_{b}) alkylsulfonyl" in the present specification indicates a haloalkyl-SO₂- group, wherein the haloalkyl has a to b carbon atoms and has the above meaning, specific examples include difluoromethylsulfonyl, trifluoromethylsulfonyl, chlorodifluoromethylsulfonyl, bromodifluoromethylsulfonyl, 2,2,2-trifluoroethylsulfonyl, 1,1,2,2-tetrafluoroethylsulfonyl, and 2-chloro-1,1,2-trifluoroethylsulfonyl, and appropriate one according to the specified numbers of carbon atoms is selected therefrom.

The expression "Cₐ-C_{b} alkenylsulfonyl" in the present specification indicates an alkenyl-SO₂- group, wherein the alkenyl has a to b carbon atoms and has the above meaning, specific examples include 2-propenylsulfonyl, 2-butenylsulfonyl, 2-methyl-2-propenylsulfonyl, and 3-methyl-2-butenylsulfonyl, and appropriate one according to the specified numbers of carbon atoms is selected therefrom.

The expression "halo (Cₐ-C_{b}) alkenylsulfonyl" in the present specification indicates a haloalkenyl-SO₂- group, wherein the haloalkenyl has a to b carbon atoms and has the above meaning, specific examples include 2-fluoro-2-propenylsulfonyl, 2-chloro-2-propenylsulfonyl, 3,3-difluoro-2-propenylsulfonyl, 3,3-dichloro-2-propenylsulfonyl, 4,4-difluoro-3-butenylsulfonyl, and 3,4,4-trifluoro-3-butenylsulfonyl, and appropriate one according to the specified numbers of carbon atoms is selected therefrom.

The expression "Cₐ-C_{b} alkynylsulfonyl" in the present specification indicates an alkynyl-SO₂- group, wherein the alkynyl has a to b carbon atoms and has the above meaning, specific examples include 2-propynylsulfonyl and 2-butynylsulfonyl, and appropriate one according to the specified numbers of carbon atoms is selected therefrom.

The expression "halo (Cₐ-C_{b}) alkynylsulfonyl" in the present specification indicates a haloalkynyl-SO₂- group, wherein the haloalkynyl has a to b carbon atoms and has the above meaning, specific examples include 3-chloro-2-propynylsulfonyl, 3-bromo-2-propynylsulfonyl, and 3-iodo-2-propynylsulfonyl, and appropriate one according to the specified numbers of carbon atoms is selected therefrom.

The expression "Cₐ-C_{b} alkylamino" in the present specification indicates an amino group in which one of the hydrogen atoms is substituted by alkyl having a to b carbon atoms and having the above meaning, specific examples include methylamino, ethylamino, n-propylamino, i-propylamino, n-butylamino, i-butylamino, and tert-butylamino, and appropriate one according to the specified numbers of carbon atoms is selected therefrom.

The expression "halo (Cₐ-C_{b}) alkylamino" in the present specification indicates an amino group in which one of the hydrogen atoms is substituted by haloalkyl having a to b carbon atoms and having the above meaning, specific examples include 2,2,2-trifluoroethylamino, 2-chloro-2,2-difluoroethylamino, and 3,3,3-trifluoropropylamino, and appropriate one according to the specified numbers of carbon atoms is selected therefrom.

The expression "di (Cₐ-C_{b}) alkylamino" in the present specification indicates an amino group in which each of the hydrogen atoms is substituted by alkyl having a to b carbon atoms and having the above meaning, wherein the two alkyl groups may be same or different from each other, specific examples include dimethylamino, ethyl(methyl)amino, diethylamino, n-propyl(methyl)amino, i-propyl(methyl)amino, di(n-propyl)amino, and di(n-butyl)amino, and appropriate one according to the specified numbers of carbon atoms is selected therefrom.

The expression "dihalo (Cₐ-C_{b}) alkylamino" in the present specification indicates an amino group in which each of the hydrogen atoms is substituted by haloalkyl having a to b carbon atoms and having the above meaning, wherein the two haloalkyl groups may be same or different from each other, specific examples include bis(2,2,2-trifluoroethyl)amino, and appropriate one according to the specified numbers of carbon atoms is selected therefrom.

The expression "Cₐ-C_{b} cycloalkyl" in the present specification indicates a cyclic saturated hydrocarbon group having a to b carbon atoms, and the cyclic saturated hydrocarbon group can form a three- to ten-membered single cyclic or composite cyclic structure. Each ring may be arbitrarily substituted with alkyl to accord with the specified numbers of carbon atoms. Specific examples include cyclopropyl, 1-methylcyclopropyl, 2-methylcyclopropyl, 2,2-dimethylcyclopropyl, cyclobutyl, cyclopentyl, and cyclohexyl, and appropriate one according to the specified numbers of carbon atoms is selected therefrom.

The expression "Cₐ-C_{b} alkylcarbonyl" in the present specification indicates an alkyl-C(O)- group, wherein the alkyl has a to b carbon atoms and has the above meaning, specific examples include acetyl, propionyl, butyryl, isobutyryl, valeryl, isovaleryl, 2-methylbutanoyl, pivaloyl, hexanoyl, and heptanoyl, and appropriate one according to the specified numbers of carbon atoms is selected therefrom.

The expression "halo (Cₐ-C_{b}) alkylcarbonyl" in the present specification indicates a haloalkyl-C(O)- group, wherein the haloalkyl has a to b carbon atoms and has the above meaning, specific examples include fluoroacetyl, chloroacetyl, difluoroacetyl, dichloroacetyl, trifluoroacetyl, chlorodifluoroacetyl, bromodifluoroacetyl, trichloroacetyl, pentafluoropropionyl, heptafluorobutanoyl, and 3-chloro-2,2-dimethylpropanoyl, and appropriate one according to the specified numbers of carbon atoms is selected therefrom.

The expression "Cₐ-C_{b} alkoxycarbonyl" in the present specification indicates an alkyl-O-C(O)- group, wherein the alkyl has a to b carbon atoms and has the above meaning, specific examples include methoxycarbonyl, ethoxycarbonyl, n-propyloxycarbonyl, i-propyloxycarbonyl, n-butoxycarbonyl, i-butoxycarbonyl, s-butoxycarbonyl, tert-butoxycarbonyl, and 2-ethylhexyloxycarbonyl, and appropriate one according to the specified numbers of carbon atoms is selected therefrom.

The expression "halo (Cₐ-C_{b}) alkoxycarbonyl" in the present specification indicates a haloalkyl-O-C(O)- group, wherein the haloalkyl has a to b carbon atoms and has the above meaning, specific examples include chloromethoxycarbonyl, 2-chloroethoxycarbonyl, 2,2-difluoroethoxycarbonyl, 2,2,2-trifluoroethoxycarbonyl, and 2,2,2-trichloroethoxycarbonyl, and appropriate one according to the specified numbers of carbon atoms is selected therefrom.

The expression "Cₐ-C_{b} alkylaminocarbonyl" in the present specification indicates a carbamoyl group in which one of the hydrogen atoms is substituted by alkyl having a to b carbon atoms and having the above meaning, specific examples include methylcarbamoyl, ethylcarbamoyl, n-propylcarbamoyl, i-propylcarbamoyl, n-butylcarbamoyl, i-butylcarbamoyl, s-butylcarbamoyl, and tert-butylcarbamoyl, and appropriate one according to the specified numbers of carbon atoms is selected therefrom.

The expression "halo (Cₐ-C_{b}) alkylaminocarbonyl" in the present specification indicates a carbamoyl group in which one of the hydrogen atoms is substituted by haloalkyl having a to b carbon atoms and having the above meaning, specific examples include 2-fluoroethylcarbamoyl, 2-chloroethylcarbamoyl, 2,2-difluoroethylcarbamoyl, and 2,2,2-trifluoroethylcarbamoyl, and appropriate one according to the specified numbers of carbon atoms is selected therefrom.

The expression "di (Cₐ-C_{b}) alkylaminocarbonyl" in the present specification indicates a carbamoyl group in which each of the hydrogen atoms is substituted by alkyl having a to b carbon atoms and having the above meanings, wherein the two alkyl groups may be same or different from each other. Specific examples include N,N-dimethylcarbamoyl, N-ethyl-N-methylcarbamoyl, N,N-diethylcarbamoyl, N,N-di(n-propyl)carbamoyl, and N,N-di(n-butyl)carbamoyl, and appropriate one according to the specified numbers of carbon atoms is selected therefrom.

The expression "Cₐ-C_{b} alkylcarbonyloxy" in the present specification indicates an alkyl-C(O)-O- group, wherein the alkyl has a to b carbon atoms and has the above meaning, specific examples include acetyloxy, propionyloxy, butyryloxy, and isobutyryloxy, and appropriate one according to the specified numbers of carbon atoms is selected therefrom.

The expression "Cₐ-C_{b} alkoxycarbonyloxy" in the present specification indicates an alkoxy-C(O)-O- group, wherein the alkoxy has a to b carbon atoms and has the above meaning, specific examples include methoxycarbonyloxy, ethoxycarbonyloxy, i-butyloxycarbonyloxy, and tert-butyloxycarbonyloxy, and appropriate one according to the specified numbers of carbon atoms is selected therefrom.

The expression "Cₐ-C_{b} alkylsulfonyloxy" in the present specification indicates an alkylsulfonyl-O- group, wherein the alkylsulfonyl has a to b carbon atoms and has the above meaning, specific examples include methylsulfonyloxy, ethylsulfonyloxy, n-propylsulfonyloxy, and i-propylsulfonyloxy, and appropriate one according to the specified numbers of carbon atoms is selected therefrom.

The expression "halo (Cₐ-C_{b}) alkylsulfonyloxy" in the present specification indicates a haloalkylsulfonyl-O- group, wherein the haloalkylsulfonyl has a to b carbon atoms and has the above meaning, specific examples include difluoromethylsulfonyloxy, trifluoromethylsulfonyloxy, chlorodifluoromethylsulfonyloxy, and bromodifluoromethylsulfonyloxy, and appropriate one according to the specified numbers of carbon atoms is selected therefrom.

The expression "Cₐ-C_{b} alkylaminosulfonyl" in the present specification indicates a sulfamoyl group in which one of the hydrogen atoms is substituted by alkyl having a to b carbon atoms and having the above meaning, specific examples include methylsulfamoyl, ethylsulfamoyl, n-propylsulfamoyl, i-propylsulfamoyl, n-butylsulfamoyl, i-butylsulfamoyl, s-butylsulfamoyl, and tert-butylsulfamoyl, and appropriate one according to the specified numbers of carbon atoms is selected therefrom.

The expression "di (Cₐ-C_{b}) alkylaminosulfonyl" in the present specification indicates a sulfamoyl group in which each of the hydrogen atoms is substituted by alkyl having a to b carbon atoms and having the above meaning, wherein the two alkyl groups may be same or different from each other, specific examples include N,N-dimethylsulfamoyl, N-ethyl-N-methylsulfamoyl, N,N-diethylsulfamoyl, N,N-di(n-propyl)sulfamoyl, and N,N-di(n-butyl)sulfamoyl, and appropriate one according to the specified numbers of carbon atoms is selected therefrom.

The expression "Cₐ-C_{b} cycloalkylcarbonyl" in the present specification indicates a cycloalkyl-C(O)- group, wherein the cycloalkyl has a to b carbon atoms and has the above meaning, specific examples include cyclopropylcarbonyl, 2-methylcyclopropylcarbonyl, and cyclobutylcarbonyl, and appropriate one according to the specified numbers of carbon atoms is selected therefrom.

The expression "halo (Cₐ-C_{b}) cycloalkylcarbonyl" in the present specification indicates a halocycloalkyl-C(O)- group, wherein the halocycloalkyl has a to b carbon atoms and has the above meaning, specific examples include 2,2-dichlorocyclopropylcarbonyl and 2,2-dichloro-1-methylcyclopropylcarbonyl, and appropriate one according to the specified numbers of carbon atoms is selected therefrom.

The expression "(Cₐ-C_{b}) alkyl substituted with R⁶" in the present specification indicates an alkyl group, wherein the alkyl has a to b carbon atoms and has the above meaning, in which any hydrogen atoms bonding to carbon atoms are partly or totally substituted by one or more substituents R⁶, and appropriate one according to the specified numbers of carbon atoms is selected therefrom; wherein if two or more substituents R⁶ are present, the substituents R⁶ may be same or different from each other.

The expression "phenyl substituted with R⁸" in the present specification indicates a phenyl group in which any hydrogen atoms bonding to carbon atoms are substituted by one or more substituents R⁸; wherein if two or more substituents R⁸ are present, the substituents R⁸ may be same or different from each other.

The expression "(Cₐ-C_{b}) alkyl substituted with R⁹" in the present specification indicates an alkyl group, wherein the alkyl has a to b carbon atoms and has the above meaning, in which any hydrogen atoms bonding to carbon atoms are partly or totally substituted by one or more substituents R⁹, and appropriate one according to the specified numbers of carbon atoms is selected therefrom; wherein if two or more substituents R⁹ are present, the substituents R⁹ may be same or different from each other.

The expression "phenyl substituted with R¹⁰" in the present specification indicates a phenyl group in which any hydrogen atoms bonding to carbon atoms are substituted by one or more substituents R¹⁰; wherein if two or more substituents R¹⁰ are present, the substituents R¹⁰ may be same or different from each other.

The expression "phenyl substituted with R¹¹" in the present specification indicates a phenyl group in which any hydrogen atoms bonding to carbon atoms are substituted by one or more substituents R¹¹; wherein if two or more substituents R¹¹ are present, the substituents R¹¹ may be same or different from each other.

Next, production methods for the presently invented compound represented by the formula (1) will be described in the following. For example, the presently invented compound can be produced with any of methods shown below. Description given below merely shows examples, and the presently invented compound represented by the formula (1) may be produced with another method. Hereinafter, "the presently invented compound represented by the formula (1)" is also referred to as "presently invented compound (1)", and "a (the) compound represented by a formula (2)" is also referred to as "compound (2)". Other compounds are described in the same manner as this.

### [Production Method 1]

Presently invented compound (1) can be produced by reacting compound (2) and compound (3): 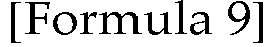 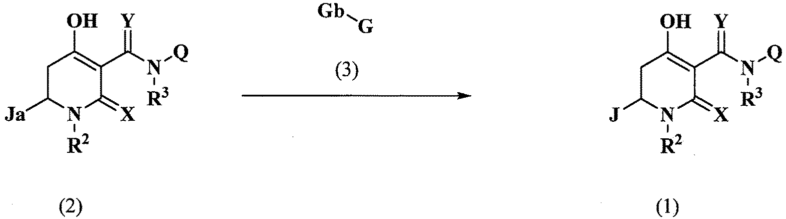 wherein Ja represents Ja-1 or Ja-2, 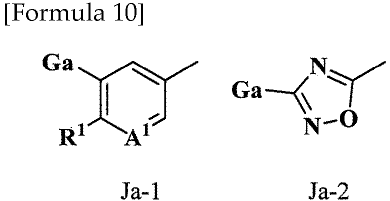 J, Q, G, X, Y, A¹, R¹, R², and R³ have the same meaning as described above, Ga represents, for example, a halogen atom, and Gb represents, for example, B(OH)₂.

Presently invented compound (1) can be produced by reacting compound (2) and compound (3) in a solvent or without any solvent.

If a solvent is used, the solvent to be used needs to be inert to the reaction, and examples thereof include: water; alcoholic solvents such as methanol and ethanol; ether solvents such as diethyl ether, tetrahydrofuran, 1,4-dioxane, and 1,2-dimethoxyethane; aromatic hydrocarbon solvents such as benzene, xylene, and toluene; aliphatic hydrocarbon solvents such as pentane, hexane, and cyclohexane; halogenated hydrocarbon solvents such as dichloromethane, chloroform, and 1,2-dichloroethane; nitrile solvents such as acetonitrile and propionitrile; amide solvents such as N,N-dimethylformamide, N,N-dimethylacetamide, N-methylpyrrolidone, and N,N'-dimethylimidazolidinone; dimethyl sulfoxide; and mixed solvents of any of them.

The reaction may be performed with a base. Examples of the applicable base include: organic bases such as pyridine, 2,6-lutidine, 4-dimethylaminopyridine, triethylamine, diisopropylethylamine, tributylamine, N,N-dimethylaniline, 1,4-diazabicyclo[2.2.2]octane (DABCO), 1,8-diazabicyclo[5.4.0]-7-undecene (DBU), and 1,5-diazabicyclo[4.3.0]-5-nonene (DBN); inorganic bases such as sodium hydroxide, potassium hydroxide, sodium hydride, sodium hydrogen carbonate, potassium carbonate, cesium carbonate, and potassium phosphate; and metal alkoxides such as sodium methoxide, sodium ethoxide, and potassium tert-butoxide. The amount of usage (equivalent) of the base can be 0.1 to 100 equivalents per equivalent of compound (2).

The reaction can be performed in the presence of a palladium catalyst. Examples of the palladium catalyst include a [1,1'-bis(diphenylphosphino)ferrocene]palladium (divalent) dichloride dichloromethane adduct. The amount of usage of the palladium catalyst can be 0.005 to 20 equivalents per equivalent of compound (2).

The reaction can be performed in the presence of a ligand. Examples of the ligand include 1,1'-bis(diphenylphosphino)ferrocene. The amount of usage of the ligand can be 0.005 to 20 equivalents per equivalent of compound (2).

The reaction temperature can be set to any temperature between -78°C and the reflux temperature of the reaction mixture, and the reaction time, which depends on the concentrations of the reaction substrates and the reaction temperature, can be arbitrarily set typically in the range of 5 minutes to 100 hours.

Compound (2) can be produced by using a method known to the public through publication, for example, according to a method described in International Publication No. WO 2021/261562.

Some kinds of compound (3) are known compounds, and some of them are obtainable as commercially available products. Even compounds other than them can be produced according to synthesis methods for the known compounds.

### [Production Method 2]

Presently invented compound (1) can be produced by reacting compound (4) and compound (5): 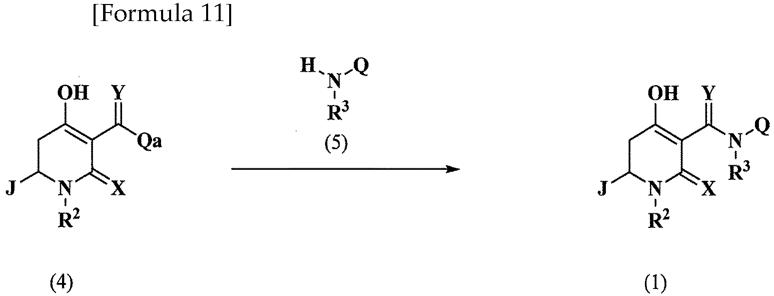 wherein **J,** Q, X, Y, R², and R³ have the same meaning as described above, and Qa represents C₁-C₆ alkoxy.

Presently invented compound (1) can be produced by reacting compound (4) and compound (5) in a solvent or without any solvent.

If a solvent is used, the solvent to be used needs to be inert to the reaction, and examples thereof include the solvents shown as examples in Production Method 1.

The reaction may be performed with a base. Examples of the applicable base include the bases shown as examples in Production Method 1. The amount of usage (equivalent) of the base can be 0.1 to 100 equivalents per equivalent of compound (4).

The reaction temperature can be set to any temperature between -78°C and the reflux temperature of the reaction mixture, and the reaction time, which depends on the concentrations of the reaction substrates and the reaction temperature, can be arbitrarily set typically in the range of 5 minutes to 100 hours.

Compound (4) can be produced by using a method known to the public through publication, for example, according to a method described in International Publication No. WO 2021/261562.

Some kinds of compound (5) are known compounds, and some of them are obtainable as commercially available products. Even compounds other than them can be produced according to synthesis methods for the known compounds.

### [Production Method 3]

Compound (1a), which is presently invented compound (1) in which J represents J-2, can be produced by reacting compound (6): 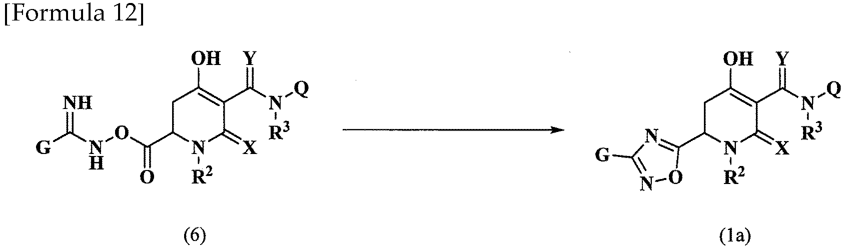 wherein, Q, G, X, Y, R², and R³ have the same meanings as described above.

Compound (1a) can be produced by reacting compound (6) in a solvent or without any solvent.

If a solvent is used, the solvent to be used needs to be inert to the reaction, and examples thereof include the solvents shown as examples in Production Method 1.

The reaction may be performed with a base. Examples of the applicable base include the bases shown as examples in Production Method 1. The amount of usage (equivalent) of the base can be 0.1 to 100 equivalents per equivalent of compound (6).

The reaction temperature can be set to any temperature between -78°C and the reflux temperature of the reaction mixture, and the reaction time, which depends on the concentrations of the reaction substrates and the reaction temperature, can be arbitrarily set typically in the range of 5 minutes to 100 hours.

Compound (6) to be used in Production Method 3 can be produced according to the following reaction formula: 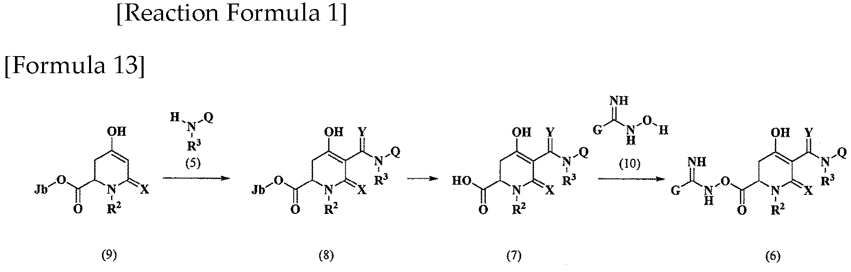 wherein Q, G, X, Y, R², and R³ have the same meanings as described above, and Jb represents C₁-C₆ alkyl.

### [Production Step 1]

Compound (8) can be produced by reacting compound (9) with compound (5) by using a method known to the public through publication, for example, according to a method described in International Publication No. WO 2016/118611.

Some kinds of compound (9) are known compounds, and some of them are obtainable as commercially available products. Even compounds other than them can be produced by using a method known to the public through publication, for example, according to a method described in The Journal of Organic Chemistry, 2004, vol. 69, pp. 130 to 141.

### [Production Step 2]

Compound (7) can be produced by reacting compound (8) by using a method known to the public through publication, for example, according to a method described in The Journal of Organic Chemistry, 2006, vol. 24, pp. 9045 to 9050.

### [Production Step 3]

Compound (6) can be produced by reacting compound (7) with compound (10) by using a method known to the public through publication, for example, according to a method described in Synthetic Communications, 2006, vol. 36, pp. 729 to 741.

Some kinds of compound (10) are known compounds, and some of them are obtainable as commercially available products. Even compounds other than them can be produced according to a method described in Bioorganic & Medicinal Chemistry, 2018, vol. 26, pp. 1713 to 1726.

In each of the reactions of Production Methods 1 to 3 and Reaction Formula 1, the reaction mixture after the completion of the reaction can be subjected to conventional aftertreatment such as direct concentration, or dissolution in organic solvent, washing with water, and then concentration, or putting in iced water, extraction with organic solvent, and then concentration, giving the target presently invented compound. If the need of purification arises, separation and purification can be performed with any purification method such as recrystallization, a column chromatograph, a thin--layer chromatograph, and separation with a liquid chromatograph.

Examples of production intermediates for the presently invented compound that can be produced with any of those methods include compounds shown in Table 1 to Table 6.

In the tables, the notations Q-1, Q-2, Q-3, Q-4, Q-6, Q-9, Q-10, and Q-12a indicate the following structures.

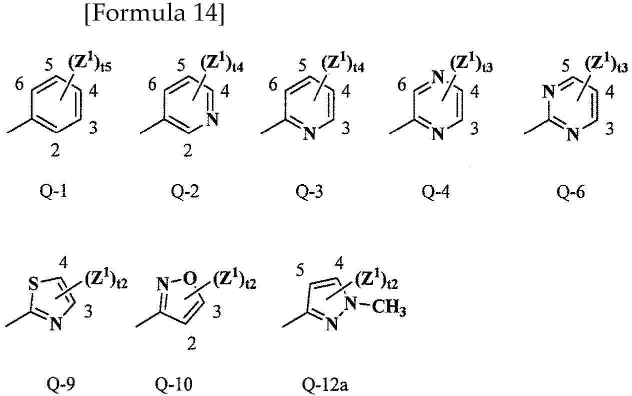

In the tables, the notations G-1, G-2, G-3, and G-7 indicate the following structures.

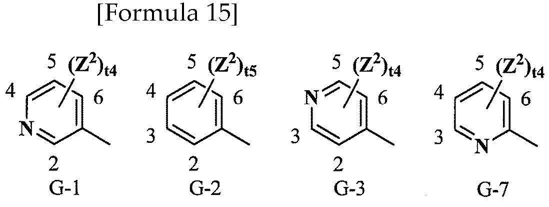

The numbers given to the structural formulas are each a number indicating the position of substitution with the substituent (Z¹)ₜ₂, (Z¹)ₜ₃, (Z¹)ₜ₄, (Z¹)ₜ₅, (Z²)ₜ₄ or (Z²)ₜ₅; for example, "Q-1(4-F)" in the tables means "4-fluorophenyl", wherein "Q-1" means "phenyl".

**[Table 1]**

| 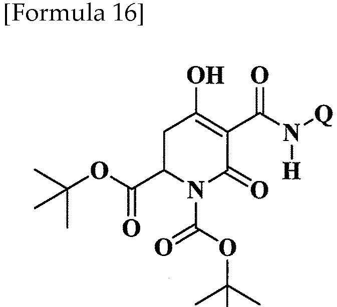 |
|---|
| Q |
| Q-1 |
| Q-1(4-F) |
| Q-1(4-Cl) |
| Q-1(4-Br) |
| Q-1(4-I) |
| Q-1(4-CN) |
| Q-1(4-CH₃) |
| Q-1(4-CF₃) |
| Q-2 |
| Q-2(4-F) |
| Q-2(4-Cl) |
| Q-2(4-Br) |
| Q-2(4-I) |
| Q-2(4-CN) |
| Q-2(4-CH₃) |
| Q-2(4-CF₃) |
| Q-3 |
| Q-3(4-F) |
| Q-3(4-Cl) |
| Q-3(4-Br) |
| Q-3(4-I) |
| Q-3(4-CN) |
| Q-3(4-CH₃) |
| Q-3(4-CF₃) |
| Q-4 |
| Q-4(4-F) |
| Q-4(4-Cl) |
| Q-4(4-Br) |
| Q-4(4-I) |
| Q-4(4-CN) |
| Q-4(4-CH₃) |
| Q-4(4-CF₃) |
| Q-6 |
| Q-6(4-F) |
| Q-6(4-Cl) |
| Q-6(4-Br) |
| Q-6(4-I) |
| Q-6(4-CN) |
| Q-6(4-CH₃) |
| Q-6(4-CF₃) |
| Q-9 |
| Q-9(4-F) |
| Q-9(4-Cl) |
| Q-9(4-Br) |
| Q-9(4-I) |
| Q-9(4-CN) |
| Q-9(4-CH₃) |
| Q-9(4-CF₃) |
| Q-10 |
| Q-10(3-F) |
| Q-10(3-Cl) |
| Q-10(3-Br) |
| Q-10(3-I) |
| Q-10(3-CN) |
| Q-10(3-CH₃) |
| Q-10(3-CF₃) |
| Q-12a |
| Q-12a(4-F) |
| Q-12a(4-Cl) |
| Q-12a(4-Br) |
| Q-12a(4-I) |
| Q-12a(4-CN) |
| Q-12a(4-CH₃) |
| Q-12a(4-CF₃) |
| Q-1(3-F) |
| Q-1(3-Cl) |
| Q-1(3-Br) |
| Q-1(3-I) |
| Q-1(3-CN) |
| Q-1(3-CH₃) |
| Q-1(3-CF₃) |
| Q-1(2-F) |
| Q-1(2-Cl) |
| Q-1(2-Br) |
| Q-1(2-I) |
| Q-1(2-CN) |
| Q-1(2-CH₃) |
| Q-1(2-CF₃) |
| Q-3(3-F) |
| Q-3(3-Cl) |
| Q-3(3-Br) |
| Q-3(3-I) |
| Q-3(3-CN) |
| Q-3(3-CH₃) |
| Q-3(3-CF₃) |
| Q-3(5-F) |
| Q-3(5-Cl) |
| Q-3(5-Br) |
| Q-3(5-I) |
| Q-3(5-CN) |
| Q-3(5-CH₃) |
| Q-3(5-CF₃) |
| Q-3(6-F) |
| Q-3(6-Cl) |
| Q-3(6-Br) |
| Q-3(6-I) |
| Q-3(6-CN) |
| Q-3(6-CH₃) |
| Q-3(6-CF₃) |
| Q-4(3-F) |
| Q-4(3-Cl) |
| Q-4(3-Br) |
| Q-4(3-I) |
| Q-4(3-CN) |
| Q-4(3-CH₃) |
| Q-4(3-CF₃) |
| Q-6(3-F) |
| Q-6(3-Cl) |
| Q-6(3-Br) |
| Q-6(3-I) |
| Q-6(3-CN) |
| Q-6(3-CH₃) |
| Q-6(3-CF₃) |
| Q-9(3-F) |
| Q-9(3-Cl) |
| Q-9(3-Br) |
| Q-9(3-I) |
| Q-9(3-CN) |
| Q-9(3-CH₃) |
| Q-9(3-CF₃) |

**[Table 2]**

| 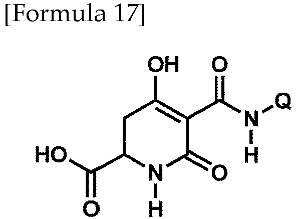 |
|---|
| Q |
| Q-1 |
| Q-1(4-F) |
| Q-1(4-Cl) |
| Q-1(4-Br) |
| Q-1(4-I) |
| Q-1(4-CN) |
| Q-1(4-CH₃) |
| Q-1(4-CF₃) |
| Q-2 |
| Q-2(4-F) |
| Q-2(4-Cl) |
| Q-2(4-Br) |
| Q-2(4-I) |
| Q-2(4-CN) |
| Q-2(4-CH₃) |
| Q-2(4-CF₃) |
| Q-3 |
| Q-3(4-F) |
| Q-3(4-Cl) |
| Q-3(4-Br) |
| Q-3(4-I) |
| Q-3(4-CN) |
| Q-3(4-CH₃) |
| Q-3(4-CF₃) |
| Q-4 |
| Q-4(4-F) |
| Q-4(4-Cl) |
| Q-4(4-Br) |
| Q-4(4-I) |
| Q-4(4-CN) |
| Q-4(4-CH₃) |
| Q-4(4-CF₃) |
| Q-6 |
| Q-6(4-F) |
| Q-6(4-Cl) |
| Q-6(4-Br) |
| Q-6(4-I) |
| Q-6(4-CN) |
| Q-6(4-CH₃) |
| Q-6(4-CF₃) |
| Q-9 |
| Q-9(4-F) |
| Q-9(4-Cl) |
| Q-9(4-Br) |
| Q-9(4-I) |
| Q-9(4-CN) |
| Q-9(4-CH₃) |
| Q-9(4-CF₃) |
| Q-10 |
| Q-10(3-F) |
| Q-10(3-Cl) |
| Q-10(3-Br) |
| Q-10(3-I) |
| Q-10(3-CN) |
| Q-10(3-CH₃) |
| Q-10(3-CF₃) |
| Q-12a |
| Q-12a(4-F) |
| Q-12a(4-Cl) |
| Q-12a(4-Br) |
| Q-12a(4-I) |
| Q-12a(4-CN) |
| Q-12a(4-CH₃) |
| Q-12a(4-CF₃) |
| Q-1(3-F) |
| Q-1(3-Cl) |
| Q-1(3-Br) |
| Q-1(3-I) |
| Q-1(3-CN) |
| Q-1(3-CH₃) |
| Q-1(3-CF₃) |
| Q-1(2-F) |
| Q-1(2-Cl) |
| Q-1(2-Br) |
| Q-1(2-I) |
| Q-1(2-CN) |
| Q-1(2-CH₃) |
| Q-1(2-CF₃) |
| Q-3(3-F) |
| Q-3(3-Cl) |
| Q-3(3-Br) |
| Q-3(3-I) |
| Q-3(3-CN) |
| Q-3(3-CH₃) |
| Q-3(3-CF₃) |
| Q-3(5-F) |
| Q-3(5-Cl) |
| Q-3(5-Br) |
| Q-3(5-I) |
| Q-3(5-CN) |
| Q-3(5-CH₃) |
| Q-3(5-CF₃) |
| Q-3(6-F) |
| Q-3(6-Cl) |
| Q-3(6-Br) |
| Q-3(6-I) |
| Q-3(6-CN) |
| Q-3(6-CH₃) |
| Q-3(6-CF₃) |
| Q-4(3-F) |
| Q-4(3-Cl) |
| Q-4(3-Br) |
| Q-4(3-I) |
| Q-4(3-CN) |
| Q-4(3-CH₃) |
| Q-4(3-CF₃) |
| Q-6(3-F) |
| Q-6(3-Cl) |
| Q-6(3-Br) |
| Q-6(3-I) |
| Q-6(3-CN) |
| Q-6(3-CH₃) |
| Q-6(3-CF₃) |
| Q-9(3-F) |
| Q-9(3-Cl) |
| Q-9(3-Br) |
| Q-9(3-I) |
| Q-9(3-CN) |
| Q-9(3-CH₃) |
| Q-9(3-CF₃) |

**[Table 3]**

| 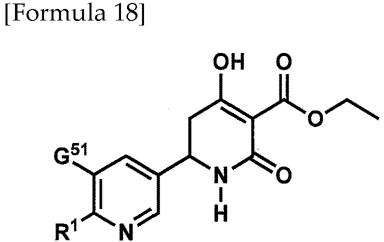 | |
|---|---|
| R¹ | G⁵¹ |
| H | Cl |
| H | Br |
| H | I |
| F | Cl |
| F | Br |
| F | I |
| Cl | Cl |
| Cl | Br |
| Cl | I |
| CHF₂ | Cl |
| CHF₂ | Br |
| CHF₂ | I |
| CF₃ | Cl |
| CF₃ | Br |
| CF₃ | I |

**[Table 4]**

| 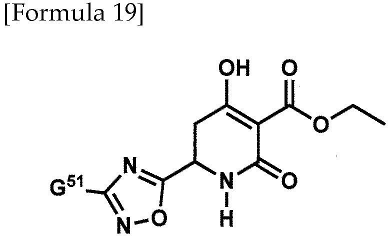 |
|---|
| G⁵¹ |
| Cl |
| Br |
| I |

**[Table 5]**

| 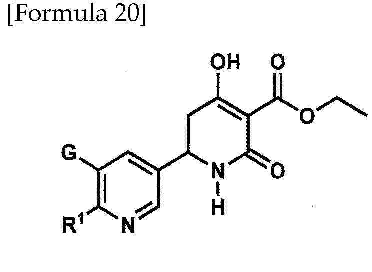 | |
|---|---|
| R¹ | G |
| Cl | G-7 |
| Cl | G-1 |
| Cl | G-3 |
| Cl | G-2 |
| Cl | G-7(3-F) |
| Cl | G-7(4-F) |
| Cl | G-7(5-F) |
| Cl | G-7(6-F) |
| Cl | G-1(2-F) |
| Cl | G-1(4-F) |
| Cl | G-1(5-F) |
| Cl | G-1(6-F) |
| Cl | G-3(2-F) |
| Cl | G-3(3-F) |
| Cl | G-2(2-F) |
| Cl | G-2(3-F) |
| Cl | G-2(4-F) |
| Cl | G-2(2-F,4-F) |
| Cl | G-2(2-F,5-F) |
| Cl | G-7(3-Cl) |
| Cl | G-7(4-Cl) |
| Cl | G-7(5-Cl) |
| Cl | G-7(6-Cl) |
| Cl | G-1(2-Cl) |
| Cl | G-1(4-Cl) |
| Cl | G-1(5-Cl) |
| Cl | G-1(6-Cl) |
| Cl | G-3(2-Cl) |
| Cl | G-3(3-Cl) |
| Cl | G-2(2-Cl) |
| Cl | G-2(3-Cl) |
| Cl | G-2(4-Cl) |
| Cl | G-2(2-Cl,4-Cl) |
| Cl | G-2(2-Cl,5-Cl) |
| Cl | G-7(3-CH₃) |
| Cl | G-7(4-CH₃) |
| Cl | G-7(5-CH₃) |
| Cl | G-7(6-CH₃) |
| Cl | G-1(2-CH₃) |
| Cl | G-1(4-CH₃) |
| Cl | G-1(5-CH₃) |
| Cl | G-1(6-CH₃) |
| Cl | G-3(2-CH₃) |
| Cl | G-3(3-CH₃) |
| Cl | G-2(2-CH₃) |
| Cl | G-2(3-CH₃) |
| Cl | G-2(4-CH₃) |
| Cl | G-2(2-CH₃,4-CH₃) |
| Cl | G-2(2-CH₃,5-CH₃) |
| Cl | G-7(3-CF₃) |
| Cl | G-7(4-CF₃) |
| Cl | G-7(5-CF₃) |
| Cl | G-7(6-CF₃) |
| Cl | G-1(2-CF₃) |
| Cl | G-1(4-CF₃) |
| Cl | G-1(5-CF₃) |
| Cl | G-1(6-CF₃) |
| Cl | G-3(2-CF₃) |
| Cl | G-3(3-CF₃) |
| Cl | G-2(2-CF₃) |
| Cl | G-2(3-CF₃) |
| Cl | G-2(4-CF₃) |
| Cl | G-2(2-CF₃,4-CF₃) |
| Cl | G-2(2-CF₃,5-CF₃) |
| CF₃ | G-7 |
| CF₃ | G-1 |
| CF₃ | G-3 |
| CF₃ | G-2 |
| CF₃ | G-7(3-F) |
| CF₃ | G-7(4-F) |
| CF₃ | G-7(5-F) |
| CF₃ | G-7(6-F) |
| CF₃ | G-1(2-F) |
| CF₃ | G-1(4-F) |
| CF₃ | G-1(5-F) |
| CF₃ | G-1(6-F) |
| CF₃ | G-3(2-F) |
| CF₃ | G-3(3-F) |
| CF₃ | G-2(2-F) |
| CF₃ | G-2(3-F) |
| CF₃ | G-2(4-F) |
| CF₃ | G-2(2-F,4-F) |
| CF₃ | G-2(2-F,5-F) |
| CF₃ | G-7(3-Cl) |
| CF₃ | G-7(4-Cl) |
| CF₃ | G-7(5-Cl) |
| CF₃ | G-7(6-Cl) |
| CF₃ | G-1(2-Cl) |
| CF₃ | G-1(4-Cl) |
| CF₃ | G-1(5-Cl) |
| CF₃ | G-1(6-Cl) |
| CF₃ | G-3(2-Cl) |
| CF₃ | G-3(3-Cl) |
| CF₃ | G-2(2-Cl) |
| CF₃ | G-2(3-Cl) |
| CF₃ | G-2(4-Cl) |
| CF₃ | G-2(2-Cl,4-Cl) |
| CF₃ | G-2(2-Cl,5-Cl) |
| CF₃ | G-7(3-CH₃) |
| CF₃ | G-7(4-CH₃) |
| CF₃ | G-7(5-CH₃) |
| CF₃ | G-7(6-CH₃) |
| CF₃ | G-1(2-CH₃) |
| CF₃ | G-1(4-CH₃) |
| CF₃ | G-1(5-CH₃) |
| CF₃ | G-1(6-CH₃) |
| CF₃ | G-3(2-CH₃) |
| CF₃ | G-3(3-CH₃) |
| CF₃ | G-2(2-CH₃) |
| CF₃ | G-2(3-CH₃) |
| CF₃ | G-2(4-CH₃) |
| CF₃ | G-2(2-CH₃,4-CH₃) |
| CF₃ | G-2(2-CH₃,5-CH₃) |
| CF₃ | G-7(3-CF₃) |
| CF₃ | G-7(4-CF₃) |
| CF₃ | G-7(5-CF₃) |
| CF₃ | G-7(6-CF₃) |
| CF₃ | G-1(2-CF₃) |
| CF₃ | G-1(4-CF₃) |
| CF₃ | G-1(5-CF₃) |
| CF₃ | G-1(6-CF₃) |
| CF₃ | G-3(2-CF₃) |
| CF₃ | G-3(3-CF₃) |
| CF₃ | G-2(2-CF₃) |
| CF₃ | G-2(3-CF₃) |
| CF₃ | G-2(4-CF₃) |
| CF₃ | G-2(2-CF₃,4-CF₃) |
| CF₃ | G-2(2-CF₃,5-CF₃) |

**[Table 6]**

| 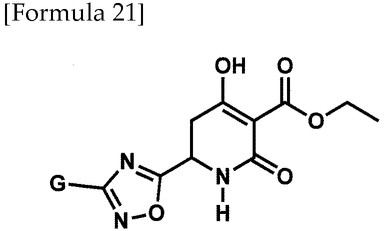 |
|---|
| G |
| G-7 |
| G-1 |
| G-3 |
| G-2 |
| G-7(3-F) |
| G-7(4-F) |
| G-7(5-F) |
| G-7(6-F) |
| G-1(2-F) |
| G-1(4-F) |
| G-1(5-F) |
| G-1(6-F) |
| G-3(2-F) |
| G-3(3-F) |
| G-2(2-F) |
| G-2(3-F) |
| G-2(4-F) |
| G-2(2-F,4-F) |
| G-2(2-F,5-F) |
| G-7(3-Cl) |
| G-7(4-Cl) |
| G-7(5-Cl) |
| G-7(6-Cl) |
| G-1(2-Cl) |
| G-1(4-Cl) |
| G-1(5-Cl) |
| G-1(6-Cl) |
| G-3(2-Cl) |
| G-3(3-Cl) |
| G-2(2-Cl) |
| G-2(3-Cl) |
| G-2(4-Cl) |
| G-2(2-Cl,4-Cl) |
| G-2(2-Cl,5-Cl) |
| G-7(3-CH₃) |
| G-7(4-CH₃) |
| G-7(5-CH₃) |
| G-7(6-CH₃) |
| G-1(2-CH₃) |
| G-1(4-CH₃) |
| G-1(5-CH₃) |
| G-1(6-CH₃) |
| G-3(2-CH₃) |
| G-3(3-CH₃) |
| G-2(2-CH₃) |
| G-2(3-CH₃) |
| G-2(4-CH₃) |
| G-2(2-CH₃,4-CH₃) |
| G-2(2-CH₃,5-CH₃) |
| G-7(3-CF₃) |
| G-7(4-CF₃) |
| G-7(5-CF₃) |
| G-7(6-CF₃) |
| G-1(2-CF₃) |
| G-1(4-CF₃) |
| G-1(5-CF₃) |
| G-1(6-CF₃) |
| G-3(2-CF₃) |
| G-3(3-CF₃) |
| G-2(2-CF₃) |
| G-2(3-CF₃) |
| G-2(4-CF₃) |
| G-2(2-CF₃,4-CF₃) |
| G-2(2-CF₃,5-CF₃) |

The term pesticides in the present invention means noxious insect control agents against harmful arthropods, for example, in the field of agriculture and horticulture or in the field of livestock industry and/or hygiene (internal parasites and external parasites in mammals or birds as livestock or pets, and sanitary insects and nuisance insects at home and in industry).

The term agrochemicals in the present invention means, for example, insecticides or acaricides, nematicides, herbicides, and microbicides in the field of agriculture and horticulture.

The term sanitary insects herein means, for example, harmful invertebrates that give rise to severe pain or allergic symptoms such as puffiness and itching and cause lethal anaphylactic shock in some cases by stinging a target animal, and occasionally spread serious diseases to bring about death in some cases through blood-sucking, invertebrates that come in contact with foods and thereby contaminate the foods with pathogens such as viruses, bacteria, and parasites in some cases, invertebrates that cause allergic diseases such as bronchial asthma, rhinitis, conjunctivitis, and atopic dermatitis via their living bodies, dead bodies, exuviae, feces, etc., as allergens, invertebrates that cause economic damage by damaging foods, cloths, housing, etc., through eating, and invertebrates that do not cause direct damage but give rise to unpleasant feelings through generation in and/or invasion into human residential environments. More specifically, the term means ants, which bite with their mandibles, wasps, which sting with their stings, mosquitoes and assassin bags, which suck blood from skin, and termites, which are omnivorous and cause damage to buildings such as houses.

The term nuisance insects herein refers to noxious insects that do not directly harm humans in human living environments but give rise to unpleasant feelings to cause mental damage through their appearances and shapes.

Specific examples of insects, mites, crustaceans, mollusks, nematodes, external parasites, and internal parasites that can be controlled with the presently invented compound include the following organisms, but not limited to them in the present invention.

Examples of noxious insects include:
Lepidoptera; allium leafminer *(Acrolepiopsis sapporensis),* yam leafminer (*Acrolepiopsis suzukiella*)*,* smaller tea tortrix (*Adoxophyes honmai*), summer fruit tortrix (*Adoxophyes orana fasciata*), fruit-piercing moth (*Adris tyrannus*)*,* sweet potato leaf worm (*Aedia leucomelas*), black cutworm (*Agrotis ipsilon*), turnip moth (*Agrotis segetum*), cotton leafworm (*Alabama argillacea*), Asiatic leafroller (*Archips breviplicanus*)*,* apple tortrix (*Archips fuscocupreanus*), oriental tussock moth (*Artaxa subflava*), bamboo zygaenid (*Artona martini*), Japanese giant looper (*Ascotis selenaria*)*,* Asiatic common looper (*Autographa nigrisigna*)*,* pear leaf miner (*Bucculatrix pyrivorella*)*,* tea leafroller (*Caloptilia theivora*)*,* peach fruit moth (*Carposina sasakii*)*,* rice stem borer (*Chilo suppressalis*)*,* rice leafroller (*Cnaphalocrocis medinalis*), yellow peach moth (*Conogethes punctiferalis*), carpenter moth (*Cossus insularis*)*,* three-spotted plusia (*Ctenoplusia agnata*)*,* codling moth (*Cydia pomonella*), pine moth (*Dendrolimus spectabilis*), Japanese hemlock caterpillar (*Dendrolimus superans*)*,* cucumber moth (*Diaphania indica*)*,* sugarcane borer (*Diatraea saccharalis*)*,* Eilema fuscodorsalis (*Eilema fuscodorsalis*), Eilema laevis (*Eilema laevis*)*,* lesser corn stalk borer (*Elasmopalpus lignosellus*)*,* grape berry moth (*Endopiza viteana*)*,* limabean pod borer (*Etiella zinckenella*)*, Euproctis piperita,* tea tussock moth (*Euproctis pseudoconspersa*)*,* Japanese bamboo lappet moth (*Euthrix albomaculata*)*,* drinker moth (*Euthrix potatoria*)*,* oriental lappet (*Gastropacha orientalis*)*,* mulberry pyralid (*Glyphodes pyloalis*)*,* plum fruit moth (*Grapholita dimorpha*)*,* oriental fruit moth (*Grapholita molesta*)*,* sweetpotato leaffolder (*Helcystogramma triannulella*)*,* cotton bollworm (*Helicoverpa armigera*), oriental tobacco budworm (*Helicoverpa assulta*)*,* corn earworm (*Helicoverpa zea*)*,* tobacco budworm (*Heliothis virescens*)*,* cabbage webworm (*Hellula undalis*)*,* oriental tea tortrix (*Homona magnanima*)*,* fall webworm moth (*Hyphantria cunea*)*,* pearleaf worm (*Illiberis pruni*)*,* prunus bud moth (*Illiberis rotundata), Quercus lasiocampid* (*Kunugia undans*), *Quercus lasiocampid* (*Kunugia yamadai*)*,* soybean pod borer (*Leguminivora glycinivorella*)*,* mulberry tiger moth (*Lemyra imparilis*)*,* gypsy moth (*Lymantria dispar*)*,* peach leafminer (*Lyonetia clerkella*)*,* Lyonetia prunifoliella malinella, cabbage armyworm (*Mamestra brassicae*)*,* tomato hornworm (*Manduca quinquemaculata*)*,* tobacco hornworm (*Manduca sexta*)*,* soybean podworm (*Matsumuraeses phaseoli*)*,* oriental moth (*Monema flavescens*)*,* rice green caterpillar (*Naranga aenescens*)*,* white-spotted tussock moth (O*rgyia thyellina*)*,* Asian corn borer (*Ostrinia furnacalis*), European corn borer (*Ostrinia nubilalis*)*,* adzuki bean borer (*Ostrinia scapulalis*)*,* dark fruit-tree tortrix (*Pandemis heparana*)*,* bluegrass webworm (*Parapediasia teterrella*), green cochlid (*Parasa consocia*), *Parasa lepida,* Chinese cochlid (*Parasa sinica*), straight swift (*Parnara guttata*)*,* pink bollworm (*Pectinophora gossypiella*)*,* citrus leafminer (*Phyllocnistis citrella*), apple leafminer (*Phyllonorycter ringoniella*), large white (*Pieris brassicae*)*,* cabbage white butterfly (Pieris rapae crucivora), diamondback moth (*Plutella xylostella*), oriental armyworm (*Pseudaletia separata*), soybean looper (*Pseudoplusia includens*), swan moth (*Sphrageidus similis*), lawn grass cutworm (*Spodoptera depravata*), southern armyworm (*Spodoptera eridania*), beet armyworm (*Spodoptera exigua*), fall armyworm (*Spodoptera frugiperda*), cotton leafworm (*Spodoptera littoralis*), common cutworm (*Spodoptera litura*)*,* persimmon fruit moth (*Stathmopoda masinissa*)*,* peach tree borer (*Synanthedon exitiosa*), cherry tree borer (*Synanthedon hector*), *Toleria romanovi,* and cabbage looper (*Trichoplusia ni*),
Coleoptera; common bean weevil (*Acanthoscelides obtectus*), cupreous chafer (*Anomala cuprea*), soybean beetle (*Anomala rufocuprea*), Asian long-horn beetle (*Anoplophora glabripennis*), white-spotted longicorn beetle (*Anoplophora malasiaca*)*,* boll weevil (*Anthonomus grandis*), cucurbit leaf beetle (*Aulacophora femoralis*), adzuki bean beetle (*Callosobruchus chinensis*), beet flea beetle (*Chaetocnema concinna*), sweetpotato weevil (*Cylas formicariu*s), leaf beetle (*Demotina fasciculata*), northern corn rootworm (*Diabrotica barberi*), southern corn rootworm (*Diabrotica undecimpunctata*), western corn rootworm (*Diabrotica virgifera*), rice plant weevil (*Echinocnemus squameus*), Mexican been beetle (*Epilachna varivestis*), large twenty-eight-spotted ladybird (*Epilachna vigintioctomaculata*), twenty-eight-spotted ladybird (*Epilachna vigintioctopunctata*), *Epuraea domina,* West Indian sweet potato weevil (*Euscepes postfasciatus*), citrus flower chafer (*Gametis jucunda*), white-fringed beetle (*Graphognatus leucoloma*), yellowish elongate chafer (*Heptophylla picea*), alfalfa weevil (*Hypera postica*), tobacco beetle (*Lasioderma serricorne*), Colorado potato beetle (*Leptinotarsa decemlineata*), rice water weevil (*Lissohoptrus oryzophilus*), sweetpotato wireworm (*Melanotus fortnumi*), sugarcane wireworm (*Melanotus tamsuyensis*), pollen beetle (*Meligethes aeneus*), Japanese pine sawyer (*Monochamus alternatus*), black vine weevil (O*tiorhynchus sulcatus*), rice leaf beetle (*Oulema oryzae*), rove beetle (*Paederus fuscipes*), mustard leaf beetle (*Phaedon cochleariae*), striped flea beetle (*Phyllotreta striolata*), Japanese beetle (*Popillia japonica*), yellow-spotted longicorn beetle (*Psacothea hilaris*), solanum flea beetle (*Psylliodes angusticollis*), peach curculio (*Rhynchites heros*), granary weevil (*Sitophilus granarius*), maize weevil (*Sitophilus zeamais*), hunting billbug (*Sphenophorus venatus vestitus*), yellow mealworm (*Tenebrio molitor*), red flour beetle (*Tribolium castaneum*), and grape borer (*Xylotrechus pyrrhoderus*),
Hymenoptera; chestnut sawfly (*Apethymus kuri*), large rose sawfly (*Arge pagana*), cabbage sawfly (*Athalia infumata*), turnip sawfly (*Athalia rosae*), Japanese carpenter ant (*Camponotus japonicus*), chestnut gall wasp (*Dryocosmus kuriphilus*), army ant (*Eciton burchellii, E. schmitti*), Argentine ant (*Linepithema humile*), pharaoh ant (*Monomorium pharaonis*), bulldog ant (*Myrmecia* spp.), European pine sawfly (*Neodiprion sertifer*), fire ant (*Solenopsis* spp.), Asian giant hornet (*Vespa mandarinia*), Japanese yellow hornet (*Vespa simillima*),
Diptera; yellow fever mosquito (*Aedes aegypti*), Asian tiger mosquito (*Aedes albopictus*), *Aedes* spp., *Aedes taeniorhynchus,* rice leaf miner (*Agromyza oryzae*), Mexican fruit fly (*Anastrepha ludens*), African malaria mosquito (*Anopheles gambiae*), African malaria mosquito (Anopheles *hyrcanus sinensis*), *Anopheles koreicus, Anopheles lesteri, Anopheles maculipennis, Anopheles* spp., soybean pod gall midge (*Asphondylia yushimai*), *Atylotus* spp., melon fly (*Bactrocera cucurbitae*), oriental fruit fly (*Bactrocera dorsalis*), Queensland fruit fly (*Bactrocera tryoni*), Japanese orange fly (*Bactrocera tsuneonis*), *Boophthora erythrocephala, Braula coeca, Braula* spp., *Calliphora erythrocephala, Calliphora lata, Calliphora* spp., bottle fly (*Calliphora vicina*), Mediterranean fruit fly (*Ceratitis capitata*), pea leaf miner (*Chromatomyia horticola*), Old World screw-worm fly (*Chrysomya bezziana*), blow fly (*Chrysomya chloropyga*), oriental latrine fly (*Chrysomya megacephala*), *Chrysomya* spp., splayed deerfly (*Chrysops caecutiens*), *Chrysops relictus, Chrysops* spp., deer fly (*Chrysops suavis*), *Chrysozona pluvialis,* New World screw-worm fly (*Cochliomyia hominivorax*), house mosquito (*Culex pipiens molestus*), house mosquito (*Culex pipiens pallens*), southern house mosquito (*Culex quinquefasciatus*), *Culex tarsalis, Culex tritaeniorhynchus, Culex* spp., biting midge (*Culicoides arakawae*), *Culicoides* spp., bot fly (*Cuterebra* spp.,) onion fly (*Delia antiqua*), seed corn maggot (*Delia platura*), human botfly (*Dermatobia hominis*), Japanese fruit fly (*Drosophila suzukii*), *Eusimulium* spp., lesser house fly (*Fannia canicularis*), *Fannia* spp., horse nose bot fly (*Gasterophilus haemorrhoidalis*), *Gasterophilus inermis,* horse bot fly (*Gasterophilus intestinalis*), throat bot fly (*Gasterophilus nasalis*), *Gasterophilus nigricornis, Gasterophilus pecorum, Gasterophilus* spp., tsetse fly (Glossina morsitans, G. palpalis), *Glossina* spp., horn fly (*Haematobia irritans*), *Haematobia irritans exigua, Haematobia* spp., *Haematobia stimulans, Haematopota italic,* common horse fly (*Haematopota pluvialis*), *Haematopota* spp., forest fly (*Hippobosca equina*), *Hippobosca* spp., *Hippobosca variegate, Hybomitra ciurea, Hybomitra* spp., smaller rice leaf miner (*Hydrellia griseola*), *Hydrotaea albipuncta,* sheep headfly (*Hydrotaea irritans*), *Hydrotaea* spp., warble fly (*Hypoderma bovis*), common cattle grub (*Hypoderma lineatum*), *Hypoderma* spp., black gnat (*Leptoconops nipponensis*), *Lipoptena capreoli, Lipoptena cervi, Lipoptena* spp., cabbage leafminer (*Liriomyza brassicae*), tomato leaf miner (*Liriomyza bryoniae*), stone leek leafminer (*Liriomyza chinensis*), pea leafminer (*Liriomyza huidobrensis*), tomato leafminer (*Liriomyza sativae*), serpentine leafminer (*Liriomyza trifolii*)*,* Australian sheep blowfly (*Lucilia cuprina*), green bottle fly (*Lucilia illustris*), common green bottle fly (*Lucilia sericata*), *Lucilia* spp., *Lutzomyia* spp., hessian fly (*Mayetiola destructor*), sheep ked (*Melophagus ovinus*), *Melophagus* spp., sweat fly (*Morellia simplex*), *Morellia* spp., face fly (*Musca autumnalis*), housefly (*Musca domestica*), *Musca* spp., Australian bush fly (*Musca vetustissima*), *Odagmia ornate, Odagmia* spp., sheep nasal bot fly (*Oestrus ovis*), *Oestrus* spp., beet leaf miner (*Pegomya cunicularia*), *Philipomyia* spp., *Phlebotomus longipalpis, Phlebotomus papatasi,* sandfly (*Phlebotomus* spp.), black blow fly (*Phormia regina*), *Prosimulium yezoensis,* northern blowfly (*Protophormia terraenovae*), *Przhevalskiana silenus,* apple maggot (*Rhagoletis pomonella*), *Rhinoestrus* spp., flesh fly (*Sarcophaga carnaria*), flesh fly (*Sarcophaga peregrina*), *Sarcophaga* spp., black fly (*Simulium ochraceum*), *Simulium reptans, Simulium* spp., orange wheat blossom midge (*Sitodiplosis mosellana*), stable fly (*Stomoxys calcitrans*), *Stomoxys* spp., *Tabanus atratus, Tabanus bromius,* greenhead horse fly (*Tabanus nigrovittatus*), *Tabanus spodopterus, Tabanus* spp., *Tabanus sudeticus,* horse fly (*Tabanus trigonus*), moth fly (*Telmatoscopus albipunctatus*), *Tipula paludosa, Tipula* spp., *Wilhelmia equine, Wilhelmia* spp., and *Wohlfahrtia* spp.,
Heteroptera (Hemiptera); green stink bug (*Acrosternum hilare*), pea aphid (*Acyrthosiphon pisum*), camellia spiny whitefly (*Aleurocanthus camelliae*), orange spiny whitefly (*Aleurocanthus spiniferus*), Indian cotton leafhopper (*Amrasca devastans*), California red scale (*Aonidiella aurantii*), cowpea aphid (*Aphis craccivora*)*,* black bean aphid (*Aphis fabae*), soybean aphid (*Aphis glycines*), cotton aphid (*Aphis gossypii*)*,* green apple aphid (*Aphis pomi*), spiraea aphid (*Aphis spiraecola*), pale greenplant bug (*Apolygus spinolae*), grape leafhopper (*Arboridia apicalis*), foxglove aphid (*Aulacorthum solani*)*,* Beardsley leafhopper (*Balclutha saltuella*), silverleaf whitefly (*Bemisia argentifolii*), sweetpotato whitefly (*Bemisia tabaci*), true chinch bug (*Blissus leucopterus*), leafcurl plum aphid (*Brachycaudus helichrysi*), cabbage aphid (*Brevicoryne brassicae*), oriental chinch bug (*Cavelerius saccharivorus*), Indian wax scale (*Ceroplastes ceriferus*), red wax scale (*Ceroplastes rubens*), walnut aphid (*Chromaphis juglandicola*), bed bug (*Cimex lectularius*), rice stink bug (*Cletus punctiger*), citricola scale (*Coccus pseudomagnoliarum*), San Jose scale (*Comstockaspis perniciosa*), citrus whitefly (*Dialeurodes citri*), Asian citrus psyllid (*Diaphorina citri*), *Dichelops furcatus,* Russian wheat aphid (*Diuraphis noxia*), sloe bug (*Dolycoris baccarum*), giant margarodid scale (*Drosicha corpulenta*), rosy apple aphid (*Dysaphis plantaginea*), red cotton bug (*Dysdercus cingulatus*)*,* potato leafhopper (*Empoasca fabae*), *Empoasca nipponica,* tea green leafhopper (*Empoasca onukii*), bean's smaller green leafhopper (*Empoasca sakaii*), grape leafhopper (*Epiacanthus stramineus*), wooly apple aphid (*Eriosoma lanigerum*), cabbage bug (*Eurydema rugosa*), brown stink bug (*Euschistus servus*), white-spotted spined bug (*Eysarcoris aeneus*), *Eysarcoris lewisi,* white-spotted stink bug (*Eysarcoris ventralis*), tea scale (*Fiorinia theae*), shield bug (*Glaucias subpunctatus*), island fleahopper (*Halticus insularis*), brown marmorated stink bug (*Halyomorpha halys*), mealy plum aphid (*Hyalopterus pruni*), cottony cushion scale (*Icerya purchasi*), small brown planthopper (*Laodelphax striatellus*), rice bug (*Leptocorisa chinensis*), turnip aphid (*Lipaphis erysimi*), western tarnished plant bug (*Lygus hesperus*), tarnished plant bug (*Lygus lineolaris*), potato aphid (*Macrosiphum euphorbiae*), aster leafhopper (*Macrosteles fascifrons*), grape Leafhopper (*Macrosteles striifrons*), sugarcane spittlebug (*Mahanarva fimbriolata*)*,* black-margined aphid (*Monellia caryella*), green peach aphid (*Myzus persicae*)*,* lettuce aphid (*Nasonovia ribisnigri*), onion aphid (*Neotoxoptera formosana*), green rice leafhopper (*Nephotettix cincticeps*), eastern green stink bug (*Nezara antennata*), southern green stink bug (*Nezara viridula*)*,* brown rice planthopper (*Nilaparvata lugens*), squash bug (*Paradasynus spinosus*), cotton mealy bug (*Phenacoccus solani*), red-banded stink bug (*Piezodorus guildinii*), red-banded shield bug (*Piezodorus hybneri*), citrus mealybug (*Planococcus citri*), Japanese mealybug (*Planococcus kraunhiae*), brown-winged green bug (*Plautia crossota*), peony scale (*Pseudaonidia paeoniae*), cotton fleahopper (*Pseudatomoscelis seriatus*), mulberry scale (*Pseudaulacaspis pentagona*), white peach scale (*Pseudaulacaspis prunicola*), Comstock mealybug (*Pseudococcus comstocki*), grape mealybug (*Pseudococcus maritimus*), pear sucker (*Psylla pyrisuga*), blood-sucking bug (*Rhodnius prolixus*), bird cherry-oat aphid (*Rhopalosiphum padi*), rice root aphid (*Rhopalosiphum rufiabdominalis*), rhopalid bug (*Rhopalus maculatus*), bean bug (*Riptortus clavatus*), greenbug (*Schizaphis graminum*), Japanese black rice bug (*Scotinophora lurida*), corn leaf aphid (*Sitobion akebiae*), English grain aphid (*Sitobion avenae*), white-backed rice planthopper (*Sogatella furcifera*), rice stink bug (*Stenodema sibiricum*), sorghum plant bug (*Stenotus rubrovittatus*), azalea lace bug (*Stephanitis pyrioides*), seed bug (*Togo hemipterus*), black citrus aphid (*Toxoptera aurantii*), brown citrus aphid (*Toxoptera citricida*), greenhouse whitefly (*Trialeurodes vaporariorum*), kissing bug (*Triatoma dimidiata*), kissing bug (*Triatoma infestans*), rice leaf bug (*Trigonotylus caelestialium*), citrus snow scale (*Unaspis citri*), euonymus scale (*Unaspis euonymi*), arrowhead scale (*Unaspis yanonensis*), and grape phylloxera (*Viteus vitifolii*),
Thysanoptera; flower thrips (*Frankliniella intonsa*), western flower thrips (*Frankliniella occidentalis*), greenhouse thrips (*Heliothrips haemorrhoidalis*), Japanese gall-forming thrips (*Ponticulothrips diospyrosi),* yellow tea thrips (*Scirtothrips dorsalis*), melon thrips (*Thrips palmi*), and onion thrips (*Thrips tabaci*),
Orthoptera; Australian plague locust (*Chortoicetes terminifera*), oriental mole cricket (*Gryllotalpa orientalis*), migratory locust (*Locusta migratoria*), lesser paddy grasshopper (*Oxya japonica*), rice grasshopper (*Oxya yezoensis*), desert locust (*Schistocerca gregaria*), and emma field cricket (*Teleogryllus emma*),
Blattodea (Dictyoptera); German cockroach (*Blattella germanica*), Formosan subterranean termite (*Coptotermes formosanus*), daikoku dry-wood termite (*Cryptotermes domesticus*), western dry-wood termite (*Incisitermes minor*), black-winged subterranean termite (*Odontotermes formosanus*), American cockroach (*Periplaneta americana*), smoky-brown cockroach (*Periplaneta fuliginosa*), Japanese cockroach (*Periplaneta japonica*), and Japanese subterranean termite *(Reticulitermes speratus),*
Isoptera; *Coptotermes formosanus, Reticulitermes speratus,* and *Odontotermes formosanus,*
Acari; cyclamen mite (*Phytonemus pallidus*), broad mite *(Polyphagotarsonemus latus), Tarsonemus bilobatus, Penthaleus erythrocephalus,* winter grain mite (*Penthaleus major*), rice spider mite (*Oligonychus shinkajii*), citrus red mite (*Panonychus citri*), *Panonychus mori,* European red mite (*Panonychus ulmi*), Kanzawa spider mite (*Tetranychus kanzawai),* two-spotted spider mite (*Tetranychus urticae*), *Acaphylla theavagrans,* dry bulb mite (*Aceria tulipae*), tomato erineum mite (*Aculops lycopersici*), pink citrus rust mite (*Aculops pelekassi*), apple rust mite *(Aculus schlechtendali), Eriophyes chibaensis, Phyllocoptruta oleivora,* bulb mite (*Rhizoglyphus robini*), mold mite (*Tyrophagus putrescentiae*), *Tyrophagus similis, Acarapis* spp., honey bee tracheal mite (*Acarapis woodi*), *Acarus* spp., lone star tick (*Amblyomma americanum*)*,* cayenne tick (*Amblyomma cajennense*), South African bont tick (*Amblyomma hebraeum*), gulf coast tick (*Amblyomma maculatum*), *Amblyomma* spp., tropical bont tick (*Amblyomma variegatum*), fowl tick (*Argas persicus*), pigeon tick (*Argas reflexus*), *Argas* spp., blue cattle tick (*Boophilus annulatus*)*, Boophilus calceratus,* African blue tick (*Boophilus decoloratus*)*,* tropical cattle tick (*Boophilus microplus*), *Boophilus* spp., *Caloglyphus* spp., *Chelacaropsis moorei, Cheyletiella blakei,* rabbit fur mite (*Cheyletiella parasitovorax*), *Cheyletiella* spp., *Cheyletiella yasguri, Cheyletus eruditus, Cheyletus malaccensis,* chorioptic mange mite (*Chorioptes bovis*), *Chorioptes* spp., *Cytodites* spp., cattle follicle mite (*Demodex bovis*), *Demodex caballi,* dog follicle mite (*Demodex canis*)*, Demodex caprae,* cat follicle mite (*Demodex cati*), *Demodex equi,* face mite (*Demodex folliculorum*), *Demodex ovis, Demodex* spp., *Sarcoptes suis* mite (*Demodex suis*)*,* winter tick *(Dermacentor albipictus),* Rocky Mountain wood tick (*Dermacentor andersoni*), ornate sheep tick (*Dermacentor marginatus*), meadow tick *(Dermacentor pictus*), ornate dog tick (*Dermacentor reticulatus*), *Dermacentor* spp., American dog tick (*Dermacentor variabilis*), poultry red mite (*Dermanyssus gallinae*), *Dermanyssus* spp., American house dust mite (*Dermatophagoides farina*), house dust mite (*Dermatophagoides pteronyssinus*), *Eutrombicula wichmanni,* storage mite (*Glycyphagus destructor*)*,* house itch mite (*Glycyphagus domesticus*), *Haemaphysalis cinnabarina,* common rodent tick (*Haemaphysalis concinna*), yellow dog tick (*Haemaphysalis leachi*), bush tick *(Haemaphysalis longicornis), Haemaphysalis otophila,* red sheep tick (*Haemaphysalis punctate*), *Haemophysalis* spp., *Haplochthonius simplex,* trombiculid mite (*Helenicula miyagawai*), tortoise tick (*Hyalomma aegyptium*), *Hyalomma anatolicum,* bont-legged tick (*Hyalomma marginatum*)*, Hyalomma mauritanicus, Hyalomma* spp., small smooth bont-legged tick (*Hyalomma transiens*), *Hypodectes* spp., dog tick (*Ixodes canisuga*), hedgehog tick (*Ixodes hexagonus*), Australian paralysis tick (*Ixodes holocyclus*)*,* western black-legged tick (*Ixodes pilosus*)*,* castor bean tick (*Ixodes ricinus*)*,* karoo paralysis tick (*Ixodes rubicundus*)*,* deer tick (*Ixodes scapularis*)*, Ixodes* spp., *Knemidocoptes* spp., *Laminosioptes* spp., *Leptotrombidium akamushi, Leptotrombidium pallida,* tsutsugamushi mite (*Leptotrombidium scutellare*)*, Listrophorus* spp., feather mite (*Megninia cubitalis*)*, Myobia* spp., *Neoschoengastia xerothermobia,* harvest mite (*Neotrombicula autumnalis*)*, Neotrombicula desaleri,* cat mange mite (*Notoedres cati*)*, Notoedres* spp., *Ornithocheyletia* spp., soft tick (*Ornithodorus moubata*)*, Ornithodorus* spp., tropical fowl mite (*Ornithonyssus bursa*)*, Ornithonyssus* spp., northern fowl mite (*Ornithonyssus sylviarum*), spinose ear tick (*Otobius megnini*)*, Otobius* spp., dog ear mite (*Otodectes cynotis*), *Otodectes* spp., canine nasal mite (*Pneumonyssoides caninum*)*, Pneumonyssoides mange, Pneumonyssus* spp., *Pneumonyssus* spp., *Psorergates* spp., psoroptic mite (*Psoroptes communis*), rabbit ear mite (*Psoroptes cuniculi*)*, Psoroptes equi,* sheep scab mite (*Psoroptes ovis*), *Psoergates ovis* (*Psoroptes ovis*), *Psoroptes* spp., feather mite (*Pterolichus obtusus*), *Pterolichus* spp., *Raillietia* spp., brown ear tick (*Rhipicephalus appendiculatus*), brown ear tick (*Rhipicephalus bursa*), *Rhipicephalus capensis,* Asian blue tick (*Rhipicephalus evertsi*), kennel tick (*Rhipicephalus sanguineus*)*, Rhipicephalus* spp., *Rhipicephalus turanicus, Rhipicephalus zambeziensis, Sarcoptes bovis, Sarcoptes equi, Sarcoptes ovis, Sarcoptes rupicaprae* (= *S. caprae*), itch mite (*Sarcoptes scabiei*)*, Sarcoptes* spp., *Sarcoptes suis,* itch mite *(Sarcoptis canis), Sternostoma* spp., *Trombicula akamushi, Trombicula* spp., cheese mite (*Tyrophagus putrescentiae*)*, Tyrophagus* spp., varroa mite (*Varroa jacobsoni*), and *Varroa* spp.,
Phthiraptera (Anoplurida); short-nosed cattle louse (*Haematopinus eurysternus*), tail switch louse (*Haematopinus quadripertusus*), *Haematopinus* spp., large pig louse (*Haematopinus suis*), buffalo louse (*Haematopinus tuberculatus),* rabbit louse (*Haemodipsus ventricosus*), dog sucking louse (*Linognathus setosus*), *Linognathus* spp., long-nosed cattle louse (*Linognathus vituri*), head louse (*Pediculus humanus*), *Pediculus* spp., mouse louse (*Polyplax serratus*)*,* crab louse (*Pthirus pubis*), *Pthirus* spp., little blue cattle louse (*Solenopotes capillatus*), and *Solenopotes* spp.,
Mallophaga; goat biting louse (*Bovicola caprae*)*, Bovicola limbata,* sheep body louse (*Bovicola ovis*), *Bovicola* spp., chicken head louse (*Cuclotogaster heterographa*), cattle chewing louse (*Damalinia bovis*), *Felicola* spp., cat louse (*Felicola subrostrata*), brown chicken louse (*Goniodes dissmilis*)*,* fluff louse (*Goniodes gallinae*), large hen louse (*Goniodes gigas*)*, Lepikentron ovis, Lepikentron* spp., wing louse (*Lipeurus caponis*), body louse (*Menacanthus cornutus*), small body louse (*Menacanthus pallidulus*), *Menacanthus* spp., chicken body louse (*Menacanthus stramineus*), chicken shaft louse (*Menopon gallinae*), *Menopon* spp., dog biting louse (*Trichodectes canis*)*, Trichodectes* spp., *Werneckiella equi,* and *Werneckiella* spp., and
Siphonaptera; hen flea *(Ceratophyllus gallinae*), dog flea (*Ctenocephalides canis*), cat flea *(*C*tenocephalides felis*), sticktight flea (*Echidnophaga gallinacean*), human flea (*Pulex irritans*)*,* chigoe flea (*Tunga penetrans*), and oriental rat flea (*Xenopsylla cheopis*), and
examples of other parasites include:
monogeneans (Monogenea); *Benedenia epinepheli, Benedenia hoshinai, Benedenia sekii, Benedenia seriolae, Bivagina tai,* sea louse (*Caligus curtus*), *Caligus elongates, Caligus labaracis, Caligus longipedis, Caligus orientalis, Caligus teres, Dactylogyrus* spp., *Gyrodactylus* spp., *Heteraxine heterocerca, Heterobothrium okamotoi, Lamellodiscus* spp., copepod (*Lepeophtheirus cuneifer*), *Lepeophtheirus hippoglossi, Lepeophtheirus pectoralis,* salmon louse (*Lepeophtheirus salmonis*), *Microcotyle sebastis, Microcotyle sebastisci, Neobenedenia, Neobenedenia congeri, Neobenedenia girellae, Neoheterobothrium hirame, Polystoma* spp., *Pseudocaligus fugu,* and *Zeuxapta japonica,*
cestodes (Cestoda); *Andyra* spp., *Anoplocephala* spp., *Avitellina* spp., *Bertiella* spp., *Bothridium* spp., *Cittotaenia* spp., *Davainea* spp., *Diorchis* spp., Manson's tapeworm (*Diphyllobothrium erinacei*)*,* fish tapeworm (*Diphyllobothrium latum*), *Diphyllobothrium* spp., *Diphyllogonoporus* spp., *Diplopylidium* spp., dog tapeworm (*Dipylidium caninum), Dipylidium* spp., dog tapeworm (*Echinococcus granulosus*), fox tapeworm (*Echinococcus multilocularis*), *Echinococcus* spp., *Echinocotyle* spp., *Echinolepsis* spp., *Hydatigera* spp., *Hymenolepis diminuta, Hymenolepis* spp., *Joyeuxiella* spp., *Ligura* spp., *Mesocestoides* spp., *Moniezia benedeni, Moniezia* spp., *Paranoplocephala* spp., *Raillietina* spp., *Schistocephalus* spp., *Spirometra* spp., *Stilesia* spp., beef tapeworm (*Taenia saginata*), pork tapeworm (*Taenia solium*), *Taenia* spp., cat tapeworm (*Taenia taeniaeformis*), *Thysaniezia* spp., and *Thysanosomsa* spp.,
trematodes (Trematoda); *Austrobilharzia* spp., *Brachylaema* spp., *Calicophoron* spp., *Catatropis* spp., Chinese river fluke (*Clonorchis sinensis*), *Clonorchis* spp., *Collyriclum* spp., *Cotylophoron* spp., *Cyclocoelum* spp., *Dicrocoelium* spp., *Diplostomum* spp., *Echinochasmus* spp., *Echinoparyphium* spp., *Echinostoma* spp., *Eurytrema coelomaticum,* pancreas fluke (*Eurytrema pancreaticum*), *Eurytrema* spp., liver fluke (*Fasciola gigantica*), sheep liver fluke (*Fasciola hepatica*), *Fasciola* spp., *Fasciolides* spp., large intestinal fluke (*Fasciolopsis bruski*), *Fasciolopsis* spp., *Fischoederius* spp., *Gastrothylacus* spp., *Gigantobilharzia* spp., *Gigantocotyle* spp., *Heterophyes* spp., *Hypoderaeum* spp., *Leucochloridium* spp., *Metagonimus* spp., *Metorchis* spp., *Nanophyetus* spp., *Notocotylus* spp., *Opisthorchis* spp., *Ornithobilharzia* spp., *Paragonimus* spp., oriental lung fluke (*Paragonimus westermani), Paramphistomum* spp., *Plagiorchis* spp., *Posthodiplostomum* spp., *Prosthogonimus* spp., urinary blood fluke (*Schistosoma haematobium*), oriental blood fluke (*Schistosoma japonicum*), *Schistosoma mansoni, Schistosoma* spp., *Trichobilharzia* spp., *Troglotrema* spp., and *Typhlocoelum* spp.,
nematodes (Nematoda); *Aelurostrongylus* spp., *Amidostomum* spp., *Ancylostoma* spp., *Angiostrongylus* spp., *Anisakis* spp., chicken roundworm *(Ascaridia galli), Ascaridia* spp., *Ascaris* spp., pig roundworm *(Ascaris suum), Aspiculuris* spp., *Brugia* spp., *Bunostomum* spp., *Capillaria* spp., *Chabertia* spp., intestinal worm (*Cooperia oncophora*)*, Cooperia* spp., *Crenosoma* spp., *Cyathostoma* spp., *Cyclococercus* spp., *Cylicostephanus* spp., *Cylindropharynx* spp., *Cystocaulus* spp., *Dictyocaulus* spp., dog filaria (*Dirofilaria immitis*), *Dirofilaria* spp., *Dracunculus* spp., *Draschia* spp., *Elaphostrongylus* spp., *Enoplida, Enterobius* spp., *Filaroides* spp., *Filicollis* spp., *Globocephalus* spp., *Gnathostoma* spp., *Gongylonema* spp., *Gyalocephalus* spp., *Habronema* spp., barber's pole worm (*Haemonchus contortus*), *Haemonchus* spp., *Heterakis* spp., *Hyostrongylus* spp., *Litomosoides* spp., *Loa* spp., *Macracanthorhynchus* spp., *Marshallagia* spp., *Metastrongylus* spp., *Micronema* spp., *Moniliformis* spp., *Muellerius* spp., *Nematodirus* spp., *Neostrongylus* spp., *Obeliscoides* spp., *Oeophagostomum dentatum, Oesophagodontus* spp., *Oesophagostomum radiatum, Oesophagostomum* spp., *Ollulanus* spp., *Onchocerca gibsoni, Onchocerca* spp., brown stomach worm (*Ostertagia ostertagi*), *Ostertagia* spp., *Oxyuris* spp., *Parabronema* spp., *Paracrenosoma* spp., *Parafilaria* spp., *Parafilaroides* spp., *Parascaris* spp., *Parelaphostrongylus* spp., *Passalurus* spp., *Physaloptera* spp., *Pneumostrongylus* spp., *Poteriostomum* spp., *Prostenorchis* spp., *Protostrongylus* spp., *Setaria* spp., *Spicocaulus* spp., *Stephanofilaria* spp., *Stephanurus* spp., *Strongyloides* spp., *Strongylus* spp., *Syngamus* spp., *Syphacia* spp., *Thelazia* spp., *Toxascaris* spp., *Toxocara* spp., *Trichinella* spp., *Trichomosoides* spp., Enoplidae (*Trichonema* spp.), ruminant nematode (*Trichostrongylus colubriformis*), *Trichostrongylus* spp., *Trichuris* spp., *Triodontophorus* spp., *Uncinaria* spp., and *Wuchereria* spp., and
protozoans (Protozoa); *Babesis* spp., *Eimeria acervulina, Eimeria bovis, Eimeria brunette, Eimeria maxima, Eimeria necatrix, Eimeria ovinoidalis, Eimeria* spp., *Eimeria tenella, Entamoeba histolytica, Giardia* spp., *Histomanas* spp., *Plasmodium* spp., *Theileria* spp., *Toxoplasma* spp., *Trichomonadidae* spp., and *Trypanosomsa cruzi.*

In addition, the presently invented compound is effective for pests that have developed resistance to an existing insecticide such as an organophosphorus compound, a carbamate compound, and a pyrethroid compound.

Specifically, the presently invented compound can effectively control pests belonging to insects such as Collembola, Dictyoptera (Blattodea), Orthoptera, Isoptera, Thysanoptera, Hemiptera (Heteroptera and Homoptera), Lepidoptera, Coleoptera, Hymenoptera, Diptera, Isoptera (Siphonaptera), and Phthiraptera, crustaceans such as Arguloida, Siphonostomatoida and Cyclopoida, mites, gastropods, cestodes, trematodes, nematodes, protozoans, and so on, at low concentration.

On the other hand, the presently invented compound has a very useful feature to cause almost no adverse effect on mammals, fishes, crustaceans, and beneficial insects (useful insects such as honeybees and bumblebees, and natural enemies such as Aphelinidae, Aphidiinae, tachinid fly, *Orius* spp., and Phytoseiidae).

In using the presently invented compound, typically, the presently invented compound is mixed with a proper solid carrier or liquid carrier with addition of a surfactant, a penetrant, a spreader, a thickening agent, an antifreezing agent, a binder, an anticaking agent, a disintegrant, a defoamer, a preservative, an antidegradant, or any other agent, as desired, and the resultant can be formulated into a formulation of an arbitrary dosage form such as a soluble concentrate, an emulsifiable concentrate, a wettable powder, a water-soluble powder, a water-dispersible granule, a water-soluble granule, a suspension concentrate, a concentrated emulsion, a suspoemulsion, a microemulsion, a dustable powder, a granule tablet, and an emulsifiable gel for practical use. For labor saving and enhanced safety, the formulation of an arbitrary dosage form can be encapsulated for use in a water-soluble capsule or a water-soluble package such as a bag of a water-soluble film.

Examples of the solid carrier include natural mineral materials such as quartz, calcite, meerschaum, dolomite, chalk, kaolinite, pyrophyllite, sericite, hallosite, metahallosite, kibushi clay, gairome clay, pottery stone, ZEEKLITE, allophane, shirasu, mica, talc, bentonite, activated clay, acid clay, pumice, attapulgite, zeolite, and diatomaceous earth; calcined products of natural mineral materials such as calcined clay, perlite, silas balloons, vermiculite, attapulgus clay, and calcined diatomaceous earth; inorganic salts such as magnesium carbonate, calcium carbonate, sodium carbonate, sodium hydrogen carbonate, ammonium sulfate, sodium sulfate, magnesium sulfate, diammonium hydrogen phosphate, ammonium dihydrogen phosphate, and potassium chloride; saccharides such as glucose, fructose, sucrose, and lactose; polysaccharides such as starch, powdered cellulose, and dextrin; organic substances such as urea, urea derivatives, benzoic acid, and salts of benzoic acid; plant materials such as wood powder, cork powder, corn rachises, walnut shells, and tobacco stems; fly ash, white carbon (e.g., hydrated synthetic silica, anhydrous synthetic silica, hydrated synthetic silicate), and fertilizers.

Examples of the liquid carrier include aromatic hydrocarbons such as xylene, alkyl (such as C₉ or C₁₀) benzene, phenylxylylethane, and alkyl (such as C₁ or C₃) naphthalene; aliphatic hydrocarbons such as machine oil, normal paraffin, isoparaffin, and naphthene; mixtures of aromatic hydrocarbon and aliphatic hydrocarbon such as kerosene; alcohols such as ethanol, isopropyl alcohol, cyclohexanol, phenoxyethanol, and benzyl alcohol; polyhydric alcohols such as ethylene glycol, propylene glycol, diethylene glycol, hexylene glycol, polyethylene glycol, and polypropylene glycol; ethers such as propylcellosolve, butylcellosolve, phenylcellosolve, propylene glycol monomethyl ether, propylene glycol monoethyl ether, propylene glycol monopropyl ether, propylene glycol monobutyl ether, and propylene glycol monophenyl ether; ketones such as acetophenone, cyclohexanone, and γ-butyrolactone; esters such as aliphatic acid methyl esters, succinic acid dialkyl esters, glutamic acid dialkyl esters, adipic acid dialkyl esters, and phthalic acid dialkyl esters; acid amides such as N-alkyl (such as C₁, C₈ or C₁₂) pyrrolidone; fats and oils such as soybean oil, linseed oil, rapeseed oil, coconut oil, cottonseed oil, and castor oil; and dimethyl sulfoxide and water.

One of those solid carriers and liquid carriers may be used singly; alternatively, two or more thereof may be used in combination.

Examples of the surfactant include nonionic surfactants such as polyoxyethylene alkyl ethers, polyoxyethylene alkyl (mono or di)phenyl ethers, polyoxyethylene (mono, di or tri)styrylphenyl ethers, polyoxyethylene polyoxypropylene block copolymers, polyoxyethylene fatty acid (mono or di)esters, sorbitan fatty acid esters, polyoxyethylene sorbitan fatty acid esters, a castor oil ethylene oxide adduct, acetylene glycol, acetylene alcohol, an ethylene oxide adduct of acetylene glycol, an ethylene oxide adduct of acetylene alcohol, and alkyl glycosides; anionic surfactants such as alkylsulfate salts, alkylbenzenesulfonic acid salts, ligninsulfonic acid salts, alkylsulfosuccinic acid salts, naphthalenesulfonic acid salts, alkylnaphthalenesulfonic acid salts, salts of a formalin condensate of naphthalenesulfonic acid, salts of a formalin condensate of alkylnaphthalenesulfonic acid, polyoxyethylene alkyl ether sulfonate or phosphate salts, polyoxyethylene (mono or di) alkylphenyl ether sulfonate or phosphate salts, polyoxyethylene (mono, di or tri) styrylphenyl ether sulfonate or phosphate salts, polycarboxylic acid salts (e.g., polyacrylic acid salts, polymaleic acid salts, copolymers of maleic acid and an olefin), and polystyrenesulfonic acid salts; cationic surfactants such as alkylamine salts and alkyl quaternary ammonium salts; amphoteric surfactants such as those of amino acid type and betaine type; and silicone surfactants, and fluorine-containing surfactants.

The surfactant content for those surfactants is not limited to a particular value, but desired to be in the range of 0.05 to 20 parts by mass relative to 100 parts by mass of the presently invented compound in typical cases. One of those surfactants may be used singly; alternatively, two or more thereof may be used in combination.

The amount of application of the presently invented compound depends on the situation of application, time of application, application method, cultivated crop, etc., and about 0.005 to 50 kg per hectare (ha), as an amount of the active ingredient, is appropriate in typical cases.

In using the presently invented compound for controlling internal or external parasites in mammals and birds as livestock and pets, an effective amount of the presently invented compound can be administered together with formulation excipients, for example, by: oral administration; parenteral administration such as injection (intramuscular, subcutaneous, intravenous, intraperitoneal, etc.); transdermal administration such as soaking, spraying, bathing, washing, pouring-on and spotting-on, and dusting; or transnasal administration. The presently invented compound can be administered in the form of a shaped product with a slip, a plate, a band, a collar, an ear mark, a limb band, an indicator, or the like.

In administration, the presently invented compound can be formulated into an arbitrary dosage form suitable for the route of administration.

In eliminating an external or internal parasite with the presently invented compound, the preferred dose of presently invented compound (1) depends on the type of the target parasite to be controlled, the species of the target animal to be subjected to administration, the administration method, or any other factor, and is 0.01 to 100 mg/kg, and preferably 0.01 to 50 mg/kg per body weight of the target animal to be subjected to administration in typical cases. In particular, the dose for dogs, which can depend on the breed or age of the targeted dog or the external parasite to be controlled, is typically 1 to 5000 mg/kg, and preferably 1 to 100 mg/kg per 1 kg fresh weight of the targeted dog.

In eliminating an external or internal parasite by administering the presently invented compound, the frequency of administration depends on the type of the target parasite to be controlled, the species of the target animal to be subjected to administration, the administration method, or any other factor, and can be arbitrarily set in the range, from every day to once a year in typical cases. The frequency of administration is preferably once a week to once per six months, and more preferably every day (every 24 hours), every month, once a month, once every two months, once every three months, or once every six months.

For time of administration to a dog in using the presently invented compound for controlling an external parasite in a dog, for example, the presently invented compound is orally administered to a dog at a time immediately 30 minutes before the beginning of feeding or 120 minutes after the end of feeding. The time 30 minutes before the beginning of feeding and the time 120 minutes after the end of feeding are based on an act of eating a feed given to a dog for the purpose of nutrient intake. In the case that a dog is allowed to have a feeding time of 20 minutes, for example, the specified time is between the time 30 minutes before the beginning of feeding and the time 120 minutes after the end of feeding, within 170 minutes in total, on the basis of an act of having a meal. Also included is the case that feeding is temporarily suspended in the middle of the feeding, the presently invented compound is orally administered, and then feeding is restarted. The word feeding herein refers to an act of eating a feed by an animal.

The number of the feeding of a dog in a day depends on dog breed, age, and habit, and is typically said to be three to four times per day for dogs younger than half a year of age, twice to three times per day for dogs older than half a year of age and younger than a year of age, twice per day for about one- to five-year old adult dogs, and about twice to three times per day for six-year-old or older senior dogs. In the present invention, the word feeding refers to an act of eating for the purpose of taking nutrients, and an act of giving a feed or the like that is intended to be given for what is called teaching or training a dog is not included.

Examples of the arbitrary dosage form to be prepared include solid preparations such as powders, granules, wettable powders, pellets, tablets, boluses, capsules, and shaped products containing the active compound; liquid preparations such as solutions and emulsions for injection, solutions and emulsions for oral administration, and solutions and emulsions for use on skin or in a body cavity; solution preparations such as pour-on agents, spot-on agents, suspension concentrates, and emulsions; and semi-solid preparations such as salves and ointments and gels.

Examples of the dosage form for oral administration of the presently invented compound include solid preparations such as tablets, chewables, capsules, pills, boluses, granules, and powders; semi-solid preparations such as pastes and gels, and liquid preparations such as drinks.

Examples of the dosage form for transdermal administration include solid preparations such as powders; semi-solid preparations such as creams, salves and ointments, pastes, and gels; and liquid preparations such as sprays, aerosols, solutions and emulsions, suspensions, and lotions.

Examples of the dosage form for administration by injection include liquid preparations such as solutions and emulsions and suspensions, and examples of the dosage form for transnasal administration include liquid preparations such as aerosols.

Examples of the dosage form for sprinkling in an animal-raring environment such as livestock sheds include solid preparations such as wettable powders, dusts, and granules; and liquid preparations such as emulsions and suspension concentrates.

It should be noted that formulations to be used for the parasiticide of the present invention are not limited to those dosage forms.

A solid preparation can be directly orally administered, or transdermally administered after diluting with water, or used, for example, by sprinkling in an animal-raring environment such as livestock sheds.

A solid preparation to be used for oral administration can be prepared by mixing presently invented compound (1) or a salt thereof and one or two or more diluents and binders suitable for oral administration, and, if necessary, a physiologically acceptable excipient such as a lubricant, a disintegrant, a dye, and a pigment, and shaping the resultant to give a desired shape.

Examples of the diluents and binders include saccharides or saccharide derivatives such as lactose, sucrose, mannitol, and sorbitol; starches such as corn starch, wheat starch, rice starch, and potato starch; celluloses or cellulose derivatives such as methylcellulose, carboxymethylcellulose, hydroxyethylcellulose, hydroxypropylcellulose, and hydroxypropylmethylcellulose; proteins or protein derivatives such as zein and gelatin; and honey, gum arabic mucilage, and synthetic polymer compounds such as polyvinyl alcohol and polyvinylpyrrolidone.

Examples of the lubricant include magnesium stearate, and examples of the disintegrant include cellulose, agar, alginic acid, crosslinked polyvinylpyrrolidinone, and carbonic acid salts.

In particular, for solid agents such as chewable agents among solid preparations for use in oral administration, excipients that impart taste, texture, and flavor preferred by animals to be subjected to administration are also used, but carriers and excipients for use in solid preparations of the pesticide composition are not limited thereto.

For example, a liquid preparation can be directly administered transdermally or by injection, or mixed with an animal feed and orally administered, or orally administered after dilution with water, or transdermally administered, or sprinkled in an animal-raring environment such as livestock sheds.

A solution or emulsion for injection can be intravenously, intramuscularly, or subcutaneously administered. A solution or emulsion for injection can be prepared by dissolving the active compound in a proper solvent and, if necessary, adding an excipient such as a solubilizer, an acid, a base, a buffering salt, an antioxidant, and a protectant.

Examples of the proper solvent include water, ethanol, butanol, benzyl alcohol, glycerin, propylene glycol, polyethylene glycol, N-methylpyrrolidone, a mixture of any of them, physiologically acceptable vegetable oil, and synthetic oil suitable for injection.

Examples of the solubilizer include polyvinylpyrrolidone, polyoxyethylated castor oil, and polyoxyethylated sorbitan ester.

Examples of the protectant include benzyl alcohol, trichlorobutanol, p-hydroxybenzoic acid ester, and n-butanol.

A solution or emulsion for oral administration can be administered directly or with dilution. A solution or emulsion for oral administration can be prepared in the same manner as for the solution or emulsion for injection.

A suspension concentrate, an emulsion, or the like can be administered directly or with dilution, transdermally, or by environmental process.

A solution or emulsion for use on skin can be administered by dropping, spreading, rubbing, spraying, or sprinkling, or applying by soaking (soaking, bathing or washing). Such a solution or emulsion can be prepared in the same manner as for the solution or emulsion for injection.

A pour-on agent and spot-on agent can be allowed to systemically act by dropping at a limited position on skin or spraying thereon to soak the skin with the active compound.

A pour-on agent and spot-on agent can be prepared by dissolving, suspending, or emulsifying active ingredients in a proper skin-compatible solvent or solvent mixture. If necessary, an auxiliary agent such as a surfactant, a coloring agent, an absorption promoter, an antioxidant, a photostabilizer, and an adhesive may be added.

Examples of the proper solvent include water, alkanol, glycol, polyethylene glycol, polypropylene glycol, glycerin, benzyl alcohol, phenylethanol, phenoxyethanol, ethyl acetate, butyl acetate, benzyl benzoate, dipropylene glycol monomethyl ether, diethylene glycol monobutyl ether, acetone, methyl ethyl ketone, aromatic and/or aliphatic hydrocarbons, vegetable or synthetic oil, DMF (N,N-dimethylformamide), liquid paraffin, light liquid paraffin, silicone, dimethylacetamide, and N-methylpyrrolidone or 2,2-dimethyl-4-oxy-methylene-1,3-dioxolane.

Examples of the absorption promoter include DMSO (dimethyl sulfoxide), isopropyl myristate, dipropylene glycol pelargonate, silicone oil, aliphatic esters, triglycerides, and fatty alcohols.

Examples of the antioxidant include sulfurous acid salts, metabisulfurous acid salts, ascorbic acid, butylhydroxytoluene, butylhydroxyanisole, and tocopherol.

An emulsion can be administered through oral administration, transdermal administration, or injection. An emulsion can be prepared by dissolving active ingredients in a hydrophobic phase or hydrophilic phase, and homogenizing the resultant with a solvent of another phase by using a proper emulsifying agent with further addition of an auxiliary agent such as a coloring agent, an absorption promoter, a protectant, an antioxidant, a shading agent, and a thickening substance, if necessary.

Examples of the hydrophobic phase (oil) include paraffin oil, silicone oil, sesame oil, almond oil, castor oil, synthetic triglyceride, ethyl stearate, di-n-butyl adipate, hexyl laurate, dipropylene glycol pelargonate, esters of branched short-chain fatty acid and saturated fatty acid having a chain length of C16 to C18, isopropyl myristate, isopropyl palmitate, caprylates/caprates of saturated aliphatic alcohol having a chain length of C12 to C18, isopropyl stearate, oleyl oleate, decyl oleate, ethyl oleate, ethyl lactate, wax-like fatty acid esters, dibutyl phthalate, diisopropyl adipate, isotridecyl alcohol, 2-octyldodecanol, cetylstearyl alcohol, and oleyl alcohol.

Examples of the hydrophilic phase include water, propylene glycol, glycerin, and sorbitol.

Examples of the emulsifying agent include nonionic surfactants such as polyoxyethylated castor oil, polyoxyethylated sorbitan monoolefinate, sorbitan monostearate, glycerin monostearate, polyoxyethyl stearate, and alkylphenol polyglycol ether; amphoteric surfactants such disodium N-lauryl-β-iminodipropionate and lecithin; anionic surfactants such as sodium laurylsulfate, fatty alcohol sulfuric ether, and monoethanolamine salts of mono/dialkyl polyglycol orthophosphate; and cationic surfactants such as cetyltrimethylammonium chloride.

Examples of other auxiliary agents include carboxymethylcellulose, methylcellulose, polyacrylate, arginate, gelatin, gum arabic, polyvinylpyrrolidone, polyvinyl alcohol, methyl vinyl ether, copolymers with maleic anhydride, polyethylene glycol, waxes, and colloidal silica.

A semi-solid preparation can be administered by applying to or spreading on skin, or by introducing into a body cavity. A gel can be prepared by adding a sufficient amount of a thickener for generating a transparent substance having ointment-like viscosity to a solution prepared as described above for the solution or emulsion for injection.

Next, formulation examples with the presently invented compound are shown. However, blend examples of formulations containing the presently invented compound are not limited thereto. In the following blend examples, the word "part" means part by mass.

### [Wettable powder]

Presently invented compound: 0.1 to 80 parts
Solid carrier: 5 to 98.9 parts
Surfactant: 1 to 10 parts
Others: 0 to 5 parts

Examples of "Others" include an anticaking agent and an antidegradant.

### [Emulsion]

Presently invented compound: 0.1 to 30 parts
Liquid carrier: 45 to 95 parts
Surfactant: 4.9 to 15 parts
Others: 0 to 10 parts

Examples of "Others" include a spreader and an antidegradant.

### [Suspension]

Presently invented compound: 0.1 to 70 parts
Liquid carrier: 15 to 98.89 parts
Surfactant: 1 to 12 parts
Others: 0.01 to 30 parts

Examples of "Others" include an antifreezing agent and a thickening agent.

### [Granular wettable powder]

Presently invented compound: 0.1 to 90 parts
Solid carrier: 0 to 98.9 parts
Surfactant: 1 to 20 parts
Others: 0 to 10 parts

Examples of "Others" include a binder and an antidegradant.

### [Solution or emulsion]

Presently invented compound: 0.01 to 70 parts
Liquid carrier: 20 to 99.99 parts
Others: 0 to 10 parts

Examples of "Others" include an antifreezing agent and a spreader.

### [Granule]

Presently invented compound: 0.01 to 80 parts
Solid carrier: 10 to 99.99 parts
Others: 0 to 10 parts

Examples of "Others" include a binder and an antidegradant.

### [Powder]

Presently invented compound: 0.01 to 30 parts
Solid carrier: 65 to 99.99 parts
Others: 0 to 5 parts

Examples of "Others" include an anti-drift agent and an antidegradant.

Next, formulation examples with the presently invented compound as an active ingredient are specifically shown, but the present invention is not limited thereto. Presently invented compound No. 1-001 shown in the following refers to N-(4-fluorophenyl)-6-[3-(6-fluoropyridin-3-yl)-4-(trifluoromethyl)phenyl]-4-hydroxy-2-oxo-1,2,5,6-tetrahydropyridine-3-carboxamide shown later in Synthesis Example 1.

### [Blend Example 1] Wettable powder

Presently invented compound No. 1-001: 20 parts
Pyrophyllite: 74 parts
SORPOL 5039: 4 parts
(product name, TOHO Chemical Industry Co., Ltd., mixture of nonionic surfactant and anionic surfactant)
CARPLEX #80D: 2 parts
(product name, Shionogi & Co., Ltd., synthetic hydrated silicate)

These are homogenously mixed and pulverized to give a wettable powder.

### [Blend Example 2] Emulsion

Presently invented compound No. 1-001: 5 parts
Xylene: 75 parts
N-Methylpyrrolidone: 15 parts
SORPOL 2680: 5 parts
(product name, TOHO Chemical Industry Co., Ltd., mixture of nonionic surfactant and anionic surfactant)

These are homogenously mixed to give an emulsion.

### [Blend Example 3] Suspension

Presently invented compound No. 1-001: 25 parts
AGRISOL S-710: 10 parts
(product name, Kao Corporation, nonionic surfactant)
RUNOX 1000C: 0.5 parts
(product name, TOHO Chemical Industry Co., Ltd., anionic surfactant)
Xanthan gum: 0.2 parts
Water: 64.3 parts

These are homogenously mixed, and then wet-pulverized to give a suspension.

### [Blend Example 4] Granular wettable powder

Presently invented compound No. 1-001: 75 parts
HITENOL NE-15: 5 parts
(Product name, DKS Co. Ltd., anionic surfactant)
VANILLEX N: 10 parts
(product name, Nippon Paper Industries Co., Ltd., anionic surfactant)
CARPLEX #80D: 10 parts
(product name, Shionogi & Co., Ltd., synthetic hydrated silicate)

These are homogenously mixed and pulverized, a small amount of water is then added, the mixture is stirred and mixed and granulated with an extrusion granulator, and the resultant is dried to give a granular wettable powder.

### [Blend Example 5] Granule

Presently invented compound No. 1-001: 5 parts
Bentonite: 50 parts
Talc: 45 parts

These are homogenously mixed and pulverized, a small amount of water is then added, the mixture is stirred and mixed and granulated with an extrusion granulator, and the resultant is dried to give a granule.

### [Blend Example 6] Powder

Presently invented compound No. 1-001: 3 parts
CARPLEX #80D: 0.5 parts
(product name, Shionogi & Co., Ltd., synthetic hydrated silicate)
Kaolinite: 95 parts
Diisopropyl phosphate: 1.5 parts

These are homogeneously mixed and pulverized to give a powder.

In use, the formulation is sprinkled after 1- to 10000-fold dilution with water, or directly sprinkled without dilution.

### [Blend Example 7] Wettable powder preparation

Presently invented compound No. 1-001: 25 parts
Sodium diisobutylnaphthalenesulfonate: 1 part
Calcium n-dodecylbenzenesulfonate: 10 parts
Alkylaryl poly glycol ether: 12 parts
Sodium salt of naphthalenesulfonic acid-formalin condensate: 3 parts
Emulsion-type silicone: 1 part
Silicon dioxide: 3 parts
Kaolin: 45 parts

### [Blend Example 8] Water-soluble thick agent preparation

Presently invented compound No. 1-001: 20 parts
Polyoxyethylene lauryl ether: 3 parts
Sodium dioctylsulfosuccinate: 3.5 parts
Dimethyl sulfoxide: 37 parts
2-Propanol: 36.5 parts

### [Blend Example 9] Solution or emulsion for spraying

Presently invented compound No. 1-001: 2 parts
Dimethyl sulfoxide: 10 parts
2-Propanol: 35 parts
Acetone: 53 parts

### [Blend Example 10] Solution or emulsion for transdermal administration

Presently invented compound No. 1-001: 5 parts
Hexylene glycol: 50 parts
2-Propanol: 45 parts

### [Blend Example 11] Solution or emulsion for transdermal administration

Presently invented compound No. 1-001: 5 parts
Propylene glycol monomethyl ether: 50 parts
Dipropylene glycol: 45 parts

### [Blend Example 12] Solution or emulsion for transdermal administration (pour-on)

Presently invented compound No. 1-001: 2 parts
Light liquid paraffin: 98 parts

### [Blend Example 13] Solution or emulsion for transdermal administration (pour-on)

Presently invented compound No. 1-001: 2 parts
Light liquid paraffin: 58 parts
Olive oil: 30 parts
O.D.O: 9 parts
(product name, The Nisshin OilliO Group, Ltd., medium-chain fatty acid oil)
Shin-Etsu Silicone: 1 part

In using as an agrochemical or a veterinary drug, the presently invented compound may be mixed with a chemical of other type in formulation or application, as necessary. Application of a mixture is expected to result in cost reduction due to reduction in the amount of application, and extension of the control spectrum and higher pesticidal effect due to the synergistic action of the mixed chemical. In this case, it is even possible to combine with multiple known chemicals at the same time.

Examples of the type of agrochemical to be mixed with the presently invented compound for use include compounds described in The Pesticide Manual, 18th ed., 2018 and The Pesticide Manual, 19th ed., 2021. Examples of the specific common names of the compounds, together with antiparasitic drugs and antibiotics to be mixed for use as a veterinary drug, are as follow, but not necessarily limited thereto.

Insecticides: abamectin, acephate, acequinocyl, acetamiprid, acrinathrin, acynonapyr, afidopyropen, afoxolaner, alanycarb, aldicarb, allethrin, alpha-cypermethrin, alpha-endosulfan, amidoflumet, amitraz, azamethiphos, azinphos-ethyl, azinphos-methyl, azocyclotin, *Bacillus thuringiensis,* bendiocarb, benfluthrin, benfuracarb, bensultap, benzoximate, benzpyrimoxan, beta-cyfluthrin, beta-cypermethrin, bifenazate, bifenthrin, bioallethrin, bioresmethrin, bistrifluron, broflanilide, bromopropylate, buprofezin, butocarboxim, carbaryl, carbofuran, carbosulfan, cartap, chinomethionat, chlorantraniliprole, chlorethoxyfos, chlorfenapyr, chlorfenvinphos, chlorfluazuron, chlormephos, chlorobenzilate, chloroprallethrin, chlorpyrifos, chlorpyrifos-methyl, chromafenozide, clofentezine, clothianidin, cyanophos, cyantraniliprole, cyclaniliprole, cycloprothrin, cyenopyrafen, cyetpyrafen, cyflumetofen, cyfluthrin, cyhalodiamide, cyhalothrin, cyhexatin, cypermethrin, cyphenothrin, cyproflanilide, cyromazine, deltamethrin, diacloden, diafenthiuron, diazinon, dichlorvos, dicloromezotiaz, dicofol, dienochlor, diflovidazin, diflubenzuron, dimefluthrin, dimethoate, dimethylvinphos, dimpropyridaz, dinotefuran, diofenolan, disulfoton, DNOC, d-T-80-phthalthrin (d-tetramethrin), emamectin-benzoate, empenthrin, endosulfan, EPN, epsilon-metofluthrin, epsilon-momfluorothrin, esfenvalerate, ethiofencarb, ethiprole, etofenprox, etoxazole, etrimfos, Febantel, fenazaquin, fenbutatin oxide, fenitrothion, fenmezoditiaz, fenobucarb, fenothiocarb, fenoxycarb, fenpropathrin, fenpyroximate, fenthion, fenvalerate, fipronil, flometoquin, flonicamid, fluacrypyrim, fluazuron, flubendiamide, fluchlordiniliprole, flucycloxuron, flucythrinate, flufenerim, flufenoxuron, flufenprox, flufiprole, fluhexafon, flumethrin, flupentiofenox, flupyradifurone, flupyrimin, flupyroxystrobin, fluralaner, fluvalinate, fluxametamide, fonophos, formetanate, formothion, furathiocarb, gamma-cyhalothrin, halfenprox, halofenozide, heptafluthrin, hexaflumuron, hexythiazox, hydramethylnon, imidacloprid, imiprothrin, indazapyroxamet, indoxacarb, indoxacarb-MP, isocycloseram, isofenphos, isoprocarb, isoxathion, kappa-bifenthrin, kappa-tefluthrin, lambda-cyhalothrin, ledprona, lepimectin, lufenuron, malathion, meperfluthrin, metaflumizone, metalcarb, metaldehyde, methacrifos, methamidophos, methidathion, methomyl, methoprene, methoxychlor, methoxyfenozide, methyl bromide, metofluthrin, milbemectin, momfluorothrin, monocrotophos, muscalure, nicofluprole, nitenpyram, novaluron, noviflumuron, omethoate, oxazosulfyl, oxydemeton-methyl, oxydeprofos, parathion, parathion-methyl, pentachlorophenol, permethrin, phenothrin, phenthoate, phorate, phosalone, phosmet, phosphamidon, phoxim, pirimicarb, pirimiphos-methyl, praziquantel, profenofos, profluthrin, propaphos, propargite, prothiofos, protrifenbute, pyflubumide, pymetrozine, pyraclofos, pyrafluprole, pyrethrins, pyridaben, pyridalyl, pyrifluquinazon, pyrimidifen, pyriprole, pyriproxyfen, resmethrin, rotenone, silafluofen, spidoxamat, spinetoram, spinosad, spirobudifen, spirodiclofen, spiromesifen, spiropidion, spirotetramat, spiromesifen, sulfiflumin, sulfotep, sulfoxaflor, sulprofos, tau-fluvalinate, tebfenozide, tebufenpyrad, teflubenzuron, tefluthrin, terbufos, tetrachlorantraniliprole, tetrachlorvinphos, tetramethrin, tetramethylfluthrin, tetraniliprole, thiacloprid, thiamethoxam, thiocyclam, thiodicarb, thiofanox, thiometon, tiorantraniliprole, tolfenpyrad, tralomethrin, transfluthrin, triazamate, triazuron, trichlorfon, triflumezopyrim, triflumuron, tyclopyrazoflor, vamidothion, and zeta-cypermethrin, and so on.

Microbicides: acibenzolar-S-methyl, acypetacs, aldimorph, allyl alcohol, ametoctradin, aminopyrifen, amisulbrom, amobam, ampropylfos, anilazine, azaconazole, azithiram, azoxystrobin, barium polysulfide, benalaxyl, benalaxyl-M, benodanil, benomyl, benquinox, bentaluron, benthiavalicarb-isopropyl, benthiazole, benzamacril, benzamorf, benzovindiflupyr, binapacryl, biphenyl, bitertanol, bixafen, blasticidin-S, bordeaux mixture, boscalid, bromuconazole, bupirimate, buthiobate, butylamine, calcium polysulfide, captafol, captan, carbamorph, carbendazim, carboxin, carpropamid, carvone, cheshunt mixture, chinomethionat, chlobenthiazone, chloraniformethane, chloranil, chlorfenazole, chloroneb, chloroinconazide, chloropicrin, chlorothalonil, chlorquinox, chlozolinate, climbazole, copper acetate, copper carbonate basic, copper hydroxide, copper naphthenate, copper oleate, basic copper chloride (copper oxychloride), copper sulfate, basic copper sulfate, copper zinc chromate, coumoxystrobin, cresol, cufraneb, cuprobam, cyazofamid, cyclafuramid, cycloheximide, cyflufenamid, cymoxanil, cypendazole, cyproconazole, cyprodinil, cyprofuram, dazomet, debacarb, decafentin, dehydroacetic acid, dichlobentiazox, dichlofluanid, dichlone, dichlorophen, dichlozoline, diclobutrazol, diclocymet, diclomezine, dicloran, diethofencarb, difenoconazole, diflumetorim, dimethirimol, dimethomorph, dimoxystrobin, diniconazole, diniconazole-M, dinobuton, dinocap, dinocap-4, dinocap-6, dinocton, dinosulfon, dinoterbon, diphenylamine, dipymetitrone, dipyrithione, disulfiram, ditalimfos, dithianon, DNOC, dodemorph, dodine, drazoxolon, edifenphos, enestrobin, enoxastrobin, epoxiconazole, ethaboxam, etaconazole, etem, ethirimol, ethoxyquin, etridiazole, famoxadone, fenamidone, fenaminosulf, fenaminstrobin, fenapanil, fenarimol, fenbuconazole, fenfuram, fenhexamid, fenitropan, fenoxanil, fenpiclonil, fenpicoxamid, fenpropidin, fenpropimorph, fenpyrazamine, fentin, ferbam, ferimzone, florylpicoxamid, fluazinam, flubeneteram, fludioxonil, flufenoxadiazam, flufenoxystrobin, fluindapyr, flumetylsulforim, flumetover, flumorph, fluopicolide, fluopimomide, fluopyram, fluoroimide, fluotrimazole, fluoxapiprolin, fluoxastrobin, fluoxytioconazole, fluquinconazole, flusilazole, flusulfamide, flutolanil, flutianil, flutriafol, fluxapyroxad, folpet, fosetyl-aluminium, fthalide, fuberidazole, furalaxyl, furametpyr, furcarbanil, furconazole, furconazole-cis, furmecyclox, furophanate, glyodin, griseofulvin, guazatine, halacrinate, hexachlorobenzene, hexaconazole, hexylthiofos, 8-hydroxyquinoline sulfate, hymexazol, imazalil, imibenconazole, iminoctadine-albesilate, iminoctadine-triacetate, inpyrfluxam, iodocarb, ipconazole, ipfentrifluconazole, ipflufenoquin, iprobenfos, iprodione, iprovalicarb, isofetamid, isoflucypram, isotianil, isoprothiolane, isopyrazam, isovaledione, kasugamycin, kresoxim-methyl, laminarin, mancopper, mancozeb, mandestrobin, mandipropamid, maneb, mebenil, mecarbinzid, mefentrifluconazole, mepanipyrim, mepronil, meptyldinocap, metalaxyl, metalaxyl-M, metam, metarylpicoxamid, metazoxolon, metconazole, methasulfocarb, methfuroxam, metyltetraprole, metiram, metominostrobin, metrafenone, metsulfovax, milneb, myclobutanil, myclozolin, nabam, naftifine, natamycin, organonickel (nickel bis(dimethyldithiocarbamate), nitrostyrene, nitrothal-isopropyl, nuarimol, octhilinone, ofurace, orysastrobin, oxadixyl, oxathiapiprolin, oxyquinoline copper (oxine copper), oxpoconazole fumarate, oxycarboxin, pefurazoate, penconazole, pencycuron, penflufen, pentachlorophenol, penthiopyrad, 2-phenylphenol, phosdiphen, phthalide, picarbutrazox, picoxystrobin, piperalin, polycarbamate, polyoxins, polyoxorim, potassium azide, potassium hydrogen carbonate, probenazole, prochloraz, procymidone, propamocarb hydrochloride, propiconazole, propineb, proquinazid, prothiocarb, pyrazophos, pyribencarb, pyrifenox, pyrimethanil, pyriminostrobin, pyroquilon, prothiocarb, prothioconazole, pydiflumetofen, pyracarbolid, pyraclostrobin, pyrametostrobin, pyraoxystrobin, pyrapropoyne, pyraziflumid, pyridachlometyl, pyridinitril, pyriofenone, pyrisoxazole, pyroxychlor, pyroxyfur, quinacetol-sulfate, quinazamid, quinconazole, quinoxyfen, quinofumelin, quintozene, rabenzazole, salicylanilide, seboctylamine, sedaxane, silthiofam, simeconazole, sodium hydrogen carbonate, sodium hypochlorite, spiroxamine, sulfur, tebuconazole, tebufloquin, tecloftalam, tecnazene, tecoram, tetraconazole, thiabendazole, thiadifluor, thicyofen, thifluzamide, thiochlorfenphim, thiophanate, thiophanate-methyl, thiram, tiadinil, tioxymid, tolclofos-methyl, tolprocarb, tolylfluanid, triadimefon, triadimenol, triamiphos, triarimol, triazbutil, triazoxide, tributyltin oxide, trichlamide, triclopyricarb, tricyclazole, tridemorph, trifloxystrobin, triflumizole, triforine, triticonazole, validamycin, valifenalate, vinclozolin, zarilamid, zinc naphthenate, zinc sulfate, zineb, ziram, zoxamide, shiitake mycelium extract, and shiitake fruiting body extract, and so on.

Nematicides: aldoxycarb, benclothiaz, cadusafos, cyclobutrifluram, DBCP, dichlofenthion, DSP, ethoprophos, fenamiphos, fensulfothion, fluazaindolizine, fluensulfone, fosthiazate, fosthietan, imicyafos, isamidofos, isazofos, oxamyl, thionazin, tioxazafen, and trifluenfuronate, and so on.

Drugs for parasites: esfenvalerate, fenpropathrin, fenvalerate, cypermethrin, bifenthrin, cypermethrin, deltamethrin, etofenprox, lambda-cyhalothrin, permethrin, tefluthrin, zeta-cypermethrin, acetamiprid, clothianidin, dinotefuran, imidacloprid, nitenpyram, thiamethoxam, chromafenozide, fenoxycarb, lufenuron, methoprene, pyriproxyfen, triflumuron, chlorpyrifos, chlorpyrifos-methyl, diazinon, dichlorvos, fenitrothion, fenthion, malathion, pirimiphos-methyl, tetrachlorvinphos, ethiprole, fipronil, propoxur, carbaryl, bendiocarb, metoxadiazone, fenobucarb, carbofuran, afoxolaner, fluralaner, fluxametamide, sarolaner, lotilaner, tigolaner, esafoxolaner, modoflaner, umifoxolaner, mivorilaner, avermectin, ivermectin, doramectin, eprinomectin, maduramycin, milbemycin, milbemycin oxime, moxidectin, selamectin, indoxacarb, amitraz, bistrifluron, spinosad, albendazole, atovaquone, bithionol, cambendazole, carnidazole, chloroquine, clazuril, clorsulon, closantel, coumaphos, dichlorophen, diethylcarbamazine, diminazene, dinitolmide, dithiazanine iodide, emodepside, epsiprantel, febantel, fenbendazole, flubendazole, glycalpyramide, imidocarb, levamisole, mebendazole, mebendazole, mefloquine hydrochloride, melarsamine hydrochloride, metronidazole, metyridine, monepantel, morantel tartrate, niclosamide, oxantel pamoate, oxantel tartrate, oxibendazole, oxyclozanide, piperazine adipate, piperazine citrate, piperazine phosphate, praziquantel, pyrantel pamoate, rafoxanide, tetramisole hydrochloride, thiabendazole, and triclabendazole, and so on.

Antibiotics: amoxicillin, ampicillin, cefapirin, cefazolin sodium, cefquinome, ceftiofur, penicillin, chlortetracycline, oxytetracycline, danofloxacin, difloxacin, oxolinic acid, enrofloxacin, florfenicol, lincomycin, lomefloxacin, marbofloxacin, miloxacin, mirosamycin, norfloxacin, ofloxacin, orbifloxacin, valnemulin, thiamphenicol, tiamulin fumarate, tilmicosin phosphate, acetylisovaleryltylosin, tylosin phosphate, tulathromycin, ketoconazole, miconazole nitrate, and clavulanic acid, and so on.

The presently invented compound has superior insecticidal and acaricidal activity against many noxious insects in agriculture, spider mites, and internal or external parasites in mammals or birds, and also exerts sufficient control effect against noxious insects that have acquired resistance to existing insecticides. In addition, the presently invented compound causes almost no adverse effect on mammals, fishes, and beneficial insects, and has low persistence with light environmental loads. Accordingly, the present invention can provide novel useful pesticides.

Based on the preceding description, embodiments of the present invention relates to [1] to [83] in the following.
[1] An aryltetrahydropyridine compound or a salt thereof, the aryltetrahydropyridine compound represented by a formula (1): 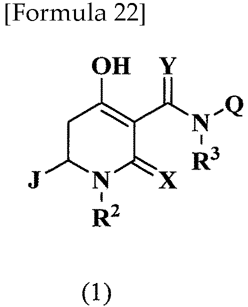 wherein
   Q represents Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, Q-37, Q-38, Q-39, Q-40, Q-41, Q-42, Q-43, Q-44, Q-45, Q-46, Q-47, Q-48, Q-49, Q-50, Q-51, Q-52, Q-53, Q-54, Q-55 or Q-56, 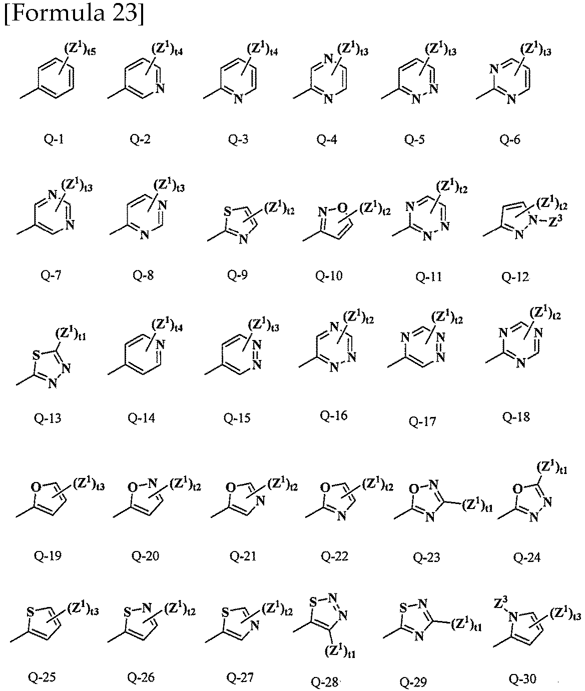 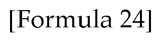 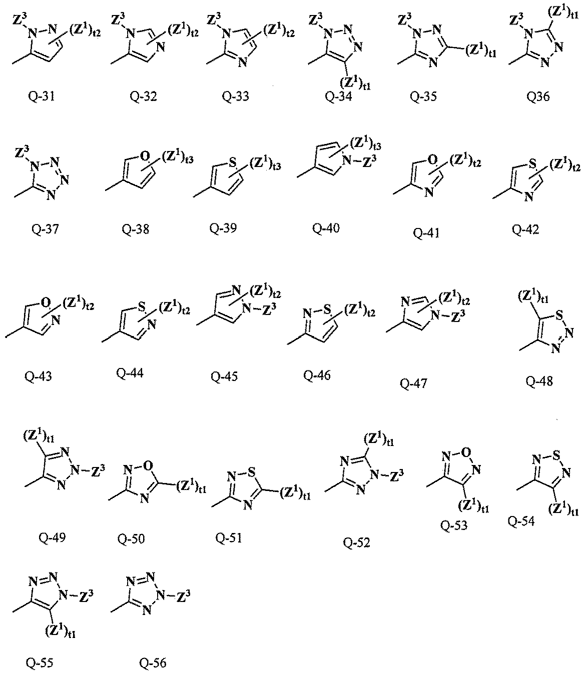
   Z¹ represents a halogen atom, C₁-C₆ alkyl, C₂-C₆ alkenyl, C₂-C₆ alkynyl, halo (C₁-C₆) alkyl, halo (C₂-C₆) alkenyl, halo (C₂-C₆) alkynyl, C₁-C₆ alkoxy, halo (C₁-C₆) alkoxy, C₂-C₆ alkoxy (C₁-C₆) alkoxy, C₂-C₆ alkenyloxy, halo (C₂-C₆) alkenyloxy, C₂-C₆ alkynyloxy, halo (C₂-C₆) alkynyloxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, C₂-C₆ alkenylthio, C₂-C₆ alkenylsulfinyl, C₂-C₆ alkenylsulfonyl, halo (C₂-C₆) alkenylthio, halo (C₂-C₆) alkenylsulfinyl, halo (C₂-C₆) alkenylsulfonyl, C₂-C₆ alkynylthio, C₂-C₆ alkynylsulfinyl, C₂-C₆ alkynylsulfonyl, halo (C₂-C₆) alkynylthio, halo (C₂-C₆) alkynylsulfinyl, halo (C₂-C₆) alkynylsulfonyl, -NH₂, C₁-C₆ alkylamino, di (C₁-C₆) alkylamino, halo (C₁-C₆) alkylamino, dihalo (C₁-C₆) alkylamino, formyl, carboxy, C₁-C₆ alkylcarbonyl, halo (C₁-C₆) alkylcarbonyl, C₁-C₆ alkoxycarbonyl, halo (C₁-C₆) alkoxycarbonyl, C₁-C₆ alkylaminocarbonyl, halo (C₁-C₆) alkylaminocarbonyl, di (C₁-C₆) alkylaminocarbonyl, hydroxy, C₁-C₆ alkylcarbonyloxy, C₁-C₆ alkoxycarbonyloxy, C₁-C₆ alkylsulfonyloxy, halo (C₁-C₆) alkylsulfonyloxy, -SO₃H, C₁-C₆ alkylaminosulfonyl, di (C₁-C₆) alkylaminosulfonyl, -C(=NOR¹²)R¹³, cyano or nitro,
   R¹² represents a hydrogen atom or C₁-C₆ alkyl,
   R¹³ represents a hydrogen atom or C₁-C₆ alkyl,
   Z³ represents a hydrogen atom or C₁-C₆ alkyl,
   J represents J-1 or J-2, 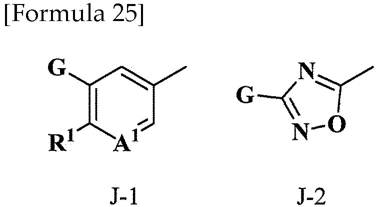
   A¹ represents a nitrogen atom or CH,
   G represents G-1, G-2, G-3, G-4, G-5, G-6, G-7, G-8, G-9, G-10, G-11, G-12, G-13, G-14, G-15, G-16, G-17, G-18, G-19, G-20, G-21, G-22, G-23, G-24, G-25, G-26, G-27, G-28, G-29, G-30, G-31, G-32, G-33, G-34, G-35, G-36, G-37, G-38, G-39, G-40, G-41, G-42, G-43, G-44, G-45, G-46, G-47, G-48, G-49, G-50, G-51, G-52, G-53, G-54, G-55, G-56, G-57, G-58, G-59, G-60 or G-61, 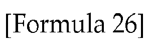 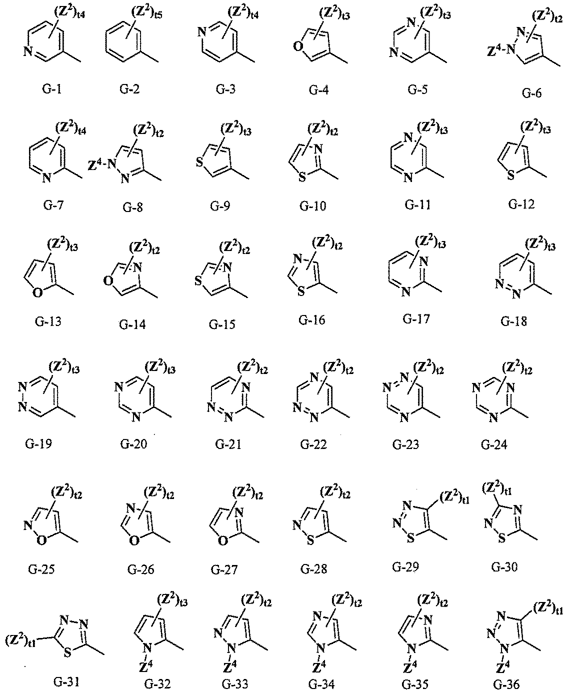 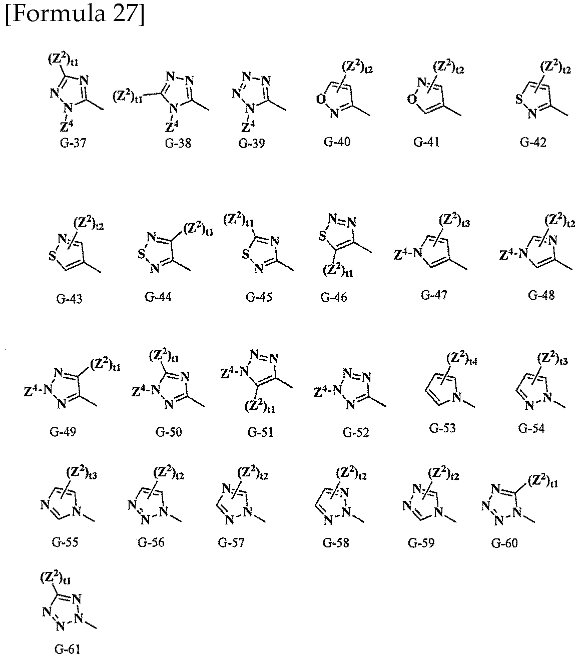
   Z² represents a halogen atom, C₁-C₆ alkyl, C₂-C₆ alkenyl, C₂-C₆ alkynyl, halo (C₁-C₆) alkyl, halo (C₂-C₆) alkenyl, halo (C₂-C₆) alkynyl, C₁-C₆ alkoxy, halo (C₁-C₆) alkoxy, C₁-C₆ alkoxy (C₁-C₆) alkoxy, C₂-C₆ alkenyloxy, halo (C₂-C₆) alkenyloxy, C₂-C₆ alkynyloxy, halo (C₂-C₆) alkynyloxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, C₂-C₆ alkenylthio, C₂-C₆ alkenylsulfinyl, C₂-C₆ alkenylsulfonyl, halo (C₂-C₆) alkenylthio, halo (C₂-C₆) alkenylsulfinyl, halo (C₂-C₆) alkenylsulfonyl, C₂-C₆ alkynylthio, C₂-C₆ alkynylsulfinyl, C₂-C₆ alkynylsulfonyl, halo (C₂-C₆) alkynylthio, halo (C₂-C₆) alkynylsulfinyl, halo (C₂-C₆) alkynylsulfonyl, -NH₂, C₁-C₆ alkylamino, di (C₁-C₆) alkylamino, halo (C₁-C₆) alkylamino, dihalo (C₁-C₆) alkylamino, formyl, carboxy, C₁-C₆ alkylcarbonyl, halo (C₁-C₆) alkylcarbonyl, C₁-C₆ alkoxycarbonyl, halo (C₁-C₆) alkoxycarbonyl, C₁-C₆ alkylaminocarbonyl, halo (C₁-C₆) alkylaminocarbonyl, di (C₁-C₆) alkylaminocarbonyl, hydroxy, C₁-C₆ alkylcarbonyloxy, C₁-C₆ alkoxycarbonyloxy, C₁-C₆ alkylsulfonyloxy, halo (C₁-C₆) alkylsulfonyloxy, -SO₃H, C₁-C₆ alkylaminosulfonyl, di (C₁-C₆) alkylaminosulfonyl, -C(=NOR¹⁴)R¹⁵, nitro or cyano,
   R¹⁴ represents a hydrogen atom or C₁-C₆ alkyl,
   R¹⁵ represents a hydrogen atom or C₁-C₆ alkyl,
   Z⁴ represents C₁-C₆ alkyl,
   R¹ represents a hydrogen atom, a halogen atom, halo (C₁-C₆) alkyl, C₁-C₆ alkyl or cyano,
   R² represents a hydrogen atom, C₁-C₆ alkyl, halo (C₁-C₆) alkyl, C₂-C₆ alkenyl, C₂-C₆ alkynyl, C₃-C₆ cycloalkyl, -C(O)R⁴, -C(O)OR⁵, D-1, D-2 or D-3, 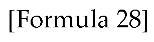 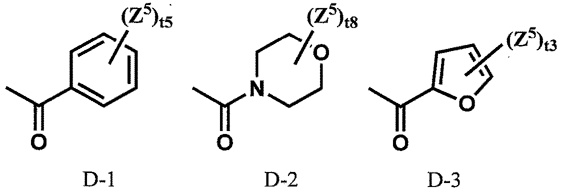
   R⁴ represents C₁-C₆ alkyl, C₂-C₆ alkenyl, C₂-C₆ alkynyl, C₃-C₆ cycloalkyl, C₁-C₆ alkylcarbonyl, di (C₁-C₆) alkylamino or (C₁-C₆) alkyl substituted with R⁶,
   R⁶ represents a halogen atom, C₁-C₆ alkoxy, cyano, nitro, phenyl or phenyl substituted with R⁸,
   Z⁵ represents a halogen atom, C₁-C₆ alkyl, C₁-C₆ alkoxy, cyano or nitro,
   R⁸ represents a halogen atom, C₁-C₆ alkyl, C₁-C₆ alkoxy, cyano or nitro,
   R⁵ represents C₁-C₆ alkyl, C₂-C₆ alkenyl, C₂-C₆ alkynyl, C₃-C₆ cycloalkyl, (C₁-C₆) alkyl substituted with R⁹, phenyl or phenyl substituted with R¹⁰,
   R⁹ represents a halogen atom, C₁-C₆ alkoxy, cyano, nitro, phenyl or phenyl substituted with R¹¹,
   R¹⁰ represents a halogen atom, C₁-C₆ alkyl, C₁-C₆ alkoxy, cyano or nitro,
   R¹¹ represents a halogen atom, C₁-C₆ alkyl, C₁-C₆ alkoxy, cyano or nitro,
   R³ represents a hydrogen atom, C₁-C₆ alkyl, halo (C₁-C₆) alkyl, C₂-C₆ alkenyl, C₂-C₆ alkynyl, C₃-C₆ cycloalkyl or C₁-C₆ alkylcarbonyl,
   X represents an oxygen atom or a sulfur atom,
   Y represents an oxygen atom or a sulfur atom,
   t1 represents an integer of 0 or 1,
   t2 represents an integer of 0, 1 or 2,
   t3 represents an integer of 0, 1, 2 or 3,
   t4 represents an integer of 0, 1, 2, 3 or 4,
   t5 represents an integer of 0, 1, 2, 3, 4 or 5, and
   t8 represents an integer of 0, 1, 2, 3 or 4.
[2] The aryltetrahydropyridine compound according to [1] or a salt thereof, wherein
   A¹ represents a nitrogen atom.
[3] The aryltetrahydropyridine compound according to any one of [1] to [2] or a salt thereof, wherein
   Z¹ represents a halogen atom, C₁-C₆ alkyl, C₂-C₆ alkenyl, C₂-C₆ alkynyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, -NH₂, C₁-C₆ alkylamino, di (C₁-C₆) alkylamino, carboxy, C₁-C₆ alkylcarbonyl, C₁-C₆ alkoxycarbonyl, C₁-C₆ alkylaminocarbonyl, di (C₁-C₆) alkylaminocarbonyl, hydroxy, C₁-C₆ alkylcarbonyloxy, C₁-C₆ alkylsulfonyloxy, cyano or nitro,
   Z² represents a halogen atom, C₁-C₆ alkyl, C₂-C₆ alkenyl, C₂-C₆ alkynyl, halo (C₁-C₆) alkyl, halo (C₂-C₆) alkenyl, halo (C₂-C₆) alkynyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, -NH₂, C₁-C₆ alkylamino, di (C₁-C₆) alkylamino, carboxy, C₁-C₆ alkylcarbonyl, C₁-C₆ alkoxycarbonyl, C₁-C₆ alkylaminocarbonyl, di (C₁-C₆) alkylaminocarbonyl, hydroxy, C₁-C₆ alkylcarbonyloxy, C₁-C₆ alkylsulfonyloxy, -C(=NOR¹⁴)R¹⁵, nitro or cyano,
   Z⁵ represents a halogen atom, C₁-C₆ alkyl or C₁-C₆ alkoxy,
   R¹ represents a hydrogen atom, a halogen atom or halo (C₁-C₆) alkyl,
   R² represents a hydrogen atom, -C(O)R⁴, -C(O)OR⁵, D-1, D-2 or D-3,
   R³ represents a hydrogen atom,
   Q represents Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-25, Q-26, Q-27, Q-29, Q-30, Q-31, Q-32, Q-33, Q-38, Q-39, Q-40, Q-41, Q-42, Q-43, Q-44, Q-45, Q-46, Q-47, Q-50, Q-51, Q-52 or Q-54, and
   G represents G-1, G-2, G-3, G-4, G-5, G-6, G-7, G-8, G-9, G-10, G-11, G-12, G-13, G-14, G-15, G-16, G-17, G-18, G-19, G-20, G-21, G-22, G-23, G-24, G-25, G-26, G-27, G-28, G-29, G-30, G-31, G-32, G-33, G-34, G-35, G-37, G-38, G-40, G-41, G-42, G-43, G-45, G-47, G-48, G-50, G-53, G-54, G-55 or G-57.
[4] The aryltetrahydropyridine compound according to any one of [1] to [3] or a salt thereof, wherein
   J represents J-1, and
   A¹ represents a nitrogen atom.
[5] The aryltetrahydropyridine compound according to any one of [1] to [3] or a salt thereof, wherein
   J represents J-2.
[6] The aryltetrahydropyridine compound according to any one of [1] to [5] or a salt thereof, wherein
   G represents G-1, G-3, G-4, G-5, G-7, G-9, G-10, G-11, G-12, G-13, G-14, G-15, G-16, G-17, G-18, G-19, G-20, G-21, G-22, G-23, G-24, G-25, G-26, G-27, G-28, G-29, G-30, G-31, G-32, G-34, G-35, G-37, G-38, G-40, G-41, G-42, G-43, G-45, G-47, G-48, G-50, G-53, G-55 or G-57.
[7] The aryltetrahydropyridine compound according to [1] or a salt thereof, wherein
   Q represents Q-1, Q-2, Q-3, Q-4, Q-5, Q-6, Q-7, Q-8, Q-9, Q-10, Q-11, Q-12, Q-13, Q-14, Q-20, Q-22, Q-29, Q-31, Q-45 or Q-52,
   Z¹ represents a halogen atom, C₁-C₆ alkyl, C₂-C₆ alkenyl, C₂-C₆ alkynyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, carboxy, C₁-C₆ alkylcarbonyl, C₁-C₆ alkoxycarbonyl, C₁-C₆ alkylaminocarbonyl, hydroxy, cyano or nitro,
   G represents G-1, G-2, G-3, G-4, G-5, G-6, G-7, G-8, G-9, G-10, G-11, G-12, G-13, G-14, G-15, G-16, G-17, G-18, G-19, G-20, G-27, G-28 or G-40,
   Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, di (C₁-C₆) alkylamino, C₁-C₆ alkylcarbonyl, C₁-C₆ alkoxycarbonyl, hydroxy, -C(=NOR¹⁴)R¹⁵, nitro or cyano,
   R¹⁴ represents C₁-C₆ alkyl,
   R¹ represents a hydrogen atom, a halogen atom or halo (C₁-C₆) alkyl,
   R² represents a hydrogen atom, -C(O)R⁴, -C(O)OR⁵, D-1, D-2 or D-3,
   R⁴ represents C₁-C₆ alkyl, C₃-C₆ cycloalkyl, C₁-C₆ alkylcarbonyl or (C₁-C₆) alkyl substituted with R⁶,
   R⁶ represents a halogen atom or C₁-C₆ alkoxy,
   Z⁵ represents C₁-C₆ alkyl,
   R⁵ represents C₁-C₆ alkyl or C₂-C₆ alkenyl,
   R³ represents a hydrogen atom, and
   X represents an oxygen atom.
[8] The aryltetrahydropyridine compound according to [7] or a salt thereof, wherein
   G represents G-1, G-3, G-4, G-5, G-7, G-9, G-10, G-11, G-12, G-13, G-14, G-15, G-16, G-17, G-18, G-19, G-20, G-27, G-28 or G-40.
[9] The aryltetrahydropyridine compound according to [8] or a salt thereof, wherein
   Q represents Q-1, Q-2, Q-3, Q-4, Q-5, Q-6, Q-7, Q-8, Q-9, Q-10, Q-12, Q-13, Q-14, Q-20, Q-22, Q-29 or Q-45,
   Z¹ represents a halogen atom, C₁-C₆ alkyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, C₁-C₆ alkoxycarbonyl, cyano or nitro,
   Z³ represents C₁-C₆ alkyl, and
   Z² represents a halogen atom, C₁-C₆ alkyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, hydroxy, nitro or cyano.
[10] The aryltetrahydropyridine compound according to [9] or a salt thereof, wherein
   J represents J-1,
   A¹ represents a nitrogen atom,
   Q represents Q-1, Q-2, Q-3, Q-4, Q-5, Q-6, Q-7, Q-8, Q-9, Q-12, Q-13, Q-14 or Q-22,
   G represents G-1, G-3, G-4 or G-5,
   Z² represents a halogen atom, C₁-C₆ alkyl, halo (C₁-C₆) alkyl, C₁-C₆ alkoxy, halo (C₁-C₆) alkoxy or nitro,
   R² represents a hydrogen atom or -C(O)OR⁵,
   R⁵ represents C₁-C₆ alkyl, and
   Y represents an oxygen atom.
[11] The aryltetrahydropyridine compound according to [10] or a salt thereof, wherein
   Q represents Q-1, Q-2, Q-3, Q-4, Q-6, Q-9 or Q-12,
   Z¹ represents a halogen atom, C₁-C₆ alkyl, halo (C₁-C₆) alkyl, cyano or nitro,
   G represents G-1 or G-3,
   Z² represents a halogen atom, C₁-C₆ alkyl or halo (C₁-C₆) alkyl,
   R¹ represents a halogen atom or halo (C₁-C₆) alkyl, and
   R² represents a hydrogen atom.
[12] The aryltetrahydropyridine compound according to [11] or a salt thereof, wherein
   J represents J-2,
   Q represents Q-1, Q-2, Q-3, Q-4, Q-6, Q-9, Q-10, Q-12, Q-20, Q-29 or Q-45,
   Z¹ represents a halogen atom, C₁-C₆ alkyl, halo (C₁-C₆) alkyl, C₁-C₆ alkoxy, C₁-C₆ alkoxycarbonyl, cyano or nitro,
   G represents G-1, G-3, G-7, G-9, G-10, G-11, G-12, G-13, G-14, G-15, G-16, G-17, G-18, G-19, G-20, G-27, G-28 or G-40, and
   Z² represents a halogen atom, C₁-C₆ alkyl, halo (C₁-C₆) alkyl, C₁-C₆ alkoxy, C₁-C₆ alkylthio, C₁-C₆ alkylsulfinyl, C₁-C₆ alkylsulfonyl or hydroxy.
[13] The aryltetrahydropyridine compound according to [12] or a salt thereof, wherein
   Q represents Q-1, Q-2, Q-3, Q-4, Q-6, Q-9 or Q-12,
   Z¹ represents a halogen atom, C₁-C₆ alkyl, halo (C₁-C₆) alkyl, cyano or nitro, G represents G-1, G-3 or G-7,
   Z² represents a halogen atom, C₁-C₆ alkyl or halo (C₁-C₆) alkyl, and
   R² represents a hydrogen atom.
[14] The aryltetrahydropyridine compound according to any one of [1] to [6] or a salt thereof, wherein
   Q represents Q-1, Q-2, Q-3, Q-4, Q-5, Q-6, Q-7, Q-8, Q-11, Q-14, Q-15, Q-16, Q-17 or Q-18.
[15] The aryltetrahydropyridine compound according to any one of [1] to [8] or a salt thereof, wherein
   Q represents Q-1, Q-2, Q-3, Q-4, Q-5, Q-6, Q-7, Q-8, Q-11 or Q-14.
[16] The aryltetrahydropyridine compound according to any one of [1] to [13] or a salt thereof, wherein
   Q represents Q-1.
[17] The aryltetrahydropyridine compound according to any one of [1] to [6] or a salt thereof, wherein
   Q represents Q-2, Q-3, Q-4, Q-5, Q-6, Q-7, Q-8, Q-14, Q-15, Q-16, Q-17 or Q-18.
[18] The aryltetrahydropyridine compound according to any one of [1] to [9] or a salt thereof, wherein
   Q represents Q-2, Q-3, Q-4, Q-5, Q-6, Q-7, Q-8 or Q-14.
[19] The aryltetrahydropyridine compound according to any one of [1] to [6] or a salt thereof, wherein
   Q represents Q-9, Q-10, Q-12, Q-13, Q-19, Q-20, Q-21, Q-22, Q-23, Q-25, Q-26, Q-27, Q-29, Q-30, Q-31, Q-32, Q-33, Q-38, Q-39, Q-40, Q-41, Q-42, Q-43, Q-44, Q-45, Q-46, Q-47, Q-50, Q-51, Q-52 or Q-54.
[20] The aryltetrahydropyridine compound according to any one of [1] to [8] or a salt thereof, wherein
   Q represents Q-9, Q-12, Q-13, Q-20, Q-22, Q-31, Q-45 or Q-52.
[21] The aryltetrahydropyridine compound according to any one of [1] to [6] or a salt thereof, wherein
   Q represents Q-12, Q-30, Q-31, Q-32, Q-33, Q-40, Q-45, Q-47, Q-49, Q-52, Q-55 or Q-56.
[22] The aryltetrahydropyridine compound according to any one of [1] to [8] or a salt thereof, wherein
   Q represents Q-31, Q-45 or Q-52.
[23] The aryltetrahydropyridine compound according to any one of [1] to [6] or a salt thereof, wherein
   Q represents Q-9, Q-13, Q-25, Q-26, Q-27, Q-29, Q-39, Q-42, Q-44, Q-46, Q-51 or Q-54.
[24] The aryltetrahydropyridine compound according to any one of [1] to [8] or a salt thereof, wherein
   Q represents Q-9 or Q-13.
[25] The aryltetrahydropyridine compound according to any one of [1] to [6] or a salt thereof, wherein
   Q represents Q-10, Q-19, Q-20, Q-21, Q-22, Q-23, Q-38, Q-41, Q-43 or Q-50.
[26] The aryltetrahydropyridine compound according to any one of [1] to [8] or a salt thereof, wherein
   Q represents Q-10, Q-20 or Q-22.
[27] The aryltetrahydropyridine compound according to any one of [1] to [8] or a salt thereof, wherein
   Q represents Q-1, Q-2, Q-3, Q-4, Q-6, Q-9, Q-10 or Q-12.
[28] The aryltetrahydropyridine compound according to any one of [1] to [13] or a salt thereof, wherein
   Q represents Q-1, Q-2, Q-3, Q-4, Q-6, Q-9 or Q-12.
[29] The aryltetrahydropyridine compound according to any one of [1] to [13] or a salt thereof, wherein
   Q represents Q-1, Q-2, Q-3, Q-4 or Q-6.
[30] The aryltetrahydropyridine compound according to any one of [1] to [8] or a salt thereof, wherein
   Q represents Q-9, Q-10 or Q-12.
[31] The aryltetrahydropyridine compound according to any one of [1] to [13] or a salt thereof, wherein
   Q represents Q-9 or Q-12.
[32] The aryltetrahydropyridine compound according to any one of [1] to [2] and [14] to [31] or a salt thereof, wherein
   Z¹ represents a halogen atom, C₁-C₆ alkyl, C₂-C₆ alkenyl, C₂-C₆ alkynyl, halo (C₁-C₆) alkyl, halo (C₂-C₆) alkenyl, halo (C₂-C₆) alkynyl, C₁-C₆ alkoxy, halo (C₁-C₆) alkoxy, C₂-C₆ alkoxy (C₁-C₆) alkoxy, C₂-C₆ alkenyloxy, halo (C₂-C₆) alkenyloxy, C₂-C₆ alkynyloxy, halo (C₂-C₆) alkynyloxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, C₂-C₆ alkenylthio, C₂-C₆ alkenylsulfinyl, C₂-C₆ alkenylsulfonyl, halo (C₂-C₆) alkenylthio, halo (C₂-C₆) alkenylsulfinyl, halo (C₂-C₆) alkenylsulfonyl, C₂-C₆ alkynylthio, C₂-C₆ alkynylsulfinyl, C₂-C₆ alkynylsulfonyl, halo (C₂-C₆) alkynylthio, halo (C₂-C₆) alkynylsulfinyl, halo (C₂-C₆) alkynylsulfonyl, cyano or nitro.
[33] The aryltetrahydropyridine compound according to any one of [1] to [2] and [14] to [31] or a salt thereof, wherein
   Z¹ represents a halogen atom, C₁-C₆ alkyl, C₂-C₆ alkenyl, C₂-C₆ alkynyl, halo (C₁-C₆) alkyl, -NH₂, C₁-C₆ alkylamino, di (C₁-C₆) alkylamino, halo (C₁-C₆) alkylamino, dihalo (C₁-C₆) alkylamino, formyl, carboxy, C₁-C₆ alkylcarbonyl, halo (C₁-C₆) alkylcarbonyl, C₁-C₆ alkoxycarbonyl, halo (C₁-C₆) alkoxycarbonyl, C₁-C₆ alkylaminocarbonyl, halo (C₁-C₆) alkylaminocarbonyl, di (C₁-C₆) alkylaminocarbonyl, cyano or nitro.
[34] The aryltetrahydropyridine compound according to any one of [1] to [13] or a salt thereof, wherein
   Z¹ represents a halogen atom, C₁-C₆ alkyl, halo (C₁-C₆) alkyl, cyano or nitro.
[35] The aryltetrahydropyridine compound according to any one of [1] to [13] or a salt thereof, wherein
   Z¹ represents a halogen atom, C₁-C₆ alkyl, halo (C₁-C₆) alkyl or cyano.
[36] The aryltetrahydropyridine compound according to any one of [1] to [35] or a salt thereof, wherein
   Z³ represents C₁-C₆ alkyl.
[37] The aryltetrahydropyridine compound according to any one of [1] to [35] or a salt thereof, wherein
   Z³ represents a hydrogen atom.
[38] The aryltetrahydropyridine compound according to any one of [1] and [14] to [37] or a salt thereof, wherein
   G represents G-1, G-3, G-4, G-5, G-6, G-7, G-8, G-9, G-10, G-11, G-12, G-13, G-14, G-15, G-16, G-17, G-18, G-19, G-20, G-21, G-22, G-23, G-24, G-25, G-26, G-27, G-28, G-29, G-30, G-31, G-32, G-33, G-34, G-35, G-36, G-37, G-38, G-39, G-40, G-41, G-42, G-43, G-44, G-45, G-46, G-47, G-48, G-49, G-50, G-51, G-52, G-53, G-54, G-55, G-56, G-57, G-58, G-59, G-60 or G-61.
[39] The aryltetrahydropyridine compound according to any one of [1] to [2] and [14] to [37] or a salt thereof, wherein
   G represents G-1, G-3, G-5, G-6, G-7, G-8, G-11, G-17, G-18, G-19, G-20, G-21, G-22, G-23, G-24, G-32, G-33, G-34, G-35, G-36, G-37, G-38, G-39, G-47, G-48, G-49, G-50, G-51, G-52, G-53, G-54, G-55, G-56, G-57, G-58, G-59, G-60 or G-61.
[40] The aryltetrahydropyridine compound according to any one of [1] to [6] and [14] to [37] or a salt thereof, wherein
   G represents G-1, G-3, G-5, G-7, G-11, G-17, G-18, G-19, G-20, G-21, G-22, G-23, G-24, G-32, G-34, G-35, G-36, G-37, G-38, G-47, G-48, G-50, G-53, G-55 or G-57.
[41] The aryltetrahydropyridine compound according to any one of [1] to [5], [7], and [14] to [37] or a salt thereof, wherein
   G represents G-1, G-3, G-5, G-6, G-7, G-8, G-11, G-17 or G-19.
[42] The aryltetrahydropyridine compound according to any one of [1] to [9] and [14] to [37] or a salt thereof, wherein
   G represents G-1, G-3, G-5, G-7, G-11, G-17 or G-19.
[43] The aryltetrahydropyridine compound according to any one of [1] to [9], [12], and [14] to [37] or a salt thereof, wherein
   G represents G-1, G-3, G-7, G-11, G-17 or G-19.
[44] The aryltetrahydropyridine compound according to any one of [1] to [5] and [14] to [37] or a salt thereof, wherein
   G represents G-1, G-2, G-3, G-5, G-7, G-11, G-17, G-18, G-19, G-20, G-21, G-22, G-23 or G-24.
[45] The aryltetrahydropyridine compound according to any one of [1] to [7] and [14] to [37] or a salt thereof, wherein
   G represents G-1, G-2, G-3, G-5, G-7, G-11, G-17 or G-19.
[46] The aryltetrahydropyridine compound according to any one of [1] to [9] and [14] to [37] or a salt thereof, wherein
   G represents G-1, G-3, G-5, G-7, G-11, G-17 or G-19.
[47] The aryltetrahydropyridine compound according to any one of [1] to [9], [12], and [14] to [37] or a salt thereof, wherein
   G represents G-1, G-3, G-7, G-11, G-17 or G-19.
[48] The aryltetrahydropyridine compound according to any one of [1] to [9], [12], and [14] to [37] or a salt thereof, wherein
   G represents G-1, G-3 or G-7.
[49] The aryltetrahydropyridine compound according to any one of [1] to [37] or a salt thereof, wherein
   G represents G-1 or G-3.
[50] The aryltetrahydropyridine compound according to any one of [1] to [49] or a salt thereof, wherein
   t1 represents an integer of 0,
   t2 represents an integer of 0,
   t3 represents an integer of 0 or 1,
   t4 represents an integer of 0, 1 or 2, and
   t5 represents an integer of 0 or 1.
[51] The aryltetrahydropyridine compound according to any one of [1] to [50] or a salt thereof, wherein
   Q represents Q-2, Q-3, Q-4, Q-6, Q-9, Q-12 or Q-14,
   Z¹ represents a halogen atom or C₁-C₆ alkyl,
   Z³ represents C₁ alkyl,
   J represents J-1 or J-2,
   A¹ represents a nitrogen atom,
   G represents G-1, G-3 or G-7,
   Z² represents a halogen atom,
   R¹ represents a halogen atom or halo (C₁-C₆) alkyl,
   R² represents a hydrogen atom,
   R³ represents a hydrogen atom,
   X represents an oxygen atom,
   Y represents an oxygen atom,
   t2 represents an integer of 0, and
   t4 represents an integer of 0, 1 or 2.
[52] The aryltetrahydropyridine compound according to any one of [1] to [50] or a salt thereof, wherein
   Q represents Q-2, Q-3, Q-4, Q-6, Q-9 or Q-12,
   Z¹ represents a halogen atom or C₁-C₆ alkyl,
   Z³ represents C₁ alkyl,
   J represents J-1,
   A¹ represents a nitrogen atom,
   G represents G-1 or G-3,
   Z² represents a halogen atom,
   R¹ represents a halogen atom or halo (C₁-C₆) alkyl,
   R² represents a hydrogen atom,
   R³ represents a hydrogen atom,
   X represents an oxygen atom,
   Y represents an oxygen atom,
   t2 represents an integer of 0, and
   t4 represents an integer of 0, 1 or 2.
[53] The aryltetrahydropyridine compound according to any one of [1] to [51] or a salt thereof, wherein
   Q represents Q-2, Q-3, Q-4, Q-6, Q-9 or Q-12,
   Z¹ represents a halogen atom or C₁-C₆ alkyl,
   Z³ represents C₁ alkyl,
   J represents J-2,
   G represents G-1 or G-3,
   Z² represents a halogen atom,
   R¹ represents a halogen atom or halo (C₁-C₆) alkyl,
   R² represents a hydrogen atom,
   R³ represents a hydrogen atom,
   X represents an oxygen atom,
   Y represents an oxygen atom,
   t2 represents an integer of 0, and
   t4 represents an integer of 0, 1 or 2.
[54] The aryltetrahydropyridine compound according to any one of [1] to [53] or a salt thereof, wherein
   Q represents Q-2 or Q-3,
   G represents G-1 or G-3, and
   t4 represents an integer of 0, 1 or 2.
[55] The aryltetrahydropyridine compound according to any one of [1] to [54] or a salt thereof, wherein
   R¹ represents a halogen atom or halo (C₁-C₆) alkyl.
[56] The aryltetrahydropyridine compound according to any one of [1] to [50] or a salt thereof, wherein
   R² represents a hydrogen atom, C₁-C₆ alkyl, C₂-C₆ alkenyl, -C(O)R⁴ or-C(O)OR⁵,
   R⁴ represents C₁-C₆ alkyl, and
   R⁵ represents C₁-C₆ alkyl.
[57] The aryltetrahydropyridine compound according to [56] or a salt thereof, wherein
   R² represents a hydrogen atom or C₁-C₆ alkyl.
[58] The aryltetrahydropyridine compound according to [57] or a salt thereof, wherein
   R² represents a hydrogen atom.
[59] The aryltetrahydropyridine compound according to any one of [1] to [58] or a salt thereof, wherein
   R³ represents a hydrogen atom.
[60] The aryltetrahydropyridine compound according to any one of [1] to [59] or a salt thereof, wherein
   X represents an oxygen atom, and
   Y represents an oxygen atom.
[61] An aryltetrahydropyridine compound or a salt thereof, the aryltetrahydropyridine compound represented by a formula (1): 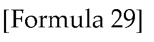 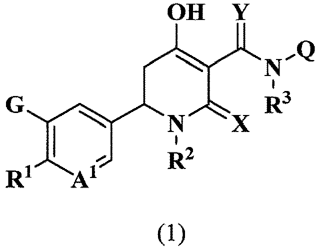 wherein
   Q represents Q-1, Q-2, Q-3 or Q-4, 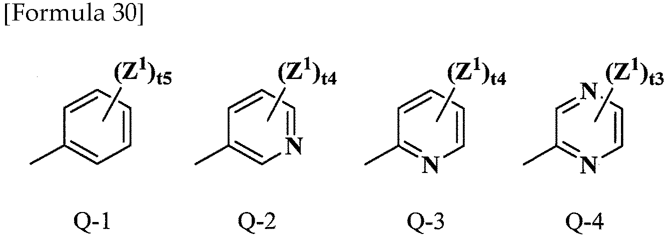
   Z¹ represents a halogen atom, halo (C₁-C₆) alkyl or cyano,
   G represents G-1, 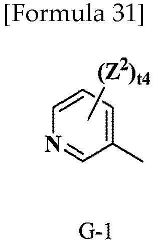
   Z² represents a halogen atom, C₁-C₆ alkyl, halo (C₁-C₆) alkyl, C₁-C₆ alkoxy or cyano,
   R¹ represents a halogen atom or halo (C₁-C₆) alkyl,
   R² represents a hydrogen atom,
   R³ represents a hydrogen atom,
   A¹ represents a nitrogen atom or CH,
   X represents an oxygen atom or a sulfur atom,
   Y represents an oxygen atom or a sulfur atom,
   t3 represents an integer of 0, 1, 2 or 3,
   t4 represents an integer of 0, 1, 2, 3 or 4, and
   t5 represents an integer of 0, 1, 2, 3, 4 or 5.
[62] The aryltetrahydropyridine compound according to [61] or a salt thereof, wherein
   Q represents Q-1,
   Z¹ represents a halogen atom,
   Z² represents a halogen atom or C₁-C₆ alkoxy,
   X represents an oxygen atom,
   Y represents an oxygen atom,
   t4 represents an integer of 1, and
   t5 represents an integer of 1.
[63] An aryltetrahydropyridine compound or a salt thereof, the aryltetrahydropyridine compound represented by a formula (1): 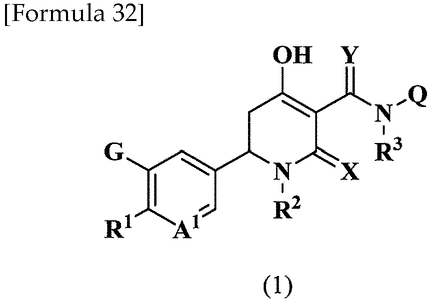 wherein
   Q represents Q-1, Q-2, Q-3, Q-4, Q-5, Q-6 or Q-7, 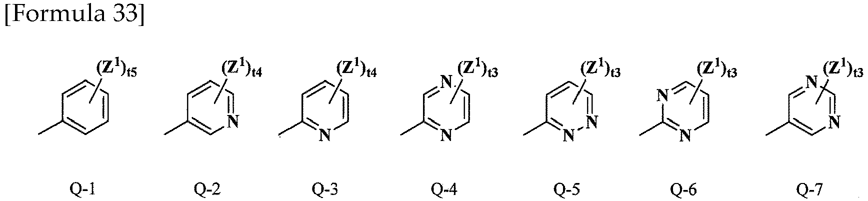
   Z¹ represents a halogen atom, C₁-C₆ alkyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio or cyano,
   G represents G-1, G-2, G-3, G-4, G-5 or G-6, 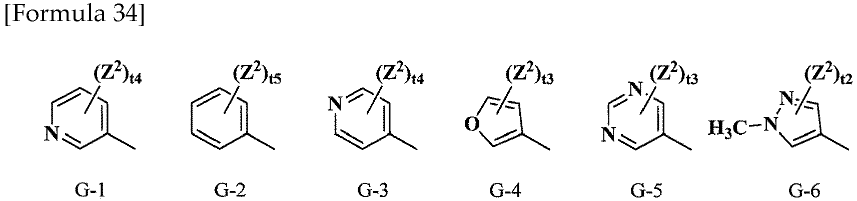
   Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, C₁-C₆ alkylcarbonyl, nitro or cyano,
   R¹ represents a hydrogen atom, a halogen atom or halo (C₁-C₆) alkyl,
   R² represents a hydrogen atom, C₁-C₆ alkyl or C₂-C₆ alkynyl,
   R³ represents a hydrogen atom or C₁-C₆ alkyl,
   A¹ represents a nitrogen atom or CH,
   X represents an oxygen atom or a sulfur atom,
   Y represents an oxygen atom or a sulfur atom,
   t2 represents an integer of 0, 1 or 2,
   t3 represents an integer of 0, 1, 2 or 3,
   t4 represents an integer of 0, 1, 2, 3 or 4, and
   t5 represents an integer of 0, 1, 2, 3, 4 or 5.
[64] The aryltetrahydropyridine compound according to [63] or a salt thereof, wherein
   Z¹ represents a halogen atom, C₁-C₆ alkyl, halo (C₁-C₆) alkyl, C₁-C₆ alkylthio, C₁-C₆ alkylsulfinyl, C₁-C₆ alkylsulfonyl, halo (C₁-C₆) alkylthio or cyano,
   Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfonyl, C₁-C₆ alkylcarbonyl, nitro or cyano,
   R² represents a hydrogen atom,
   R³ represents a hydrogen atom,
   X represents an oxygen atom, and
   Y represents an oxygen atom.
[65] An aryltetrahydropyridine compound or a salt thereof, the aryltetrahydropyridine compound represented by a formula (1): 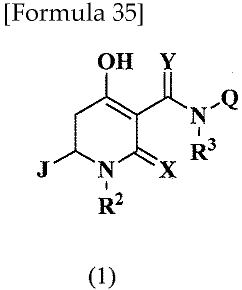 wherein
   Q represents Q-1, Q-2, Q-3, Q-4, Q-5, Q-6, Q-7 or Q-8, 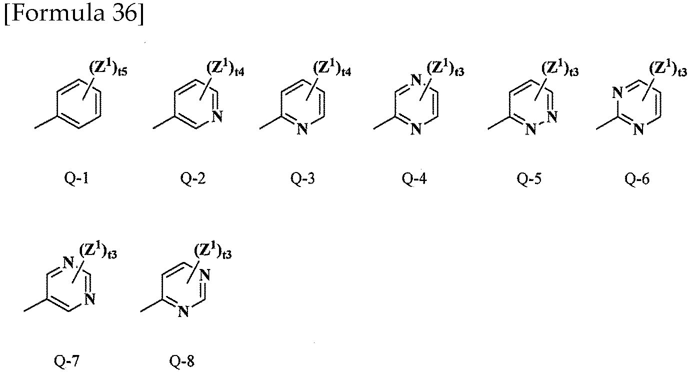
   Z¹ represents a halogen atom, C₁-C₆ alkyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio or cyano,
   J represents J-1 or J-2, 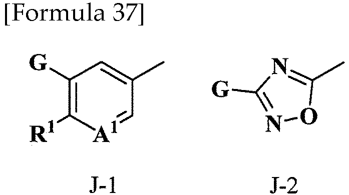
   G represents G-1, G-2, G-3, G-4, G-5, G-6, G-7, G-8 or G-9, 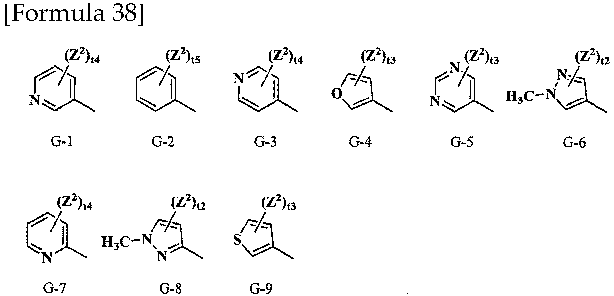
   Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, C₁-C₆ alkylcarbonyl, nitro or cyano,
   R¹ represents a hydrogen atom, a halogen atom or halo (C₁-C₆) alkyl,
   R² represents a hydrogen atom, C₁-C₆ alkyl or C₂-C₆ alkynyl,
   R³ represents a hydrogen atom or C₁-C₆ alkyl,
   A¹ represents a nitrogen atom or CH,
   X represents an oxygen atom or a sulfur atom,
   Y represents an oxygen atom or a sulfur atom,
   t2 represents an integer of 0, 1 or 2,
   t3 represents an integer of 0, 1, 2 or 3,
   t4 represents an integer of 0, 1, 2, 3 or 4, and
   t5 represents an integer of 0, 1, 2, 3, 4 or 5.
[66] The aryltetrahydropyridine compound according to [65] or a salt thereof, wherein
   Z¹ represents a halogen atom, C₁-C₆ alkyl, halo (C₁-C₆) alkyl, C₁-C₆ alkoxy, C₁-C₆ alkylthio, C₁-C₆ alkylsulfinyl, C₁-C₆ alkylsulfonyl, halo (C₁-C₆) alkylthio or cyano,
   Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfonyl, C₁-C₆ alkylcarbonyl, nitro or cyano,
   R² represents a hydrogen atom,
   R³ represents a hydrogen atom,
   X represents an oxygen atom, and
   Y represents an oxygen atom.
[67] An aryltetrahydropyridine compound or a salt thereof, the aryltetrahydropyridine compound represented by a formula (1): 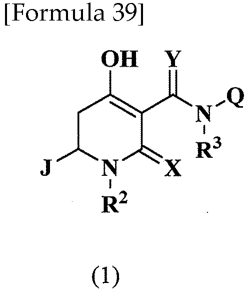 wherein
   Q represents Q-1, Q-2, Q-3, Q-4, Q-5, Q-6, Q-7, Q-8 or Q-9, 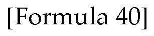 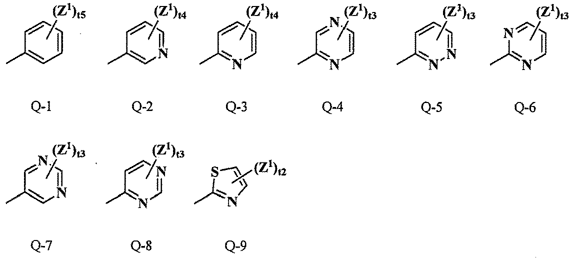
   Z¹ represents a halogen atom, C₁-C₆ alkyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, cyano or nitro,
   J represents J-1 or J-2, 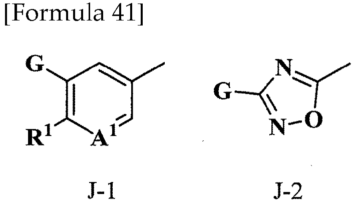
   G represents G-1, G-2, G-3, G-4, G-5, G-6, G-7, G-8 or G-9, 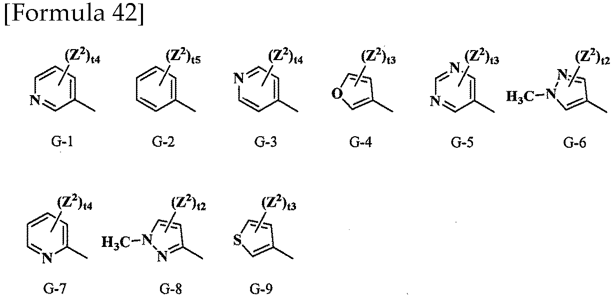
   Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, C₁-C₆ alkylcarbonyl, nitro or cyano,
   R¹ represents a hydrogen atom, a halogen atom or halo (C₁-C₆) alkyl,
   R² represents a hydrogen atom, C₁-C₆ alkyl, C₂-C₆ alkynyl or C₁-C₆ alkoxycarbonyl,
   R³ represents a hydrogen atom or C₁-C₆ alkyl,
   A¹ represents a nitrogen atom or CH,
   X represents an oxygen atom or a sulfur atom,
   Y represents an oxygen atom or a sulfur atom,
   t2 represents an integer of 0, 1 or 2,
   t3 represents an integer of 0, 1, 2 or 3,
   t4 represents an integer of 0, 1, 2, 3 or 4, and
   t5 represents an integer of 0, 1, 2, 3, 4 or 5.
[68] The aryltetrahydropyridine compound according to [67] or a salt thereof, wherein
   Z¹ represents a halogen atom, C₁-C₆ alkyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio or cyano,
   Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfonyl, C₁-C₆ alkylcarbonyl, nitro or cyano,
   R² represents a hydrogen atom,
   R³ represents a hydrogen atom,
   X represents an oxygen atom, and
   Y represents an oxygen atom.
[69] An aryltetrahydropyridine compound or a salt thereof, the aryltetrahydropyridine compound represented by a formula (1): 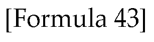 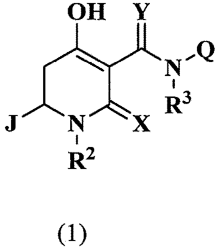 wherein
   Q represents Q-1, Q-2, Q-3, Q-4, Q-5, Q-6, Q-7, Q-8, Q-9, Q-10, Q-11, Q-12 or Q-13, 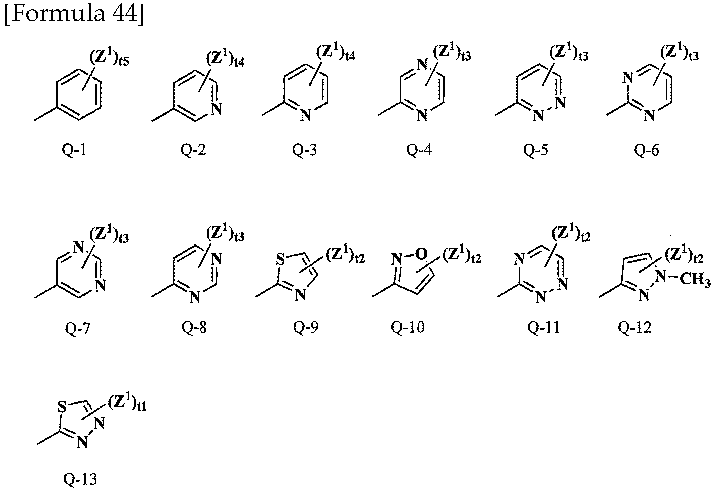
   Z¹ represents a halogen atom, C₁-C₆ alkyl, C₂-C₆ alkenyl, C₂-C₆ alkynyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, hydroxy, cyano or nitro,
   J represents J-1 or J-2, 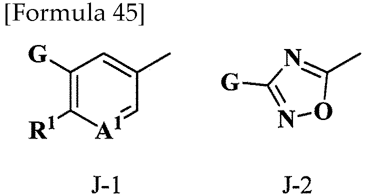
   G represents G-1, G-2, G-3, G-4, G-5, G-6, G-7, G-8, G-9, G-10, G-11, G-12, G-13, G-14, G-15, G-16 or G-17, 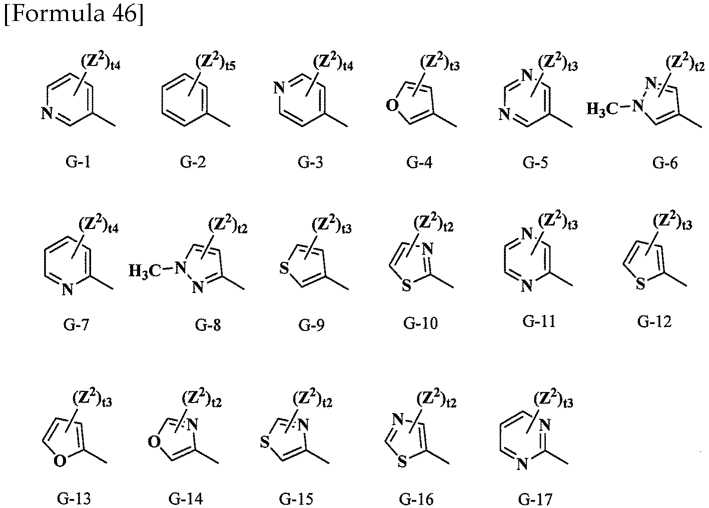
   Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, C₁-C₆ alkylcarbonyl, hydroxy, nitro or cyano,
   R¹ represents a hydrogen atom, a halogen atom or halo (C₁-C₆) alkyl,
   R² represents a hydrogen atom, C₁-C₆ alkyl, C₂-C₆ alkenyl, C₂-C₆ alkynyl, -C(O)R⁴ or -C(O)OR⁵
   R⁴ represents C₁-C₆ alkyl, C₃-C₆ cycloalkyl, C₁-C₆ alkylcarbonyl or (Cₐ-C_{b}) alkyl substituted with R⁶,
   R⁶ represents a halogen atom or C₁-C₆ alkoxy,
   R⁵ represents C₁-C₆ alkyl or C₂-C₆ alkenyl,
   R³ represents a hydrogen atom or C₁-C₆ alkyl,
   A¹ represents a nitrogen atom or CH,
   X represents an oxygen atom or a sulfur atom,
   Y represents an oxygen atom or a sulfur atom,
   t1 represents an integer of 0 or 1,
   t2 represents an integer of 0, 1 or 2,
   t3 represents an integer of 0, 1, 2 or 3,
   t4 represents an integer of 0, 1, 2, 3 or 4, and
   t5 represents an integer of 0, 1, 2, 3, 4 or 5.
[70] The aryltetrahydropyridine compound according to [69] or a salt thereof, wherein
   Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfonyl, C₁-C₆ alkylcarbonyl, hydroxy, nitro or cyano,
   R² represents a hydrogen atom, -C(O)R⁴ or -C(O)OR⁵,
   R³ represents a hydrogen atom,
   X represents an oxygen atom, and
   Y represents an oxygen atom.
[71] A production intermediate for the aryltetrahydropyridine compound according to any one of [1] to [70] or a salt thereof, the production intermediate represented by a formula (1-1): 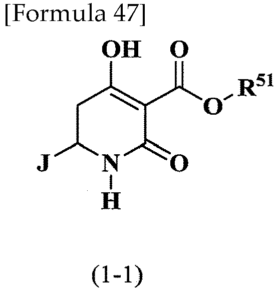 wherein J represents J-1 or J-2, 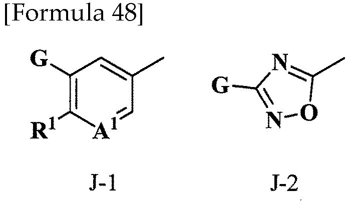
   A¹ represents a nitrogen atom,
   G represents G-1, G-2, G-3, G-4, G-5, G-6, G-7, G-8, G-9, G-10, G-11, G-12, G-13, G-14, G-15, G-16, G-17, G-18, G-19, G-20, G-27, G-28 or G-40, 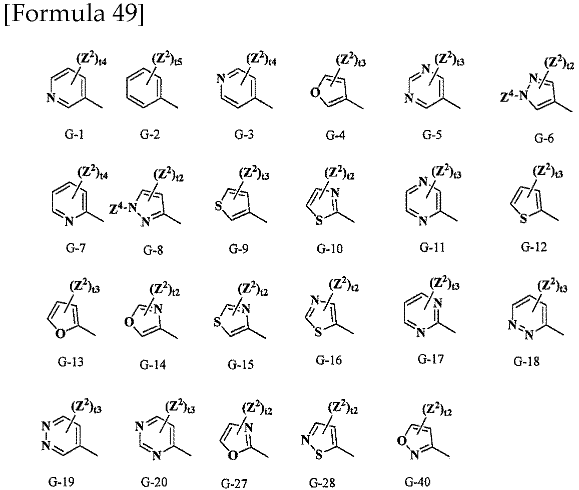
   Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, di (C₁-C₆) alkylamino, C₁-C₆ alkylcarbonyl, C₁-C₆ alkoxyarbonyl, hydroxy, -C(=NOR¹⁴)R¹⁵, nitro or cyano,
   R¹⁴ represents C₁-C₆ alkyl,
   R¹⁵ represents a hydrogen atom or C₁-C₆ alkyl,
   Z⁴ represents C₁-C₆ alkyl,
   R¹ represents a hydrogen atom, a halogen atom or halo (C₁-C₆) alkyl,
   R⁵¹ represents C₁-C₆ alkyl,
   t2 represents an integer of 0, 1 or 2,
   t3 represents an integer of 0, 1, 2 or 3,
   t4 represents an integer of 0, 1, 2, 3 or 4, and
   t5 represents an integer of 0, 1, 2, 3, 4 or 5.
[72] The production intermediate according to [71] for the aryltetrahydropyridine compound or a salt thereof, wherein
   J represents J-1,
   G represents G-1, G-2 or G-3,
   Z² represents a halogen atom or C₁-C₆ alkyl, and
   R¹ represents a halogen atom or halo (C₁-C₆) alkyl.
[73] A production intermediate for the aryltetrahydropyridine compound according to any one of [1] to [70] or a salt thereof, the production intermediate represented by a formula (1-2): 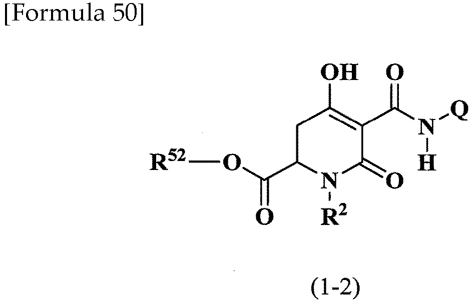 wherein R⁵² represents a hydrogen atom or C₁-C₆ alkyl,
   R² represents a hydrogen atom, -C(O)R⁴ or -C(O)OR⁵,
   R⁴ represents C₁-C₆ alkyl,
   R⁵ represents C₁-C₆ alkyl,
   Q represents Q-1, Q-2, Q-3, Q-4, Q-5, Q-6, Q-7, Q-8, Q-9, Q-10, Q-11, Q-12, Q-13, Q-14, Q-20, Q-22, Q-29, Q-31, Q-45 or Q-52, 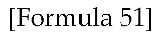 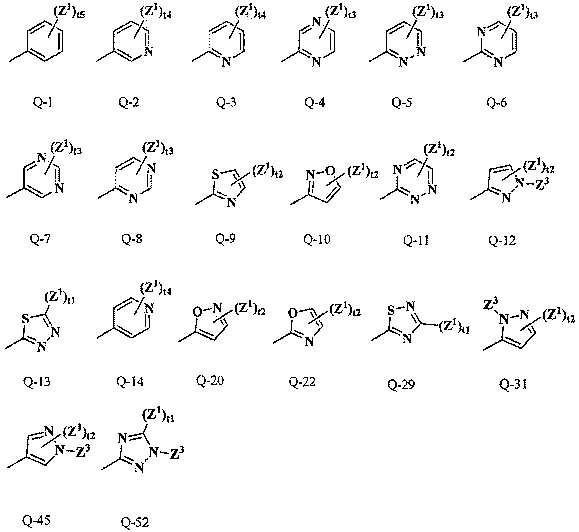
   Z¹ represents a halogen atom, C₁-C₆ alkyl, C₂-C₆ alkenyl, C₂-C₆ alkynyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, carboxy, C₁-C₆ alkylcarbonyl, C₁-C₆ alkoxycarbonyl, C₁-C₆ alkylaminocarbonyl, hydroxy, cyano or nitro,
   Z³ represents a hydrogen atom or C₁-C₆ alkyl,
   t1 represents an integer of 0 or 1,
   t2 represents an integer of 0, 1 or 2,
   t3 represents an integer of 0, 1, 2 or 3,
   t4 represents an integer of 0, 1, 2, 3 or 4, and
   t5 represents an integer of 0, 1, 2, 3, 4 or 5.
[74] The production intermediate according to [73] for the aryltetrahydropyridine compound or a salt thereof, wherein
   Q represents Q-1, Q-2, Q-3, Q-4, Q-6 or Q-9,
   Z¹ represents a halogen atom, C₁-C₆ alkyl or cyano, and
      R² represents a hydrogen atom or -C(O)OR⁵.
[75] A pesticide containing one or two or more selected from the aryltetrahydropyridine compound according to any one of [1] to [70] and salts thereof as an active ingredient.
[76] An agrochemical containing one or two or more selected from the aryltetrahydropyridine compound according to any one of [1] to [70] and salts thereof as an active ingredient.
[77] A control agent for an internal or external parasite in a mammal or a bird, the control agent containing one or two or more selected from the aryltetrahydropyridine compound according to any one of [1] to [70] and salts thereof as an active ingredient.
[78] The control agent according to [77], wherein the external parasite is a species belonging to Siphonaptera or a species belonging to Acari.
[79] An insecticide or acaricide containing one or two or more selected from the aryltetrahydropyridine compound according to any one of [1] to [70] and salts thereof as an active ingredient.
[80] An agent for seed treatment, the agent containing one or two or more selected from the aryltetrahydropyridine compound according to any one of [1] to [70] and salts thereof as an active ingredient.
[81] The agent for seed treatment according to [80], wherein the seed treatment is performed by soaking.
[82] An agent for soil treatment, the agent containing one or two or more selected from the aryltetrahydropyridine compound according to any one of [1] to [70] and salts thereof as an active ingredient.
[83] The agent for soil treatment according to [82], wherein the soil treatment is performed by soil drenching.

### Examples

The following more specifically describes the present invention by showing Synthesis Examples and Test Examples for the presently invented compound as Examples, but the present invention is not limited by them.

For medium pressure preparative liquid chromatography shown in Synthesis Examples, the medium pressure separation apparatus YFLC-Wprep (flow rate: 18 ml/min, silica gel 40-µm-column) manufactured by YAMAZEN CORPORATION was used.

Chemical shift values in proton nuclear magnetic resonance spectra (hereinafter, referred to as ¹H-NMR) shown below were determined with use of Me₄Si (tetramethylsilane) as a standard substance in deuterated chloroform solvent at 300 MHz (model: JNM-ECX300 or JNM-ECP300, manufactured by JEOL Ltd.) or 400 MHz (model: JNM-ECZ400S, manufactured by JEOL Ltd.). Signs for chemical shift values in ¹H-NMR have the following meanings. s: singlet, d: doublet, dd: double doublet, t: triplet, q: quartet, m: multiplet, brs: broad singlet

### Synthesis Example 1: Synthesis of N-(4-fluorophenyl)-6-[3-(6-fluoropyridin-3-yl)-4-(trifluoromethyl)phenyl]-4-hydroxy-2-oxo-1,2,5,6-tetrahydropyridine-3-carboxamide (presently invented compound No. 1-001)

To a mixed solution of 94 mg of 6-[3-bromo-4(trifluoromethyl)phenyl]-N-(4-fluorophenyl)-4-hydroxy-2-oxo-1,2,5,6-tetrahydropyridine-3-carboxamide, 1.6 ml of 1,4-dioxane, and 0.4 ml of water, 42 mg of 2-fluoropyridine-5-boronic acid, 83 mg of potassium carbonate, and 16 mg of a [1,1'-bis(diphenylphosphino)ferrocene]palladium (divalent) dichloride dichloromethane adduct were added, and the resultant was stirred under a nitrogen atmosphere at 90°C for 5 hours. After the completion of the reaction, 5 ml of 1 mol/l hydrochloric acid was added, and extraction was performed with ethyl acetate (5 ml × 3 times). The resulting organic layer was dehydrated and dried with anhydrous sodium sulfate, and the solvent was then distilled off under reduced pressure. The resulting residue was purified by medium pressure preparative liquid chromatography [gradient from n-hexane:ethyl acetate = 100:0 to 0:100 (volume ratio, the same applies hereinafter)], giving 31 mg of the target substance as a white solid.
Melting point: 200 to 202°C
¹H-NMR (300 MHz): δ 11.5-12.0 (m, 1H), 8.18 (brs, 1H), 7.85 (d, J = 8.7 Hz, 1H), 7.65-7.8 (m, 1H), 7.4-7.65 (m, 4H), 7.33 (brs, 1H), 6.95-7.1 (m, 3H), 5.45-5.7 (m, 1H), 4.8-5.0 (m, 1H), 2.75-3.0 (m, 2H).

### Synthesis Example 2: Synthesis of 6'-chloro-N-(4-chlorophenyl)-6"-fluoro-4-hydroxy-6-oxo-1,2,3,6-tetrahydro-[2,3':5',3"-terpyridine]-5-carboxamide (presently invented compound No. 2-035)

To a mixed solution of 100 mg of ethyl 6'-chloro-6"-fluoro-4-hydroxy-6-oxo-1,2,3,6-tetrahydro-[2,3':5',3"-terpyridine]-5-carboxylate and 0.7 ml of 1,4-dioxane, 32 mg of 4-chloroaniline was added, and the resultant was stirred at 80°C for 1 hour. After the completion of the reaction, the solvent was distilled off under reduced pressure. The resulting residue was washed with a mixed solution of ethyl acetate:n-hexane = 1:2, giving 41 mg of the target substance as a pale brown solid.
Melting point: 199 to 201°C
¹H-NMR (300 MHz): δ 11.6 (brs, 1H), 8.48 (brs, 1H), 8.29 (brs, 1H), 7.85-8.0 (m, 1H), 7.65-7.8 (m, 1H), 7.4-7.6 (m, 2H), 7.25-7.35 (m, 2H), 7.05-7.15 (m, 2H), 5.3-5.8 (m, 1H), 4.85-4.95 (m, 1H), 2.7-3.05 (m, 2H).

### Synthesis Example 3: Synthesis of N-(5-fluoropyridin-2-yl)-6-(3-(2-fluoropyridin-4-yl)-1,2,4-oxadiazol-5-yl)-4-hydroxy-2-oxo-1,2,5,6-tetrahydropyridine-3-carboxamide (presently invented compound No. 3-036)

To a mixed solution of 50 mg of 6-(((2-fluoroisonicotinimidamide)oxy)carbonyl)-N-(5-fluoropyridin-2-yl)-4-hydroxy-2-oxo-1,2,5,6-tetrahydropyridine-3-carboxamide and 1.7 ml of toluene, 17 mg of triethylamine was added, and the resultant was stirred at 110°C for 3 hours. After the completion of the reaction, the solvent was distilled off under reduced pressure. To the resulting residue, 5 ml of 1 mol/l hydrochloric acid was added, and extraction was performed with ethyl acetate (5 ml × 3 times). The resulting organic layer was dehydrated and dried with anhydrous sodium sulfate, and the solvent was then distilled off under reduced pressure. To the resulting residue, 1 ml of N,N-dimethylformamide, 3 ml of water, and 1 ml of 1 mol/l hydrochloric acid were added. A solid precipitated was collected by filtration, giving 39 mg of the target substance as a white solid.
Melting point: 159 to 161°C

The following shows a synthesis example for the production intermediate used in Synthesis Example 2 for the presently invented compound.

### Intermediate Synthesis Example 1: Synthesis of ethyl 6'-chloro-6"-fluoro-4-hydroxy-6-oxo-1,2,3,6-tetrahydro-[2,3':5',3"-terpyridine]-5-carboxylate

### Step 1: Synthesis of ethyl 3-(5-bromo-6-chloropyridin-3-yl)-3-(3-ethoxy-3-oxopropanamide)propanoate

To a mixed solution of 13 g of ethyl 3-amino-3-(5-bromo-6-chloropyridin-3-yl)propanoate, 142 ml of ethyl acetate, 7.2 g of sodium hydrogen carbonate, and 142 ml of water, 6.7 g of ethyl malonyl chloride was added at 0°C. After the completion of the reaction, the resulting organic layer was dehydrated and dried with brine and anhydrous sodium sulfate, and the solvent was then distilled off under reduced pressure; thus, 18 g of the target substance was given as a brown oily substance.

¹H-NMR (300 MHz): δ 8.3-8.4 (m, 2H), 7.9-7.95 (m, 1H), 5.35-5.5 (m, 1H), 4.24 (q, J = 7.2 Hz, 2H), 4.13 (q, J = 7.2 Hz, 2H), 3.36 (brs, 2H), 2.89 (d, J = 5.7 Hz, 2H), 1.31 (t, J = 7.2 Hz, 3H), 1.20 (t, J = 7.2 Hz, 3H).

### Step 2: Synthesis of ethyl 5'-bromo-6'-chloro-4-hydroxy-6-oxo-1,2,3,6-tetrahydro-[2,3'-bipyridine]-5-carboxylate

To a mixed solution of 22 g of ethyl 3-(5-bromo-6-chloropyridin-3-yl)-3-(3-ethoxy-3-oxopropanamide)propanoate and 94 ml of dehydrated ethanol, 2.0 g of sodium hydride [manufactured by YONEYAMA YAKUHIN KOGYO CO., LTD., product name: sodium hydride. oily (ca. 63% by mass), purity: 57.5 to 67.5%, product code: 03414] was added, and the resultant was stirred under a nitrogen atmosphere at 55°C for 5 hours. After the completion of the reaction, the solvent was distilled off under reduced pressure. To the resulting residue, 150 ml of diipropyl ether was added, and extraction was performed with 150 ml of water. To the resulting aqueous layer, 35% by mass hydrochloric acid was added until the pH reached 2, and extraction was performed with a mixed solution of ethyl acetate:tetrahydrofuran = 9:1 (150 ml × 3 times). The resulting organic layer was dehydrated and dried with brine and anhydrous sodium sulfate, and the solvent was then distilled off under reduced pressure; thus, 16 g of the target substance was given as a brown solid.
Melting point: 124 to 126°C
¹H-NMR (400 MHz): δ 8.36 (d, J = 2.0 Hz, 1H), 7.98 (d, J = 2.4 Hz, 1H), 6.00 (brs, 1H), 4.7-4.8 (m, 1H), 4.35-4.5 (m, 2H), 2.7-3.05 (m, 2H), 1.40 (t, J = 7.2 Hz, 3H).

### Step 3: Synthesis of ethyl 6'-chloro-6"-fluoro-4-hydroxy-6-oxo-1,2,3,6-tetrahydro-[2,3':5',3"-terpyridine]-5-carboxylate

To a mixed solution of 5.6 g of ethyl 5'-bromo-6'-chloro-4-hydroxy-6-oxo-1,2,3,6-tetrahydro-[2,3'-bipyridine]-5-carboxylate, 40 ml of 1,4-dioxane, and **10 ml of** water, 1.9 g of 2-fluoropyridine-5-boronic acid, 9.5 g of tripotassium phosphate, and 606 mg of a [1,1'-bis(diphenylphosphino)ferrocene]palladium (divalent) dichloride dichloromethane adduct were added, and the resultant was stirred under a nitrogen atmosphere at 55°C for 2 hours. After the completion of the reaction, 200 ml of diisopropyl ether was added, and extraction was performed with a mixed solution of 10 g of potassium carbonate and 74 ml of water. To the resulting aqueous layer, 35% by mass hydrochloric acid was added until the pH reached 2, and extraction was performed with ethyl acetate (150 ml × 3 times). The resulting organic layer was dehydrated and dried with brine and anhydrous sodium sulfate, and the solvent was then distilled off under reduced pressure. The resulting residue was washed with a mixed solution of ethanol:diisopropyl ether = 1:2, giving 2.0 g of the target substance as a pale brown solid.
Melting point: 178 to 180°C
¹H-NMR (400 MHz): δ 8.47 (d, J = 2.4 Hz, 1H), 8.30 (brs, 1H), 7.9-8.0 (m, 1H), 7.71 (d, J = 2.0 Hz, 1H), 7.05-7.1 (m, 1H), 5.70 (brs, 1H), 4.8-4.9 (m, 2H), 4.35-4.5 (m, 2H), 2.7-3.05 (m, 2H), 1.40 (t, J = 7.2 Hz, 3H).

The following shows a synthesis example for the production intermediate used in Synthesis Example 3 for the presently invented compound.

### Intermediate Synthesis Example 2: Synthesis of 6-(((2-fluoroisonicotinimidamide)oxy)carbonyl)-N-(5-fluoropyridin-2-yl)-4-hydroxy-2-oxo-1,2,5,6-tetrahydropyridine-3-carboxamide

### Step 1: Synthesis of di-tert-butyl 5-((5-fluoropyridin-2-yl)carbamoyl)-4-hydroxy-6-oxo-3,6-dihydropyridine-1,2(2H)-dicarboxylate

To a mixed solution of 18.3 g of di-tert-butyl 4-hydroxy-6-oxo-3,6-dihydropyridine-1,2(2H)-dicarboxylate and 60 ml of acetonitrile, 4.0 g of imidazole and 6.6 g of 1,1'-carbonyldiimidazole were added, and the resultant was stirred at 85°C for 30 minutes. After cooling to room temperature, 6.6 g of 5-fluoropyridin-2-amine was added, and the resultant was stirred for 18 hours. Thereto, 350 ml of diisopropyl ether was added, the resultant was stirred at 0°C for 1.5 hours, and a solid precipitated was collected by filtration. The resulting solid was washed with a mixed solution of acetonitrile:diisopropyl ether = 1:5, giving 26.3 g of the target substance as a pale red solid.
Melting point: 152 to 154°C

### Step 2: Synthesis of 5-((5-fluoropyridin-2-yl)carbamoyl)-4-hydroxy-6-oxo-1,2,3,6-tetrahydropyridine-2-carboxylic acid

To a mixed solution of 15.4 g of di-tert-butyl 5-((5-fluoropyridin-2-yl)carbamoyl)-4-hydroxy-6-oxo-3,6-dihydropyridine-1,2(2H)-dicarboxylate and 70 ml of dichloromethane, 50 ml of trifluoroacetic acid was added, and the resultant was stirred at room temperature for 25 hours. Thereto, 140 ml of toluene was added, and the solvent was distilled off under reduced pressure. Thereto, 100 ml of toluene was added, and the solvent was distilled off under reduced pressure. Thereto, 100 ml of toluene was added, and the solvent was distilled off under reduced pressure. Thereto, 100 ml of chloroform was added, and a solid was collected by filtration. The solid obtained was washed with chloroform, giving 14.6 g of the target substance as a white solid.
Melting point: 223 to 225°C

### Step 3: Synthesis of 6-(((2-fluoroisonicotinimidamide)oxy)carbonyl)-N-(5-fluoropyridin-2-yl)-4-hydroxy-2-oxo-1,2,5,6-tetrahydropyridine-3-carboxamide

To a mixed solution of 100 mg of 5-((5-fluoropyridin-2-yl)carbamoyl)-4-hydroxy-6-oxo-1,2,3,6-tetrahydropyridine-2-carboxylic acid, 55 mg of 2-fluoro-N-hydroxyisonicotinimidamide, and 4 ml of N,N-dimethylformamide, 72 mg of 1-(3-dimethylaminopropyl)-3-ethylcarbodiimide hydrochloride and 5 mg of 1-hydroxy-7-azabenzotriazole were added, and the resultant was stirred at room temperature for 1 hour. After the completion of the reaction, 12 ml of water and 1 ml of 1 mol/l hydrochloric acid were added, and a solid precipitated was collected by filtration. The solid obtained was washed with water, giving 80 mg of the target substance as a white solid.
Melting point: 146 to 148°C

The presently invented compound and production intermediates can be synthesized according to Production Methods and Synthesis Examples. Examples of aryltetrahydropyridine compounds produced in the same manner as in Synthesis Examples 1 to 3 are shown in Table 7 to Table 12, but the aryltetrahydropyridine compound of the present invention is not limited to them. Examples of production intermediates produced in the same manner as in Intermediate Synthesis Examples 1 and 2 are shown in Table 13 to Table 15, but the production intermediate of the present invention is not limited to them.

The notations Q-1, Q-2, Q-3, Q-4, Q-5, Q-6, Q-7, Q-8, Q-9, Q-10, Q-11, Q-12a, Q-12b, Q-13, Q-14, Q-20, Q-22, Q-29, Q-31a, Q-45a, and Q-52a in the tables indicate the following structures.

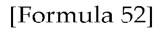

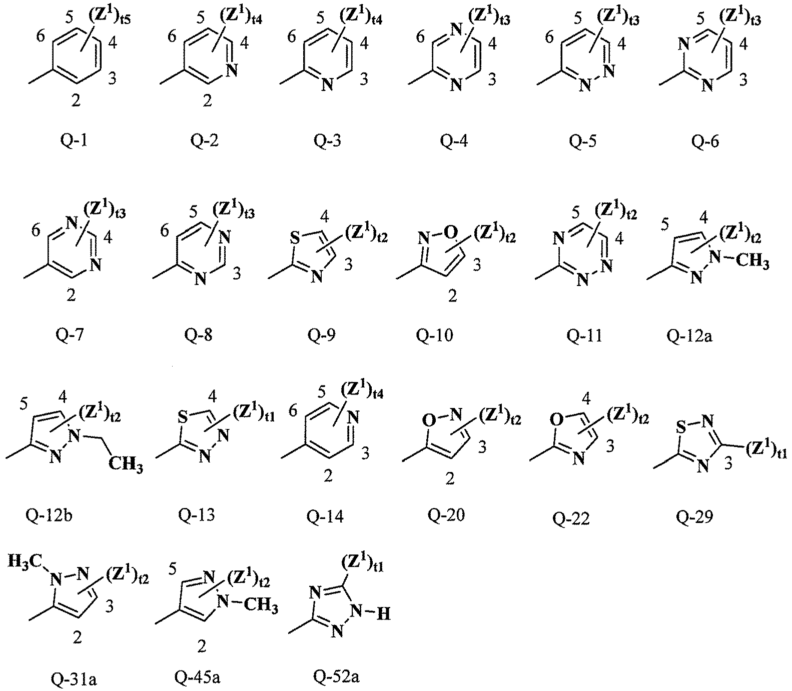

The notations G-1, G-2, G-3, G-4, G-5, G-6a, G-7, G-8a, G-9, G-10, G-11, G-12, G-13, G-14, G-15, G-16, G-17, G-18, G-19, G-20, G-27, G-28, and G-40 indicate the following structures.

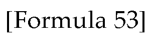

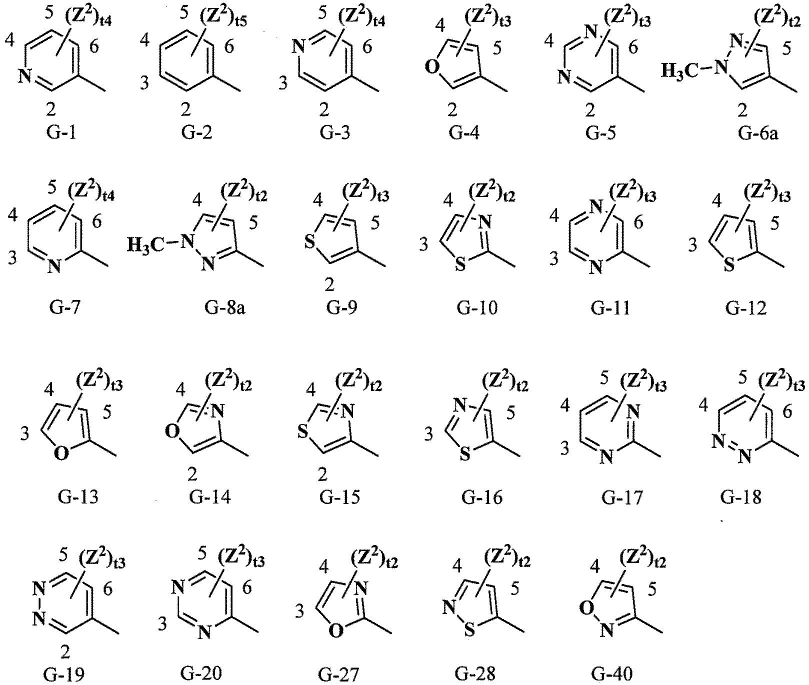

The notations D-1, D-2, and D-3 indicate the following structures.

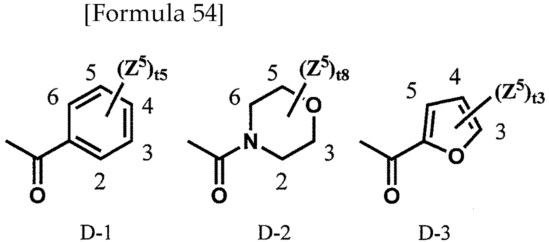

The numbers given to the structural formulas are each a number indicating the position of substitution with the substituent (Z¹)ₜ₁, (Z¹)ₜ₂, (Z¹)ₜ₃, (Z¹)ₜ₄, (Z¹)ₜ₅, (Z²)ₜ₂, (Z²)ₜ₃, (Z²)ₜ₄, (Z²)ₜ₅, (Z⁵)ₜ₃, (Z⁵)ₜ₅ or (Z⁵)ₜ₈; for example, "Q-1(4-F)", "G-2", and "G-1(2-OCH₃, 4-OCH₃)" in the tables mean "4-fluorophenyl", "phenyl", and "2,6-dimethoxypyridin-3-yl", respectively.

The notations "i-Bu" and "c-Pr" in the tables indicate an isobutyl group and a cyclopropyl group, respectively.

"m.p." is melting point (unit: °C), and the notation "*1" indicates that the compound is an oily substance or resin-like compound without melting point. ">" in description of melting point in the tables indicates that the melting point of the compound is higher than the temperature shown; for example, the notation ">300" indicates that the compound did not melt at 300°C.

**[Table 7]**

| 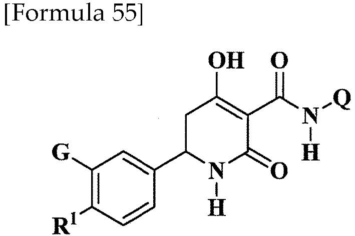 | | | | |
|---|---|---|---|---|
| No. | R¹ | Q | G | m.p.(°C) |
| 1-001 | CF₃ | Q-1(4-F) | G-1(4-F) | 200-202 |
| 1-002 | CF₃ | Q-1(4-F) | G-1(5-F) | 188-190 |
| 1-003 | CF₃ | Q-1(4-F) | G-1(4-OCH₃) | 95-97 |
| 1-004 | CF₃ | Q-1(4-F) | G-2 | 96-98 |
| 1-005 | CF₃ | Q-1(4-F) | G-2(2-F) | 177-179 |
| 1-006 | CF₃ | Q-1(4-F) | G-2(3-F) | 157-159 |
| 1-007 | CF₃ | Q-1(4-F) | G-2(4-F) | 176-178 |
| 1-008 | CF₃ | Q-1(4-F) | G-2(2-SCH₃) | 195-197 |
| 1-009 | CF₃ | Q-1(4-F) | G-2(4-SCH₃) | 121-123 |
| 1-010 | CF₃ | Q-1(4-F) | G-2(2-COCH₃) | 181-183 |
| 1-011 | CF₃ | Q-1(4-F) | G-2(3-COCH₃) | 159-161 |
| 1-012 | CF₃ | Q-1(4-F) | G-2(4-COCH₃) | 176-178 |
| 1-013 | CF₃ | Q-1(4-F) | G-1 | 232-234 |
| 1-014 | CF₃ | Q-1(4-F) | G-3 | 176-178 |
| 1-015 | CF₃ | Q-1(4-F) | G-4 | 174-176 |
| 1-016 | CF₃ | Q-1(4-F) | G-2(2-OCH₃) | 187-189 |
| 1-017 | CF₃ | Q-1(4-F) | G-2(3-OCH₃) | 137-139 |
| 1-018 | CF₃ | Q-1(4-F) | G-2(4-OCH₃) | 174-176 |
| 1-019 | CF₃ | Q-1(4-F) | G-2(4-SO₂CH₃) | 204-206 |
| 1-020 | CF₃ | Q-1(4-F) | G-1(2-OCH₃,4-OCH₃) | 177-179 |
| 1-021 | CF₃ | Q-1(4-F) | G-1(4-CF₃) | 199-200 |
| 1-022 | CF₃ | Q-1(4-F) | G-2(3-CN) | 164-166 |
| 1-023 | CF₃ | Q-1(4-F) | G-2(4-CN) | 209-211 |
| 1-024 | CF₃ | Q-1(4-F) | G-1(4-CN) | 226-228 |

**[Table 8]**

| 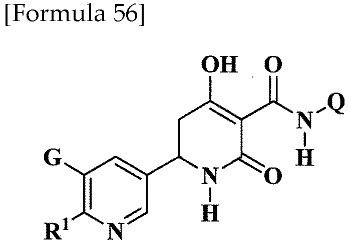 | | | | |
|---|---|---|---|---|
| No. | R¹ | Q | G | m.p.(°C) |
| 2-001 | Cl | Q-1(4-F) | G-1(4-F) | 210-212 |
| 2-002 | H | Q-1(4-F) | G-2(2-CF₃) | 212-216 |
| 2-003 | H | Q-1(4-F) | G-2(3-CF₃) | 175-177 |
| 2-004 | H | Q-1(4-F) | G-2(4-CF₃) | 191-193 |
| 2-005 | H | Q-1(4-F) | G-1(4-F) | 236-238 |
| 2-006 | H | Q-1(4-F) | G-1(4-OCHF₂) | 200-202 |
| 2-007 | H | Q-1(4-F) | G-1(4-OCH₂CH₂CH₃) | 187-189 |
| 2-008 | Cl | Q-2(4-F) | G-1(4-F) | 131-133 |
| 2-009 | Cl | Q-3(4-F) | G-1(4-F) | 174-176 |
| 2-010 | Cl | Q-2(4-Cl) | G-1(4-F) | 169-171 |
| 2-011 | Cl | Q-4(4-Cl) | G-1(4-F) | 223-225 |
| 2-012 | Cl | Q-2(4-CF₃) | G-1(4-F) | 197-199 |
| 2-013 | Cl | Q-2(4-CN) | G-1(4-F) | 105-107 |
| 2-014 | Cl | Q-1(4-F) | G-1(4-OCH₃) | 172-174 |
| 2-015 | Cl | Q-1(4-SO₂CH₃) | G-1(4-OCH₃) | 186-188 |
| 2-016 | Cl | Q-2(4-F) | G-1(4-OCH₃) | 189-192 |
| 2-017 | Cl | Q-2(4-Cl) | G-1(4-OCH₃) | 201-204 |
| 2-018 | Cl | Q-3(4-F) | G-6a | 139-141 |
| 2-019 | Cl | Q-3(4-F) | G-1 | 176-178 |
| 2-020 | Cl | Q-3(4-F) | G-3 | 174-176 |
| 2-021 | Cl | Q-3(4-F) | G-4 | 131-134 |
| 2-022 | Cl | Q-3(4-F) | G-1(2-OCH₃) | 129-131 |
| 2-023 | Cl | Q-2(4-F) | G-1 | 144-147 |
| 2-024 | Cl | Q-2(4-F) | G-5 | 164-166 |
| 2-025 | Cl | Q-2(4-F) | G-1(2-OCH₃) | 138-140 |
| 2-026 | Cl | Q-7(4-CF₃) | G-1(4-F) | 197-199 |
| 2-027 | Cl | Q-2(4-F) | G-2(4-F) | 186-188 |
| 2-028 | Cl | Q-2(4-F) | G-1(5-F) | 124-126 |
| 2-029 | Cl | Q-2(4-F) | G-1(5-OCH₃) | 117-119 |
| 2-030 | Cl | Q-3(4-F) | G-2(4-F) | 162-164 |
| 2-031 | Cl | Q-3(4-F) | G-3(3-OCH₃) | 169-171 |
| 2-032 | Cl | Q-3(4-F) | G-2 | 122-124 |
| 2-033 | Cl | Q-2(4-F) | G-1(6-OCH₃) | 164-166 |
| 2-034 | Cl | Q-3(4-F) | G-1(2-F) | 161-163 |
| 2-035 | Cl | Q-1(4-Cl) | G-1(4-F) | 199-201 |
| 2-036 | Cl | Q-1(4-Br) | G-1(4-F) | 202-204 |
| 2-037 | Cl | Q-1(4-CF₃) | G-1(4-F) | 202-204 |
| 2-038 | Cl | Q-1(4-SCF₃) | G-1(4-F) | 196-198 |
| 2-039 | Cl | Q-1(4-SCH₃) | G-1(4-F) | 174-176 |
| 2-040 | Cl | Q-1(3-F,4-F) | G-1(4-F) | 142-144 |
| 2-041 | Cl | Q-1(2-F,4-F) | G-1(4-F) | 167-169 |
| 2-042 | Cl | Q-1(4-F) | G-1(4-Cl) | 234-236 |
| 2-043 | Cl | Q-1(4-SOCH₃) | G-1(4-Cl) | 237-239 |
| 2-044 | Cl | Q-3(4-CN) | G-1(4-Cl) | 199-201 |
| 2-045 | Cl | Q-4(4-Cl) | G-1(4-Cl) | 209-211 |
| 2-046 | Cl | Q-5(4-Cl) | G-1(4-Cl) | 206-208 |
| 2-047 | Cl | Q-3(4-Cl) | G-3 | 194-196 |
| 2-048 | Cl | Q-2(4-Cl) | G-1(5-F) | 224-226 |
| 2-049 | Cl | Q-3(4-F) | G-1(5-F) | 192-194 |
| 2-050 | Cl | Q-4(4-Br) | G-1(5-F) | 240-242 |
| 2-051 | Cl | Q-6(4-CF₃) | G-1(5-F) | 195-197 |
| 2-052 | Cl | Q-5(4-Cl) | G-1(5-F) | 201-203 |
| 2-053 | Cl | Q-2(4-Br) | G-1(5-F) | 224-226 |
| 2-054 | Cl | Q-2(4-CF₃) | G-1(5-F) | 185-187 |
| 2-055 | Cl | Q-2(4-CN) | G-1(5-F) | 237-239 |
| 2-056 | Cl | Q-3(4-CF₃) | G-1(5-F) | 205-207 |
| 2-057 | Cl | Q-3(4-CN) | G-1(5-F) | 246-248 |
| 2-058 | Cl | Q-1(4-F) | G-1(5-F) | 231-233 |
| 2-059 | Cl | Q-4(4-CF₃) | G-1(5-F) | 135-137 |
| 2-060 | Cl | Q-6(4-Cl) | G-1(5-F) | 220-222 |
| 2-061 | Cl | Q-6(4-Br) | G-1(5-F) | 217-219 |
| 2-062 | Cl | Q-1(4-CH₃) | G-1(4-F) | 219-221 |
| 2-063 | Cl | Q-3(4-CH₃) | G-1(4-F) | 171-173 |
| 2-064 | Cl | Q-3(4-Cl) | G-2(4-CH₂CH₃) | 174-176 |
| 2-065 | Cl | Q-3(4-Cl) | G-2(4-CH=CH₂) | 149-151 |
| 2-066 | Cl | Q-3(4-Cl) | G-2(4-COCH₃) | 187-189 |
| 2-067 | Cl | Q-3(4-Cl) | G-2(3-CH₃) | 161-163 |
| 2-068 | Cl | Q-3(4-Cl) | G-2(3-OCH₃) | 112-114 |
| 2-069 | Cl | Q-3(4-Cl) | G-2(3-COCH₃) | 131-133 |
| 2-070 | Cl | Q-1(4-CN) | G-2(4-F) | 205-207 |
| 2-071 | Cl | Q-1(4-CN) | G-1(5-F) | 160-162 |
| 2-072 | Cl | Q-1(4-CN) | G-1(4-OCH₃) | 177-179 |
| 2-073 | Cl | Q-7(4-CF₃) | G-2(4-F) | 185-187 |
| 2-074 | Cl | Q-7(4-CF₃) | G-1(5-F) | 246-248 |
| 2-075 | Cl | Q-7(4-CF₃) | G-1(4-OCH₃) | 222-224 |
| 2-076 | Cl | Q-3(4-Cl) | G-1(4-NO₂) | 204-206 |
| 2-077 | Cl | Q-3(4-Cl) | G-2(4-OCH₃) | 122-124 |
| 2-078 | Cl | Q-3(4-Cl) | G-2(4-SCH₃) | 122-124 |
| 2-079 | Cl | Q-3(4-Cl) | G-2(4-CN) | 139-141 |
| 2-080 | Cl | Q-3(4-Cl) | G-2(3-F) | 182-184 |
| 2-081 | Cl | Q-3(4-Cl) | G-2(3-SCH₃) | 150-152 |
| 2-082 | Cl | Q-3(4-Cl) | G-2 | 129-131 |
| 2-083 | Cl | Q-1(4-CN) | G-1(4-F) | 197-199 |
| 2-084 | Cl | Q-3(4-Cl) | G-1(4-F) | 205-207 |
| 2-085 | Cl | Q-4(4-Br) | G-1(4-F) | 215-217 |
| 2-086 | Cl | Q-3(4-F,6-F) | G-1(4-F) | 207-209 |
| 2-087 | Cl | Q-6(4-Cl) | G-1(4-F) | 190-192 |
| 2-088 | Cl | Q-3(4-CN) | G-1(4-F) | 195-197 |
| 2-089 | Cl | Q-1(4-F) | G-2 | 222-224 |
| 2-090 | Cl | Q-1(4-CN) | G-2 | 220-222 |
| 2-091 | Cl | Q-2(4-F) | G-2 | 205-207 |
| 2-092 | Cl | Q-2(4-Cl) | G-2 | 229-231 |
| 2-093 | Cl | Q-2(4-CN) | G-2 | 174-176 |
| 2-094 | Cl | Q-3(4-CN) | G-2 | 171-173 |
| 2-095 | Cl | Q-4(4-Cl) | G-2 | 217-219 |
| 2-096 | Cl | Q-3 | G-1(4-F) | 99-101 |
| 2-097 | Cl | Q-2(4-CN) | G-1 | 222-224 |
| 2-098 | Cl | Q-4(4-Cl) | G-1 | 186-188 |
| 2-099 | Cl | Q-4(4-Br) | G-1 | 234-236 |
| 2-100 | Cl | Q-6(4-Cl) | G-1 | 237-239 |
| 2-101 | Cl | Q-8 | G-1(4-F) | 121-123 |
| 2-102 | Cl | Q-2(4-OCH₃) | G-1(4-F) | 136-138 |
| 2-103 | Cl | Q-3(4-F,6-Cl) | G-1(4-F) | 129-131 |
| 2-104 | Cl | Q-3(4-F,6-CH₃) | G-1(4-F) | 159-161 |
| 2-105 | Cl | Q-2(2-F,4-CH₃) | G-1(4-F) | 211-213 |
| 2-106 | Cl | Q-2(2-CH₃,4-F) | G-1(4-F) | 110-112 |
| 2-107 | Cl | Q-3(4-F,5-CH₃) | G-1(4-F) | 181-183 |
| 2-108 | Cl | Q-3(3-CH₃,4-F) | G-1(4-F) | 208-210 |
| 2-109 | Cl | Q-2(2-F,4-F) | G-1(4-F) | 200-201 |
| 2-110 | Cl | Q-3(4-Cl,6-Cl) | G-1(4-F) | 138-140 |
| 2-111 | Cl | Q-3(4-Cl,6-F) | G-1(4-F) | 186-188 |
| 2-112 | Cl | Q-3(4-CH₃,6-F) | G-1(4-F) | 151-153 |
| 2-113 | Cl | Q-1(4-F) | G-3(3-F) | 228-230 |
| 2-114 | Cl | Q-2(4-F) | G-3(3-F) | 186-188 |
| 2-115 | Cl | Q-3(4-F) | G-3(3-F) | 208-210 |
| 2-116 | Cl | Q-2(2-CN,4-Cl) | G-1(4-F) | 209-211 |
| 2-117 | Cl | Q-2(4-Cl,6-OCH₃) | G-1(4-F) | 201-203 |
| 2-118 | Cl | Q-2(2-OCH₃,4-Cl) | G-1(4-F) | 129-131 |
| 2-119 | Cl | Q-2(4-CH₃) | G-1(4-F) | 161-163 |
| 2-120 | Cl | Q-6(4-CH₃) | G-1(4-F) | 97-99 |
| 2-121 | Cl | Q-1(4-OCF₃) | G-1(4-F) | 214-216 |
| 2-122 | Cl | Q-3(4-CF₃,6-F) | G-1(4-F) | 177-179 |
| 2-123 | Cl | Q-6(4-Br) | G-1(4-F) | 186-188 |
| 2-124 | Cl | Q-2(4-F,5-CH₃) | G-1(4-F) | 181-183 |
| 2-125 | Cl | Q-2(4-F,6-CH₃) | G-1(4-F) | 187-189 |
| 2-126 | Cl | Q-2(4-Cl,5-Cl) | G-1(4-F) | 169-171 |
| 2-127 | Cl | Q-3(4-Cl,6-CN) | G-1(4-F) | 182-184 |
| 2-128 | Cl | Q-2(2-Cl,4-Cl) | G-1(4-F) | 211-213 |
| 2-129 | Cl | Q-2(4-Cl,5-CH₃) | G-1(4-F) | 156-158 |
| 2-130 | Cl | Q-3(4-Cl,6-CH₃) | G-1(4-F) | 177-179 |
| 2-131 | Cl | Q-4(4-Cl,6-Cl) | G-1(4-F) | 186-188 |
| 2-132 | Cl | Q-3(3-F,4-Br) | G-1(4-F) | 171-173 |
| 2-133 | Cl | Q-4 | G-1(4-F) | 146-148 |
| 2-134 | Cl | Q-6 | G-1(4-F) | 151-153 |
| 2-135 | Cl | Q-9 | G-1(4-F) | 199-201 |
| 2-136 | Cl | Q-3(5-F) | G-1(4-F) | 124-126 |
| 2-137 | Cl | Q-2(5-F) | G-1(4-F) | 226-228 |
| 2-138 | Cl | Q-3(4-Br,6-F) | G-1(4-F) | 159-161 |
| 2-139 | Cl | Q-2(4-Br,5-F) | G-1(4-F) | 219-221 |
| 2-140 | Cl | Q-3(4-Br,5-F) | G-1(4-F) | 218-220 |
| 2-141 | Cl | Q-3(3-F) | G-1(4-F) | 187-189 |
| 2-142 | Cl | Q-3(6-F) | G-1(4-F) | 181-183 |
| 2-143 | Cl | Q-4(4-CN) | G-1(4-F) | 169-171 |
| 2-144 | Cl | Q-3(4-Br) | G-1(4-F) | 206-208 |
| 2-145 | Cl | Q-3(4-CF₃) | G-1(4-F) | 209-211 |
| 2-146 | Cl | Q-2(4-F) | G-1(5-Cl) | 122-124 |
| 2-147 | Cl | Q-4 | G-1(5-Cl) | 172-174 |
| 2-148 | Cl | Q-4 | G-1(5-CH₃) | 191-193 |
| 2-149 | Cl | Q-4 | G-1(4-CH₃) | 191-193 |
| 2-150 | Cl | Q-3(3-Cl) | G-1(4-F) | 187-189 |
| 2-151 | Cl | Q-3(3-CH₃) | G-1(4-F) | 197-199 |
| 2-152 | Cl | Q-3(4-NO₂) | G-1(4-F) | 202-204 |
| 2-153 | Cl | Q-3(5-CH₃) | G-1(4-F) | 107-109 |
| 2-154 | Cl | Q-9(4-CH₃) | G-1(4-F) | 207-209 |
| 2-155 | Cl | Q-2(4-Cl) | G-3(3-F) | 191-193 |
| 2-156 | Cl | Q-3(4-Cl) | G-3(3-F) | 180-182 |
| 2-157 | Cl | Q-3 | G-3(3-F) | 201-203 |
| 2-158 | Cl | Q-1(4-CN) | G-3(3-F) | 219-221 |
| 2-159 | Cl | Q-3(4-CN) | G-3(3-F) | 221-223 |
| 2-160 | Cl | Q-3(4-F,6-F) | G-3(3-F) | 199-201 |
| 2-161 | Cl | Q-4 | G-3(3-F) | 205-207 |
| 2-162 | Cl | Q-3(4-CH₃) | G-3(3-F) | 192-194 |
| 2-163 | Cl | Q-3(4-Cl,6-F) | G-3(3-F) | 183-185 |
| 2-164 | Cl | Q-9 | G-3(3-F) | 212-214 |
| 2-165 | Cl | Q-6(4-Cl) | G-3(3-F) | 199-201 |
| 2-166 | Cl | Q-3(6-F) | G-3(3-F) | 202-204 |
| 2-167 | CF₃ | Q-3(4-F) | G-1(4-F) | 178-180 |
| 2-168 | CF₃ | Q-2(4-F) | G-1(4-F) | 174-176 |
| 2-169 | CF₃ | Q-3(4-Cl) | G-1(4-F) | 191-193 |
| 2-170 | CF₃ | Q-2(4-Cl) | G-1(4-F) | 131-132 |
| 2-171 | CF₃ | Q-3(4-CH₃) | G-1(4-F) | 111-113 |
| 2-172 | CF₃ | Q-12a | G-1(4-F) | 191-193 |
| 2-173 | CF₃ | Q-9(4-CH₃) | G-1(4-F) | 199-201 |
| 2-174 | CF₃ | Q-9(4-Cl) | G-1(4-F) | 159-161 |
| 2-175 | CF₃ | Q-4(4-Cl) | G-1(4-F) | 204-206 |
| 2-176 | CF₃ | Q-4 | G-1(4-F) | 194-196 |
| 2-177 | CF₃ | Q-3(4-Br) | G-1(4-F) | 186-188 |
| 2-178 | CF₃ | Q-1(4-F) | G-1(4-F) | 201-203 |
| 2-179 | CF₃ | Q-3(4-CF₃) | G-1(4-F) | 204-206 |
| 2-180 | CF₃ | Q-6(4-Cl) | G-1(4-F) | 182-184 |
| 2-181 | CF₃ | Q-3(4-Cl,6-F) | G-1(4-F) | 181-183 |
| 2-182 | CF₃ | Q-3(4-F,6-F) | G-1(4-F) | 199-201 |
| 2-183 | CF₃ | Q-1(4-CN) | G-1(4-F) | 172-174 |
| 2-184 | CF₃ | Q-9 | G-1(4-F) | 199-201 |
| 2-185 | CF₃ | Q-13(4-CH₃) | G-1(4-F) | 191-193 |
| 2-186 | Cl | Q-3(4-F) | G-3(3-Cl) | 162-164 |
| 2-187 | Cl | Q-3(4-Cl) | G-3(3-Cl) | 124-126 |
| 2-188 | Cl | Q-4(4-Cl) | G-3(3-Cl) | 131-133 |
| 2-189 | Cl | Q-2(4-Cl) | G-3(3-Cl) | 171-173 |
| 2-190 | Cl | Q-3 | G-3(3-Cl) | 142-144 |
| 2-191 | CF₃ | Q-3(4-F) | G-3(3-F) | 169-171 |
| 2-192 | CF₃ | Q-2(4-F) | G-3(3-F) | 149-151 |
| 2-193 | CF₃ | Q-3(4-Cl) | G-3(3-F) | 112-114 |
| 2-194 | CF₃ | Q-2(4-Cl) | G-3(3-F) | 136-138 |
| 2-195 | CF₃ | Q-3(4-CH₃) | G-3(3-F) | 116-118 |
| 2-196 | CF₃ | Q-12a | G-3(3-F) | 101-103 |
| 2-197 | CF₃ | Q-9(4-CH₃) | G-3(3-F) | 111-113 |
| 2-198 | CF₃ | Q-4(4-Cl) | G-3(3-F) | 154-156 |
| 2-199 | CF₃ | Q-3 | G-3(3-F) | 151-153 |
| 2-200 | CF₃ | Q-3 | G-1(4-F) | 136-138 |
| 2-201 | CF₃ | Q-4 | G-3(3-F) | 157-159 |
| 2-202 | CF₃ | Q-3(4-Br) | G-3(3-F) | 174-176 |
| 2-203 | CF₃ | Q-1(4-F) | G-3(3-F) | 172-174 |
| 2-204 | CF₃ | Q-3(4-CF₃) | G-3(3-F) | 116-118 |
| 2-205 | CF₃ | Q-6(4-Cl) | G-3(3-F) | 141-143 |
| 2-206 | CF₃ | Q-3(4-Cl,6-F) | G-3(3-F) | 154-156 |
| 2-207 | CF₃ | Q-3(4-F,6-F) | G-3(3-F) | 159-161 |
| 2-208 | CF₃ | Q-1(4-CN) | G-3(3-F) | 144-146 |
| 2-209 | CF₃ | Q-9 | G-3(3-F) | 114-116 |
| 2-210 | CF₃ | Q-13(4-CH₃) | G-3(3-F) | 168-170 |
| 2-211 | CF₃ | Q-3(3-F) | G-3(3-F) | 149-151 |
| 2-212 | CF₃ | Q-3(6-F) | G-3(3-F) | 99-101 |
| 2-213 | CF₃ | Q-3(4-CN) | G-3(3-F) | 112-114 |
| 2-214 | CF₃ | Q-2(4-CN) | G-3(3-F) | 122-124 |
| 2-215 | CF₃ | Q-2(4-CF₃) | G-3(3-F) | 112-114 |
| 2-216 | CF₃ | Q-4(4-Br) | G-3(3-F) | 177-179 |
| 2-217 | CF₃ | Q-6(4-Br) | G-3(3-F) | 144-146 |
| 2-218 | CF₃ | Q-1(4-CH₃) | G-3(3-F) | 187-189 |
| 2-219 | CF₃ | Q-3(3-Cl) | G-3(3-F) | 164-166 |
| 2-220 | Cl | Q-3(4-Cl) | G-2(4-F) | 146-148 |
| 2-221 | Cl | Q-2(4-Cl) | G-2(4-F) | 199-201 |
| 2-222 | Cl | Q-3(4-CH₃) | G-2(4-F) | 162-164 |
| 2-223 | Cl | Q-12a | G-2(4-F) | 107-109 |
| 2-224 | Cl | Q-9(4-CH₃) | G-2(4-F) | 156-158 |
| 2-225 | Cl | Q-4(4-Cl) | G-2(4-F) | 156-158 |
| 2-226 | Cl | Q-3 | G-2(4-F) | 141-143 |
| 2-227 | Cl | Q-4 | G-2(4-F) | 132-134 |
| 2-228 | Cl | Q-3(4-CN) | G-2(4-F) | 122-124 |
| 2-229 | Cl | Q-1(4-F) | G-2(4-F) | 187-189 |
| 2-230 | Cl | Q-3(4-CF₃) | G-2(4-F) | 164-166 |
| 2-231 | Cl | Q-6(4-Cl) | G-2(4-F) | 171-173 |
| 2-232 | Cl | Q-3(4-Cl,6-F) | G-2(4-F) | 174-176 |
| 2-233 | Cl | Q-2(4-CN) | G-2(4-F) | 200-202 |
| 2-234 | Cl | Q-2(4-CF₃) | G-2(4-F) | 181-183 |
| 2-235 | Cl | Q-9 | G-2(4-F) | 154-156 |
| 2-236 | Cl | Q-13(4-CH₃) | G-2(4-F) | 184-186 |
| 2-237 | Cl | Q-11 | G-2(4-F) | 161-163 |
| 2-238 | Cl | Q-6 | G-2(4-F) | 127-129 |
| 2-239 | Cl | Q-13 | G-2(4-F) | 146-148 |
| 2-240 | Cl | Q-10(3-CH₃) | G-2(4-F) | 172-174 |
| 2-241 | Cl | Q-1(4-OH) | G-2(4-F) | 129-131 |
| 2-242 | Cl | Q-1(4-CH=CH₂) | G-2(4-F) | 182-184 |
| 2-243 | Cl | Q-1(4-C≡CH) | G-2(4-F) | 164-166 |
| 2-244 | CF₃ | Q-3(4-F) | G-2(4-F) | 111-113 |
| 2-245 | CF₃ | Q-3(4-F) | G-2(4-OCH₃) | 167-169 |
| 2-246 | CF₃ | Q-3(4-F) | G-2(3-F) | 159-161 |
| 2-247 | CF₃ | Q-3(4-F) | G-2(3-OCH₃) | 144-146 |
| 2-248 | CF₃ | Q-3(4-F) | G-2(2-CH₃) | 176-178 |
| 2-249 | CF₃ | Q-3(4-F) | G-2(2-F) | 216-218 |
| 2-250 | CF₃ | Q-3(4-F) | G-2(2-OCH₃) | 174-176 |
| 2-251 | CF₃ | Q-3(4-F) | G-1 | 136-138 |
| 2-252 | Cl | Q-3(4-F) | G-2(3-CH₃) | 176-178 |
| 2-253 | Cl | Q-2(4-F) | G-2(3-CH₃) | 179-181 |
| 2-254 | Cl | Q-2(4-Cl) | G-2(3-CH₃) | 201-203 |
| 2-255 | Cl | Q-3(4-CH₃) | G-2(3-CH₃) | 179-181 |
| 2-256 | Cl | Q-12a | G-2(3-CH₃) | 171-173 |
| 2-257 | Cl | Q-9(4-CH₃) | G-2(3-CH₃) | 207-209 |
| 2-258 | Cl | Q-1(4-OCH₃) | G-2(3-CH₃) | 202-204 |
| 2-259 | Cl | Q-4(4-Cl) | G-2(3-CH₃) | 196-198 |
| 2-260 | Cl | Q-3 | G-2(3-CH₃) | 111-113 |
| 2-261 | Cl | Q-4 | G-2(3-CH₃) | 181-183 |
| 2-262 | Cl | Q-3(4-CN) | G-2(3-CH₃) | 179-181 |
| 2-263 | Cl | Q-1(4-F) | G-2(3-CH₃) | 202-204 |
| 2-264 | Cl | Q-6(4-Cl) | G-2(3-CH₃) | 174-176 |
| 2-265 | Cl | Q-3(4-Cl,6-F) | G-2(3-CH₃) | 181-183 |
| 2-266 | Cl | Q-2(4-CN) | G-2(3-CH₃) | 189-191 |
| 2-267 | Cl | Q-2(4-CF₃) | G-2(3-CH₃) | 204-206 |
| 2-268 | Cl | Q-9 | G-2(3-CH₃) | 197-199 |
| 2-269 | Cl | Q-1(4-CN) | G-2(3-CH₃) | 172-174 |
| 2-270 | Cl | Q-11 | G-2(3-CH₃) | 152-154 |
| 2-271 | Cl | Q-6 | G-2(3-CH₃) | 126-128 |
| 2-272 | Cl | Q-3(3-CH₃) | G-2(3-CH₃) | 179-181 |
| 2-273 | Cl | Q-3(5-CH₃) | G-2(3-CH₃) | 182-184 |
| 2-274 | Cl | Q-2(2-Cl) | G-2(3-CH₃) | 187-189 |
| 2-275 | Cl | Q-2(2-F) | G-2(3-CH₃) | 164-166 |
| 2-276 | Cl | Q-3(3-Cl) | G-2(3-CH₃) | 191-193 |
| 2-277 | Cl | Q-3(3-F) | G-2(3-CH₃) | 186-188 |
| 2-278 | Cl | Q-3(4-NO₂) | G-2(3-CH₃) | 187-189 |
| 2-279 | Cl | Q-1(4-NO₂) | G-2(3-CH₃) | 172-174 |
| 2-280 | Cl | Q-1(4-C≡CH) | G-2(3-CH₃) | 169-171 |
| 2-281 | Cl | Q-6(3-Cl) | G-2(3-CH₃) | 147-149 |
| 2-282 | Cl | Q-1(4-CH₃) | G-2(3-CH₃) | 186-188 |
| 2-283 | Cl | Q-8(3-Cl) | G-2(3-CH₃) | 137-139 |
| 2-284 | Cl | Q-1(3-SCH₃) | G-2(3-CH₃) | 191-193 |
| 2-285 | Cl | Q-1(3-SO₂CH₃) | G-2(3-CH₃) | 156-158 |
| 2-286 | Cl | Q-22 | G-1(4-F) | *1 |
| 2-287 | Cl | Q-2 | G-1(4-F) | *1 |
| 2-288 | Cl | Q-14 | G-1(4-F) | *1 |
| 2-289 | Cl | Q-5 | G-1(4-F) | *1 |
| 2-290 | Cl | Q-31a | G-2(4-F) | 139-140 |
| 2-291 | Cl | Q-52a | G-2(4-F) | 152-154 |
| 2-292 | Cl | Q-1(4-C(O)OCH₃) | G-2(4-F) | 194-196 |
| 2-293 | Cl | Q-1 (4-C(O)OH) | G-2(4-F) | 231-233 |
| 2-294 | Cl | Q-1(4-C(O)N(H)CH₃) | G-2(4-F) | 212-214 |
| 2-295 | Cl | Q-1(4-C(O)CH₃) | G-2(4-F) | 211-213 |
| 2-296 | Cl | Q-1(4-C(O)OC(CH₃)₃) | G-2(4-F) | 156-158 |
| 2-297 | CF₃ | Q-3(4-F) | G-3(3-CF₃) | 129-131 |
| 2-298 | CF₃ | Q-3(4-F) | G-3(3-CH₃) | 206-208 |
| 2-299 | Cl | Q-3(4-CF₃) | G-3(3-F) | 182-184 |
| 2-300 | Cl | Q-4(4-Cl) | G-3(3-F) | 172-174 |
| 2-301 | Cl | Q-2(4-CN) | G-3(3-F) | 216-218 |
| 2-302 | Cl | Q-3(3-CH₃) | G-3(3-F) | 207-209 |
| 2-303 | Cl | Q-3(4-Br) | G-3(3-F) | 191-193 |
| 2-304 | Cl | Q-4(4-Br) | G-3(3-F) | 154-156 |
| 2-305 | Cl | Q-3(3-F) | G-3(3-F) | 209-211 |

**[Table 9]**

| 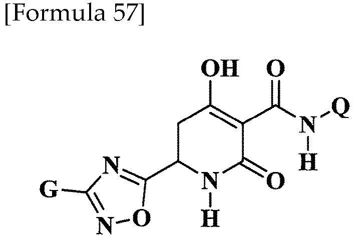 | | | |
|---|---|---|---|
| No. | Q | G | m.p.(°C) |
| 3-001 | Q-1(4-F) | G-2 | 200-202 |
| 3-002 | Q-1(4-F) | G-2(2-CF₃) | 177-179 |
| 3-003 | Q-1(4-F) | G-2(4-Cl) | 228-230 |
| 3-004 | Q-1(4-F) | G-2(4-CF₃) | 217-219 |
| 3-005 | Q-1(4-F) | G-2(3-CF₃) | 181-183 |
| 3-006 | Q-1(4-F) | G-7 | 206-208 |
| 3-007 | Q-1(4-F) | G-1 | 207-209 |
| 3-008 | Q-1(4-F) | G-2(4-SO₂CH₃) | 226-228 |
| 3-009 | Q-1(4-F) | G-7(4-F) | 201-203 |
| 3-010 | Q-1(4-F) | G-2(4-F) | 228-230 |
| 3-011 | Q-1(4-F) | G-2(3-F) | 221-223 |
| 3-012 | Q-1(4-F) | G-2(2-F) | 207-209 |
| 3-013 | Q-1(4-F) | G-6a(2-Cl) | 238-240 |
| 3-014 | Q-1(4-F) | G-8a | 230-232 |
| 3-015 | Q-1(4-F) | G-1(4-Cl) | 230-232 |
| 3-016 | Q-1(4-F) | G-1(4-F) | 216-218 |
| 3-017 | Q-2(4-Cl) | G-2 | 230-232 |
| 3-018 | Q-2(4-Cl) | G-2(3-F) | 225-227 |
| 3-019 | Q-2(4-Cl) | G-2(2-F) | 229-231 |
| 3-020 | Q-1(4-F) | G-7(3-F) | 222-224 |
| 3-021 | Q-1(4-F) | G-1(5-F) | 208-210 |
| 3-022 | Q-1(4-F) | G-3(3-F) | 216-218 |
| 3-023 | Q-1(4-F) | G-7(6-F) | 215-217 |
| 3-024 | Q-1(4-F) | G-9(5-Br) | 210-212 |
| 3-025 | Q-1(4-F) | G-7(5-F) | 193-195 |
| 3-026 | Q-1(4-F) | G-2(2-F,5-F) | 241-243 |
| 3-027 | Q-1(4-F) | G-2(3-F,4-F) | 212-214 |
| 3-028 | Q-1(4-F) | G-2(3-Br) | 199-201 |
| 3-029 | Q-1(4-F) | G-2(3-Cl) | 208-210 |
| 3-030 | Q-1(4-F) | G-2(3-CH₃) | 191-193 |
| 3-031 | Q-1(4-F) | G-3 | 239-241 |
| 3-032 | Q-2(4-F) | G-1(4-F) | 228-230 |
| 3-033 | Q-1(4-F) | G-2(2-F,3-F) | 242-244 |
| 3-034 | Q-1(4-F) | G-2(3-F,5-F) | 219-221 |
| 3-035 | Q-3(4-F) | G-1(4-F) | 214-216 |
| 3-036 | Q-3(4-F) | G-3(3-F) | 159-161 |
| 3-037 | Q-2(4-F) | G-3(3-F) | 216-218 |
| 3-038 | Q-2(4-F) | G-3 | 229-231 |
| 3-039 | Q-3(4-F) | G-7(3-F) | 187-189 |
| 3-040 | Q-3(4-F) | G-7(6-F) | 189-191 |
| 3-041 | Q-3(4-F) | G-7(5-F) | 206-208 |
| 3-042 | Q-3(4-F) | G-7(4-F) | 169-171 |
| 3-043 | Q-3(4-F) | G-6a(2-Cl) | 181-183 |
| 3-044 | Q-3(4-F) | G-3 | 197-199 |
| 3-045 | Q-3(4-F) | G-7 | 207-209 |
| 3-046 | Q-2(4-F) | G-7(3-F) | 191-193 |
| 3-047 | Q-2(4-F) | G-7(6-F) | 197-199 |
| 3-048 | Q-2(4-F) | G-6a(2-Cl) | 222-224 |
| 3-049 | Q-3(4-F) | G-2(2-F,3-F) | 192-194 |
| 3-050 | Q-3(4-F) | G-2(2-F,5-F) | 207-209 |
| 3-051 | Q-3(4-F) | G-2(3-Br) | 197-199 |
| 3-052 | Q-3(4-F) | G-2(3-Cl) | 199-201 |
| 3-053 | Q-3(4-F) | G-2(3-CH₃) | 194-196 |
| 3-054 | Q-3(4-F) | G-1(4-Cl) | 241-243 |
| 3-055 | Q-3(4-F) | G-2 | 187-189 |
| 3-056 | Q-3(4-F) | G-2(4-F) | 177-179 |
| 3-057 | Q-3(4-F) | G-2(3-F) | 191-193 |
| 3-058 | Q-3(4-F) | G-2(2-F) | 196-198 |
| 3-059 | Q-3(4-F) | G-2(3-F,5-F) | 186-187 |
| 3-060 | Q-2(4-Cl) | G-3(3-F) | 217-221 |
| 3-061 | Q-3(4-F) | G-3(3-CH₃) | 101-103 |
| 3-062 | Q-3(4-F) | G-2(3-F,4-F) | 194-196 |
| 3-063 | Q-3(4-F) | G-2(2-F,4-F) | 192-194 |
| 3-064 | Q-3(4-F) | G-3(2-F) | 204-206 |
| 3-065 | Q-3 | G-3(3-F) | 194-196 |
| 3-066 | Q-4(4-Br) | G-3(3-F) | 199-201 |
| 3-067 | Q-6(4-Cl) | G-3(3-F) | 199-201 |
| 3-068 | Q-3(4-Br) | G-3(3-F) | 221-223 |
| 3-069 | Q-3(4-Cl) | G-3(3-F) | 209-211 |
| 3-070 | Q-2(4-CN) | G-3(3-F) | 231-233 |
| 3-071 | Q-1(4-CN) | G-3(3-F) | 203-205 |
| 3-072 | Q-3(4-CN) | G-3(3-F) | 97-99 |
| 3-073 | Q-3(4-F,6-F) | G-3(3-F) | 97-99 |
| 3-074 | Q-1(4-Cl) | G-3(3-F) | 209-211 |
| 3-075 | Q-4 | G-3(3-F) | 167-169 |
| 3-076 | Q-4(4-Cl) | G-3(3-F) | 187-189 |
| 3-077 | Q-3(4-Cl,6-F) | G-3(3-F) | 131-133 |
| 3-078 | Q-3(4-CF₃) | G-3(3-F) | 129-131 |
| 3-079 | Q-2(4-Cl) | G-1(4-F) | 224-226 |
| 3-080 | Q-2(4-Cl) | G-2(4-F) | 241-243 |
| 3-081 | Q-2(4-F) | G-7(4-F) | 198-200 |
| 3-082 | Q-2(4-F) | G-2(4-F) | 224-226 |
| 3-083 | Q-3(4-F) | G-1(5-F) | 189-191 |
| 3-084 | Q-3(4-F) | G-2(3-CF₃) | 184-186 |
| 3-085 | Q-3(4-F) | G-8a | 207-209 |
| 3-086 | Q-3(4-F) | G-9(5-Br) | 144-146 |
| 3-087 | Q-3(4-F) | G-1(4-CH₃) | 232-234 |
| 3-088 | Q-3(4-F) | G-10 | 185-187 |
| 3-089 | Q-1(4-F) | G-3(3-CH₃) | 207-209 |
| 3-090 | Q-1(4-F) | G-3(3-Cl) | 196-198 |
| 3-091 | Q-3(4-F) | G-11 | 217-219 |
| 3-092 | Q-3(4-F) | G-12 | 209-211 |
| 3-093 | Q-3(4-F) | G-13 | 212-214 |
| 3-094 | Q-3(4-F) | G-13(3-CH₃) | 211-212 |
| 3-095 | Q-3(4-F) | G-14 | 207-209 |
| 3-096 | Q-3(4-F) | G-15 | 199-201 |
| 3-097 | Q-3(4-F) | G-16 | 211-212 |
| 3-098 | Q-3(4-F) | G-3(3-Cl) | 174-177 |
| 3-099 | Q-3(4-F) | G-3(3-OH) | 201-203 |
| 3-100 | Q-3(4-F) | G-3(3-OCH₃) | 144-146 |
| 3-101 | Q-3(4-F) | G-1(4-OH) | 214-216 |
| 3-102 | Q-3(4-Cl) | G-1(4-F) | 182-184 |
| 3-103 | Q-3 | G-1(4-F) | 184-186 |
| 3-104 | Q-3(4-CH₃) | G-1(4-F) | 185-187 |
| 3-105 | Q-9(4-CH₃) | G-1(4-F) | 208-210 |
| 3-106 | Q-1(2-F,4-Cl) | G-3(3-F) | 182-184 |
| 3-107 | Q-1(3-F,4-F) | G-3(3-F) | 174-176 |
| 3-108 | Q-1(3-F,5-F) | G-3(3-F) | 212-214 |
| 3-109 | Q-1(4-F) | G-9 | 222-224 |
| 3-110 | Q-3(4-F) | G-9 | 218-220 |
| 3-111 | Q-1(4-F) | G-17 | 226-228 |
| 3-112 | Q-3(4-F) | G-17 | 219-221 |
| 3-113 | Q-3(4-F) | G-6a(2-Cl,5-CH₃) | 159-161 |
| 3-114 | Q-3(4-F) | G-6a(2-Cl,5-Cl) | 171-173 |
| 3-115 | Q-1(4-F) | G-6a(2-Cl,5-Cl) | 199-201 |
| 3-116 | Q-2(4-OCH₃) | G-3(3-F) | 202-204 |
| 3-117 | Q-1(4-F) | G-2(4-N(CH₃)₂) | 222-225 |
| 3-118 | Q-3(4-F) | G-2(4-N(CH₃)₂) | 219-220 |
| 3-119 | Q-3(4-F) | G-7(3-CH₃) | 208-210 |
| 3-120 | Q-1(3-Cl,4-NO₂) | G-3(3-F) | 279-281 |
| 3-121 | Q-20(3-CH₃) | G-3(3-F) | 298-300 |
| 3-122 | Q-12a | G-3(3-F) | 201-203 |
| 3-123 | Q-45ₐ | G-3(3-F) | 209-211 |
| 3-124 | Q-3(4-F) | G-2(3-C(=NOCH₃)H) | 184-186 |
| 3-125 | Q-3(4-F) | G-2(3-C(=NOCH₃)CH₃) | 211-213 |
| 3-126 | Q-2 | G-3(3-F) | 191-193 |
| 3-127 | Q-3(4-CH₃) | G-2(4-F) | 182-184 |
| 3-128 | Q-3(4-CH₃) | G-2(3-F) | 167-169 |
| 3-129 | Q-3(4-CH₃) | G-2(2-F) | 157-159 |
| 3-130 | Q-3(4-F) | G-1(5-CH₃) | 229-231 |
| 3-131 | Q-1(4-F) | G-2(2-F,4-F) | 213-215 |
| 3-132 | Q-3 | G-2(2-F,4-F) | 196-198 |
| 3-133 | Q-3(4-F) | G-3(3-CF₃) | 209-211 |
| 3-134 | Q-3(4-F) | G-19 | 196-198 |
| 3-135 | Q-3(4-F) | G-6a(5-Cl) | 186-188 |
| 3-136 | Q-4(4-Cl) | G-6a(2-Cl,5-CH₃) | 179-181 |
| 3-137 | Q-3(4-F) | G-2(4-OCH₃) | 199-201 |
| 3-138 | Q-3(4-F) | G-2(3-F,4-F,5-F) | 214-216 |
| 3-139 | Q-3(4-F) | G-2(2-F,4-F,5-F) | 222-224 |
| 3-140 | Q-3(4-F) | G-2(2-Cl,4-F) | 162-164 |
| 3-141 | Q-3(4-F) | G-2(3-Cl,4-F) | 197-199 |
| 3-142 | Q-3(4-F) | G-2(2-F,4-Cl) | 201-203 |
| 3-143 | Q-3(4-F) | G-2(2-F,6-Cl) | 189-191 |
| 3-144 | Q-3(4-F) | G-2(2-F,4-F,6-F) | 181-183 |
| 3-145 | Q-3(4-F) | G-2(3-F,4-Cl) | 194-196 |
| 3-146 | Q-3(4-F) | G-2(3-Br,4-F) | 199-201 |
| 3-147 | Q-3(4-F) | G-7(4-Cl,6-Cl) | 192-194 |
| 3-148 | Q-3(4-F) | G-7(4-CF₃,6-Cl) | 177-179 |
| 3-149 | Q-3(4-F) | G-1(4-CF₃) | 197-199 |
| 3-150 | Q-3(4-CH₃) | G-3 | >300 |
| 3-151 | Q-3(4-CH₃) | G-1 | 238-240 |
| 3-152 | Q-3(4-CH₃) | G-7 | 203-205 |
| 3-153 | Q-3(4-F) | G-7(5-CH₃) | 194-196 |
| 3-154 | Q-12a | G-2(2-F,4-F) | 92-94 |
| 3-155 | Q-3(4-Cl) | G-2(2-F,4-F) | 206-208 |
| 3-156 | Q-2(4-F) | G-2(2-F,4-F) | 216-218 |
| 3-157 | Q-1(2-F,4-F) | G-2(2-F,4-F) | 221-223 |
| 3-158 | Q-3 | G-6a(2-Cl) | 196-198 |
| 3-159 | Q-3(4-F) | G-6a(2-Cl,5-CF₃) | 79-81 |
| 3-160 | Q-3(4-F) | G-6a(5-CF₃) | 79-81 |
| 3-161 | Q-1(3-F,4-F) | G-2(2-F,4-F) | 229-231 |
| 3-162 | Q-1(4-CN) | G-3(3-CH₃) | 194-196 |
| 3-163 | Q-3(5-CH₃) | G-3(3-F) | 181-183 |
| 3-164 | Q-3(4-F) | G-3(3-Br) | 181-183 |
| 3-165 | Q-3(4-CH₃) | G-3(3-F) | *1 |
| 3-166 | Q-1(2-F,6-F) | G-3(3-F) | *1 |
| 3-167 | Q-3(4-CH₃) | G-17 | 233-235 |
| 3-168 | Q-3(4-CH₃) | G-9 | 191-193 |
| 3-169 | Q-3(4-CH₃) | G-3(3-Cl) | 187-189 |
| 3-170 | Q-3 | G-6a(2-Cl,5-Cl) | 167-169 |
| 3-171 | Q-9 | G-6a(2-Cl,5-Cl) | 202-204 |
| 3-172 | Q-3(4-Cl,6-F) | G-3(3-CH₃) | 174-176 |
| 3-173 | Q-4(4-Cl) | G-3(3-CH₃) | 206-208 |
| 3-174 | Q-3(3-CH₃) | G-2(2-F,4-F) | 192-194 |
| 3-175 | Q-3(5-CH₃) | G-2(2-F,4-F) | 174-176 |
| 3-176 | Q-3(4-CH₃) | G-3(3-CH₃) | 117-119 |
| 3-177 | Q-2 | G-2(2-F,4-F) | 189-191 |
| 3-178 | Q-3(4-CH₃) | G-2(2-F,4-F) | 209-211 |
| 3-179 | Q-3(4-OCH₃) | G-3(3-F) | 201-203 |
| 3-180 | Q-3(4-Cl) | G-2(3-F) | 196-198 |
| 3-181 | Q-3(4-Cl) | G-3 | 221-223 |
| 3-182 | Q-3(4-Cl) | G-7 | 197-199 |
| 3-183 | Q-3(4-Cl) | G-3(3-Cl) | 169-171 |
| 3-184 | Q-3 | G-2(4-F) | 192-194 |
| 3-185 | Q-3 | G-2(3-F) | 152-154 |
| 3-186 | Q-3 | G-2(2-F) | 162-164 |
| 3-187 | Q-3 | G-3(3-Cl) | 177-179 |
| 3-188 | Q-3(4-F) | G-2(3-NO₂) | 174-176 |
| 3-189 | Q-3(4-F) | G-2(4-CN) | 224-226 |
| 3-190 | Q-3(4-F) | G-2(3-OCH₃) | 171-173 |
| 3-191 | Q-3(4-F) | G-2(3-OCF₃) | 164-166 |
| 3-192 | Q-3(4-F) | G-2(3-CN) | 211-213 |
| 3-193 | Q-3(4-F) | G-2(3-C(O)OCH₂CH₃) | 184-186 |
| 3-194 | Q-3(3-F) | G-3(3-F) | 218-220 |
| 3-195 | Q-3(3-F) | G-3(3-CH₃) | 197-199 |
| 3-196 | Q-3(3-F) | G-2(2-F,4-F) | 207-209 |
| 3-197 | Q-3(3-F) | G-3(3-Cl) | 201-203 |
| 3-198 | Q-9 | G-3(3-CH₃) | 226-228 |
| 3-199 | Q-3(5-F) | G-2(2-F,4-F) | 182-184 |
| 3-200 | Q-12b | G-3(3-F) | 151-153 |
| 3-201 | Q-12a(4-CH₃) | G-3(3-F) | 214-216 |
| 3-202 | Q-12a | G-2(4-F) | 189-191 |
| 3-203 | Q-12a | G-2(3-F) | 174-176 |
| 3-204 | Q-12a | G-1(4-F) | 169-171 |
| 3-205 | Q-12a | G-3(3-Cl) | 186-188 |
| 3-206 | Q-12a | G-3(3-CH₃) | 164-166 |
| 3-207 | Q-12a | G-2(3-F,4-F) | 181-183 |
| 3-208 | Q-12a | G-6a(2-Cl,5-Cl) | *1 |
| 3-209 | Q-1(4-CN) | G-3(3-Cl) | 182-184 |
| 3-210 | Q-1(4-CN) | G-1(4-F) | 235-236 |
| 3-211 | Q-2(4-Cl) | G-3 | 222-224 |
| 3-212 | Q-3(4-F) | G-20 | 218-220 |
| 3-213 | Q-2(4-F) | G-3(3-CF₃) | 189-191 |
| 3-214 | Q-2(4-Cl) | G-3(3-CF₃) | 179-181 |
| 3-215 | Q-12a | G-3(3-CF₃) | 211-213 |
| 3-216 | Q-2(4-F) | G-3(3-Cl) | 172-174 |
| 3-217 | Q-2(4-F) | G-3(3-CH₃) | 211-213 |
| 3-218 | Q-3(4-F) | G-16(3-Cl) | 239-241 |
| 3-219 | Q-3(3-CH₃) | G-3(3-Cl) | 201-203 |
| 3-220 | Q-3(3-CH₃) | G-3(3-CH₃) | 192-194 |
| 3-221 | Q-3(3-CH₃) | G-1(4-F) | 202-204 |
| 3-222 | Q-3(3-CF₃) | G-3(3-F) | 177-179 |
| 3-223 | Q-3 | G-3(3-CF₃) | 199-201 |
| 3-224 | Q-1(4-F) | G-3(3-CF₃) | 184-186 |
| 3-225 | Q-4(4-Cl) | G-3(3-CF₃) | 190-192 |
| 3-226 | Q-9 | G-3(3-CF₃) | 202-204 |
| 3-227 | Q-3(4-F) | G-28 | 211-213 |
| 3-228 | Q-2(4-Cl) | G-3(3-Cl) | 187-189 |
| 3-229 | Q-3(4-Br) | G-3 | 236-238 |
| 3-230 | Q-3(4-Br) | G-1 | 241-243 |
| 3-231 | Q-3(4-Br) | G-7 | 217-219 |
| 3-232 | Q-3(4-Br) | G-3(3-Cl) | 191-193 |
| 3-233 | Q-3(3-Br) | G-1(4-F) | 184-186 |
| 3-234 | Q-3(4-F) | G-9(4-Cl) | 209-211 |
| 3-235 | Q-3(4-F) | G-9(2-Cl) | 202-204 |
| 3-236 | Q-3(5-F) | G-3(3-Cl) | 199-201 |
| 3-237 | Q-3(3-F) | G-1(4-F) | 191-193 |
| 3-238 | Q-3(6-F) | G-3(3-F) | 162-164 |
| 3-239 | Q-3(6-F) | G-3(3-Cl) | 176-178 |
| 3-240 | Q-3(6-F) | G-3(3-CH₃) | 181-183 |
| 3-241 | Q-3(6-F) | G-1(4-F) | 196-198 |
| 3-242 | Q-12a(4-Br) | G-3(3-F) | 229-231 |
| 3-243 | Q-12a(4-CF₃) | G-3(3-F) | 206-208 |
| 3-244 | Q-3(4-F) | G-15(4-CF₃) | 215-217 |
| 3-245 | Q-9 | G-3(3-Cl) | 246-248 |
| 3-246 | Q-9 | G-3(3-F) | 255-257 |
| 3-247 | Q-9 | G-1(4-F) | 220-221 |
| 3-248 | Q-1(2-F,3-Cl) | G-3(3-F) | 241-243 |
| 3-249 | Q-3(3-OCH₃) | G-3(3-F) | 187-189 |
| 3-250 | Q-3(3-C(O)OCH₃) | G-3(3-F) | 201-203 |
| 3-251 | Q-12a(4-Br) | G-3(3-CH₃) | 212-214 |
| 3-252 | Q-12a(4-Br) | G-1(4-F) | 219-221 |
| 3-253 | Q-3(4-F) | G-40 | 177-179 |
| 3-254 | Q-3(4-F) | G-28(4-Cl,5-Cl) | 184-186 |
| 3-255 | Q-3(4-F) | G-11(3-Cl) | 184-186 |
| 3-256 | Q-3(4-F) | G-11(6-Cl) | 191-193 |
| 3-257 | Q-4 | G-1(4-F) | 162-164 |
| 3-258 | Q-4 | G-3(3-Cl) | 197-199 |
| 3-259 | Q-4 | G-3(3-CH₃) | 226-228 |
| 3-260 | Q-4 | G-3(3-CF₃) | 204-206 |
| 3-261 | Q-4 | G-7(4-F) | 201-203 |
| 3-262 | Q-4 | G-28(4-Cl,5-Cl) | 194-195 |
| 3-263 | Q-3 | G-28(4-Cl,5-Cl) | 211-213 |
| 3-264 | Q-3(4-F) | G-16(3-CF₃) | 206-208 |
| 3-265 | Q-3(4-F) | G-27 | 203-206 |
| 3-266 | Q-1(2-CH₃,4-CH₃) | G-3(3-F) | 217-219 |
| 3-267 | Q-1(3-Cl,4-CH₃) | G-3(3-F) | 203-205 |
| 3-268 | Q-4(4-Cl) | G-3(3-Cl) | 148-150 |
| 3-269 | Q-4(4-Cl) | G-1(4-F) | 187-189 |
| 3-270 | Q-3(4-Cl,6-F) | G-3(3-Cl) | 143-145 |
| 3-271 | Q-3(4-Cl,6-F) | G-1(4-F) | 170-172 |
| 3-272 | Q-1(3-F,4-F) | G-3(3-Cl) | 189-191 |
| 3-273 | Q-1(3-F,4-F) | G-3(3-CH₃) | 110-112 |
| 3-274 | Q-1(3-F,4-F) | G-1(4-F) | 122-124 |
| 3-275 | Q-12a(4-CF₃) | G-3(3-CH₃) | 119-121 |
| 3-276 | Q-12a(4-CF₃) | G-1(4-F) | 164-166 |
| 3-277 | Q-3(4-F) | G-3(3-SCH₃) | 201-203 |
| 3-278 | Q-3(4-F) | G-3(3-S(O)CH₃) | 179-181 |
| 3-279 | Q-3(4-F) | G-3(3-S(O)₂CH₃) | 209-211 |
| 3-280 | Q-4(4-CH₃) | G-3(3-F) | 149-151 |
| 3-281 | Q-3(3-F) | G-3(3-CF₃) | 130-132 |
| 3-282 | Q-3(5-F) | G-3(3-CF₃) | 136-139 |
| 3-283 | Q-3(3-CH₃) | G-3(3-CF₃) | 164-166 |
| 3-284 | Q-3(4-CH₃) | G-3(3-CF₃) | 151-153 |
| 3-285 | Q-3(6-F) | G-3(3-CF₃) | 61-63 |
| 3-286 | Q-3 | G-3(3-CH₃) | 161-163 |
| 3-287 | Q-9(3-CH₃) | G-3(3-F) | 144-146 |
| 3-288 | Q-9(3-CH₃) | G-3(3-Cl) | 140-142 |
| 3-289 | Q-9(3-CH₃) | G-3(3-CH₃) | 156-158 |
| 3-290 | Q-9(3-CH₃) | G-1(4-F) | 158-160 |
| 3-291 | Q-29(3-CH₂CH₃) | G-3(3-F) | 136-138 |
| 3-292 | Q-3(4-Cl,6-F) | G-3(3-CF₃) | 88-91 |
| 3-293 | Q-3(4-F) | G-18 | 198-200 |
| 3-294 | Q-10(3-CH₃) | G-3(3-F) | 204-206 |

**[Table 10]**

| 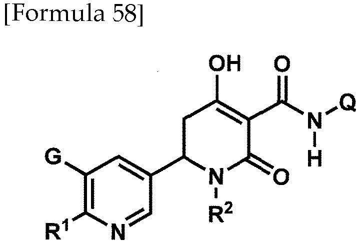 | | | | | |
|---|---|---|---|---|---|
| No. | R¹ | Q | G | R² | m.p.(°C) |
| 4-001 | Cl | Q-3(4-F) | G-1(4-F) | C(O)OCH₂CH₃ | 117-119 |

**[Table 11]**

| 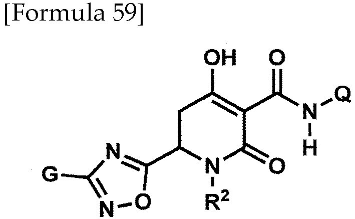 | | | | |
|---|---|---|---|---|
| No. | Q | G | R² | m.p.(°C) |
| 5-001 | Q-3(4-F) | G-3(3-F) | C(O)OCH₂CH₃ | *1 |
| 5-002 | Q-3(4-F) | G-3(3-F) | C(O)i-Bu | *1 |
| 5-003 | Q-3(4-F) | G-3(3-F) | C(O)c-Pr | 121-123 |
| 5-004 | Q-3(4-F) | G-3(3-F) | C(O)CH₂OCH₃ | *1 |
| 5-005 | Q-3(4-F) | G-3(3-F) | C(O)OCH₃ | *1 |
| 5-006 | Q-3(4-F) | G-3(3-F) | C(O)C(O)CH₂CH₃ | 137-139 |
| 5-007 | Q-3(4-F) | G-3(3-F) | C(O)OCH₂CH=CH₂ | 148-150 |
| 5-008 | Q-3(4-F) | G-3(3-F) | C(O)CH₃ | 190-192 |
| 5-009 | Q-3(4-F) | G-3(3-F) | C(O)CH₂CF₃ | 204-206 |
| 5-010 | Q-2(4-F) | G-3(3-F) | C(O)OCH₃ | 83-85 |
| 5-011 | Q-2(4-F) | G-3(3-F) | C(O)CH₂OCH₃ | 86-88 |
| 5-012 | Q-2(4-F) | G-3(3-F) | C(O)CH₃ | *1 |
| 5-013 | Q-3(4-F) | G-3(3-F) | D-1(2-CH₃) | 188-191 |
| 5-014 | Q-3(4-F) | G-3(3-F) | D-2 | *1 |
| 5-015 | Q-3(4-F) | G-3(3-F) | D-3 | *1 |
| 5-016 | Q-2(4-F) | G-3(3-F) | D-3 | 113-115 |

**[Table 12]**

| 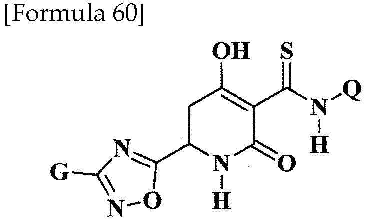 | | | |
|---|---|---|---|
| No. | Q | G | m.p.(°C) |
| 6-001 | Q-2(4-Cl) | G-3(3-F) | 196-198 |
| 6-002 | Q-3(4-F) | G-3(3-F) | 200-202 |
| 6-003 | Q-3(4-F) | G-2(4-F) | 181-182 |
| 6-004 | Q-3(4-F) | G-2(2-F,4-F) | 169-172 |
| 6-005 | Q-3(4-F) | G-3(3-CH₃) | 191-192 |

**[Table 13]**

| 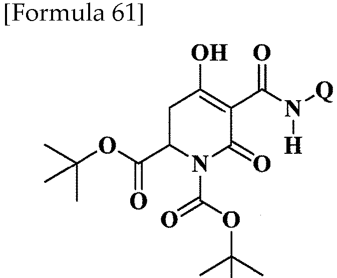 | | |
|---|---|---|
| No. | Q | m.p.(°C) |
| i1-001 | Q-1 | |
| i1-002 | Q-1(4-F) | 151-153 |
| i1-003 | Q-1(4-Cl) | |
| i1-004 | Q-1(4-Br) | |
| i1-005 | Q-1(4-I) | |
| i1-006 | Q-1(4-CN) | 161-163 |
| i1-007 | Q-1(4-CH₃) | |
| i1-008 | Q-1(4-CF₃) | |
| i1-009 | Q-2 | |
| i1-010 | Q-2(4-F) | 144-146 |
| i1-011 | Q-2(4-Cl) | 172-174 |
| i1-012 | Q-2(4-Br) | |
| i1-013 | Q-2(4-I) | |
| i1-014 | Q-2(4-CN) | |
| i1-015 | Q-2(4-CH₃) | |
| i1-016 | Q-2(4-CF₃) | |
| i1-017 | Q-3 | 154-156 |
| i1-018 | Q-3(4-F) | 152-154 |
| i1-019 | Q-3(4-Cl) | 182-184 |
| i1-020 | Q-3(4-Br) | 186-188 |
| i1-021 | Q-3(4-I) | |
| i1-022 | Q-3(4-CN) | |
| i1-023 | Q-3(4-CH₃) | 167-169 |
| i1-024 | Q-3(4-CF₃) | |
| i1-025 | Q-4 | 146-148 |
| i1-026 | Q-4(4-F) | |
| i1-027 | Q-4(4-Cl) | 174-176 |
| i1-028 | Q-4(4-Br) | 164-166 |
| i1-029 | Q-4(4-I) | |
| i1-030 | Q-4(4-CN) | |
| i1-031 | Q-4(4-CH₃) | |
| i1-032 | Q-4(4-CF₃) | |
| i1-033 | Q-6 | |
| i1-034 | Q-6(4-F) | |
| i1-035 | Q-6(4-Cl) | 154-156 |
| i1-036 | Q-6(4-Br) | |
| i1-037 | Q-6(4-I) | |
| i1-038 | Q-6(4-CN) | |
| i1-039 | Q-6(4-CH₃) | |
| i1-040 | Q-6(4-CF₃) | |
| i1-041 | Q-9 | 182-184 |
| i1-042 | Q-9(4-F) | |
| i1-043 | Q-9(4-Cl) | 186-188 |
| i1-044 | Q-9(4-Br) | 166-168 |
| i1-045 | Q-9(4-I) | |
| i1-046 | Q-9(4-CN) | |
| i1-047 | Q-9(4-CH₃) | |
| i1-048 | Q-9(4-CF₃) | |

**[Table 14]**

| 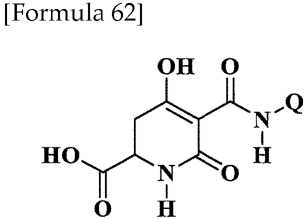 | | |
|---|---|---|
| No. | Q | m.p.(°C) |
| i2-002 | Q-1(4-F) | 205-207 |
| i2-006 | Q-1(4-CN) | 246-248 |
| i2-010 | Q-2(4-F) | 186-188 |
| i2-011 | Q-2(4-Cl) | 243-245 |
| i2-017 | Q-3 | 171-173 |
| i2-018 | Q-3(4-F) | 211-213 |
| i2-019 | Q-3(4-Cl) | 199-201 |
| i2-020 | Q-3(4-Br) | 191-193 |
| i2-023 | Q-3(4-CH₃) | 174-176 |
| i2-025 | Q-4 | 224-226 |
| i2-027 | Q-4(4-Cl) | 222-224 |
| i2-041 | Q-9 | 206-208 |

**[Table 15]**

| 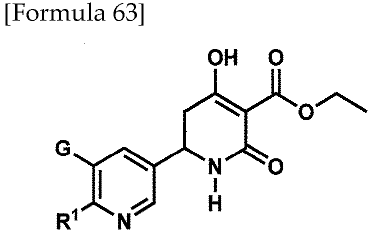 | | | |
|---|---|---|---|
| No. | R1 | G | m.p.(°C) |
| i5-003 | Cl | G-3 | 181-183 |
| i5-004 | Cl | G-2 | 162-164 |
| i5-010 | Cl | G-1(4-F) | 178-180 |
| i5-014 | Cl | G-3(3-F) | 184-186 |
| i5-017 | Cl | G-2(4-F) | 159-161 |
| i5-029 | Cl | G-3(3-Cl) | 174-176 |
| i5-040 | Cl | G-1(4-CH₃) | 196-198 |
| i5-044 | Cl | G-3(3-CH₃) | 182-184 |
| i5-078 | CF₃ | G-3(3-F) | 159-161 |

Table 16 shows ¹H-NMR data for compounds as the presently invented compound for which melting point is not shown. Chemical shift values in proton nuclear magnetic resonance spectra (hereinafter, referred to as ¹H-NMR) shown below were determined with use of Me₄Si (tetramethylsilane) as a standard substance at 300 MHz (model: JNM-ECX300 or JNM-ECP300, manufactured by JEOL Ltd.) or 400 MHz (model: JNM-ECZ400S, manufactured by JEOL Ltd.). Signs for chemical shift values in ¹H-NMR have meanings shown below, and the notation "*2" indicates that any signal corresponding to a proton of OH or NH was not observed.
s: singlet, d: doublet, dd: double doublet, t: triplet, q: quartet, m: multiplet, brs: broad singlet

**[Table 16]**

| No. | ¹H-NMR (Me₄Si) |
|---|---|
| 2-286 (400MHz, DMSO-d₆, *2):δ12.4(brs, 1H), 8.50(brs, 1H), 8.40(brs, 1H), 8.25-8.15(m, 1H), 8.10-8.00(m, 1H), 7.92(brs, 1H), 7.40-7.30(m, 2H), 7.20-7.10(m, 1H), 5.15-4.90(m, 2H), 2.60-3.1(m, 1H). | |
| 2-287 (400MHz, DMSO-d₆):δ12.1 and 11.9(brs, 1H), 8.7-8.8(m, 2H), 8.5-8.6(brs, 1H), 8.4-8.5(m, 1H), 8.3-8.4(m, 1H), 8.2-8.3(m, 1H), 8.05-8.15(m, 1H), 7.95-8.05(m, 1H), 7.35-7.45(m, 2H), 4.9-5.1(m, 1H), 2.8-3.2(m, 2H). | |
| 2-288 (400MHz, DMSO-d₆):612.4(brs, 1H), 8.9-9.0(m, 1H), 8.7-8.9(brs, 1H), 8.5-8.6(m, 1H), 8.4(brs, 2H), 8.15-8.3(m, 1H), 8.05-8.15(m, 1H), 7.5-7.7(m, 2H), 7.3-7.4(m, 1H), 4.85-4.95(m, 1H), 2.8-3.0(m, 2H). | |
| 2-289 (400MHz, DMSO-d₆, *2):δ9.68(brs, 1H), 9.02(brs, 1H), 8.5-8.6(m, 2H), 8.4-8.5(m, 1H), 8.2-8.35(m, 2H), 8.1(brs, 1H), 7.7-7.8(m, 1H), 7.35-7.45(m, 1H), 5.05(brs, 1H), 2.8-3.2(m, 2H). | |
| 3-165 (400MHz, CDCl₃):δ12.1(brs, 1H), 8.77(brs, 1H), 8.47(d, J=4.8Hz, 1H), 8.17(brs, 1H), 7.8-7.95(m, 1H), 7.6-7.7(m, 2H), 7.0-7.15(m, 1H), 5.25-5.55(m, 1H), 3.0-3.1(m, 2H), 2.07(s, 3H). | |
| 3-166 (400MHz, CDCl₃):δ10.9(brs, 1H), 8.35-8.5(m, 2H), 7.8-7.95(m, 2H), 7.6-7.7(m, 2H), 6.9-7.1(m, 1H), 5.1-5.2(m, 1H), 3.0-3.3(m, 2H). | |
| 3-208 (300MHz, DMSO-d₆, *2): 11.90-12.06(m, 1H), 9.63-8.68(m, 1H), 7.67(brs, 1H), 6.39-6.52(m, 1H), 5.23-5.55(m, 1H), 3.91(s, 3H), 3.80(s, 3H), 3.56(dd, J=17.7, 7.5Hz, 1H), 2.86-3.15(m, 1H). | |
| 5-001 (400MHz, CDCl₃, *2):δ11.7(brs, 1H), 8.37(d, J=5.2Hz, 1H), 8.23(d, J=3.2Hz, 1H), 8.04(dd, J=13.2, 4.0Hz, 1H), 7.8-7.85(m, 1H), 7.57(brs, 1H), 7.4-7.5(m, 1H), 6.0-6.1(m, 1H), 4.46(q, J=7.2Hz, 2H) , 3.45-3.55(m, 1H), 3.25-3.35(m, 1H), 1.43(t, J=7.2Hz, 3H). | |
| 5-002 (400MHz, CDCl₃, *2):δ11.7(brs, 1H), 8.36(d, J=5.2Hz, 1H), 8.25(d, J=3.2Hz, 1H), 8.08(dd, J=9.2, 4.0Hz, 1H), 7.75-7.85(m, 1H), 7.55(brs, 1H), 7.4-7.5(m, 1H), 6.3-6.4(m, 1H), 3.39(d, J=4.4Hz, 2H), 3.1-3.2(m, 1H), 2.8-2.9(m, 1H), 2.15-2.25(m, 1H), 1.06(d, J=6.8Hz, 3H), 1.0(d, J=6.8Hz, 3H). | |
| 5-004 (400MHz, CDCl₃, *2):δ11.5(brs, 1H), 8.37(d, J=5.2Hz, 1H), 8.25(d, J=2.8Hz, 1H), 8.07(dd, J=9.2, 4.0Hz, 1H), 7.75-7.85(m, 1H), 7.55(brs, 1H), 7.4-7.5(m, 1H), 6.4-6.45(m, 1H), 4.92(d, J=18Hz, 1H) , 4.66(d, J=18Hz, 1H), 3.54(s, 3H), 3.43(d, J=4.4Hz, 2H). | |
| 5-005 (400MHz, CDCl₃, *2):δ11.7(brs, 1H), 8.37(d, J=5.2Hz, 1H), 8.21(d, J=2.4Hz, 1H), 8.0-8.1(m, 1H), 7.8-7.85(m, 1H), 7.55-7.65(m, 1H), 7.4-7.5(m, 1H), 6.06(dd, J=6.4, 1.6Hz, 1H), 4.02(s, 3H), 3.4-3.55(m 1H), 3.3-3.4(m, 1H). | |
| 5-012 (400MHz, CDCl₃, *2):δ11.4(brs, 1H), 8.35-8.45(m, 2H), 8.05-8.15(m, 1H), 7.8-7.85(m, 1H), 7.55-7.6(m, 1H), 6.9-7.0(m, 1H), 6.3-6.4(m, 1H), 3.41(d, J=4.0Hz, 2H), 2.73(s, 3H). | |
| 5-014 (400MHz, CDCl₃, *2):δ11.5(brs, 1H), 8.37(d, J=5.2Hz, 1H), 8.25(d, J=2.8Hz, 1H), 8.07(dd, J=9.2, 4.0Hz, 1H), 7.75-7.85(m, 1H), 7.55(brs, 1H), 7.4-7.5(m, 1H), 6.4-6.45(m, 1H), 3.65-3.75(m, 4H), 3.25-3.3(m, 4H), 2.8-2.9(m, 1H), 2.0-2.2(m, 1H). | |
| 5-015 (400MHz, CDCl₃, *2):δ11.6(brs, 1H), 8.36(d, J=5.2Hz, 1H), 8.21(d, J=3.2Hz, 1H), 8.06(dd, J=9.2, 4.0Hz, 1H), 7.75-7.85(m, 1H), 7.55-7.65(m, 2H), 7.4-7.5(m, 1H), 7.3-7.35(m, 1H), 6.55-6.6(m, 1H) , 5.8-5.85(m 1H), 3.61(dd, J=18.0, 7.2Hz, 1H) , 3.3-3.45(m, 1H). | |

### [Test Examples]

Next, the usefulness of the presently invented compound as a pesticide will be specifically described in Test Examples shown in the following, but the present invention is not limited thereto.

### Test Example 1: Insecticidal test for brown rice planthoppers

A 10% by mass emulsion of the presently invented compound prepared according to the above formulation example for an emulsion was diluted with water containing a spreader to prepare a chemical solution having a concentration of 500 ppm. Rice leaf sheaths were soaked in the chemical solution for approximately 10 seconds. After the completion of the soaking operation, the rice leaf sheaths treated with the chemical solution were air-dried, and then put in test tubes. Into each test tube, five third-instar brown rice planthoppers (*Nilaparvata lugens*) larvae were released, and the test tubes were capped with sponge and accommodated in a thermostatic room at 25°C. Six days after the accommodation, the number of dead brown rice planthoppers individuals in the test tubes were examined, and the death rate was calculated from a calculation formula shown below. The test was carried out in duplicate. Death rate (%) = (number of dead individuals / number of tested individuals) × 100

The result showed that the following compounds exhibited death rates of 90% or more.
The presently invented compound: 1-001, 1-002, 1-003, 1-013, 1-014, 1-020, 2-001, 2-005, 2-008, 2-009, 2-010, 2-011, 2-012, 2-013, 2-014, 2-016, 2-018, 2-019, 2-020, 2-021, 2-022, 2-023, 2-024, 2-025, 2-027, 2-028, 2-029, 2-030, 2-031, 2-032, 2-033, 2-034, 2-035, 2-036, 2-037, 2-038, 2-039, 2-040, 2-041, 2-042, 2-043, 2-044, 2-045, 2-047, 2-048, 2-049, 2-050, 2-051, 2-052, 2-053, 2-054, 2-055, 2-056, 2-057, 2-058, 2-059, 2-060, 2-061, 2-062, 2-063, 2-067, 2-069, 2-071, 2-074, 2-076, 2-077, 2-079, 2-080, 2-081, 2-082, 2-083, 2-084, 2-085, 2-086, 2-087, 2-088, 2-089, 2-090, 2-091, 2-092, 2-093, 2-094, 2-095, 2-096, 2-097, 2-098, 2-099, 2-100, 2-101, 2-102, 2-103, 2-104, 2-105, 2-106, 2-107, 2-108, 2-109, 2-110, 2-111, 2-112, 2-113, 2-114, 2-115, 2-116, 2-117, 2-118, 2-119, 2-120, 2-122, 2-123, 2-124, 2-125, 2-126, 2-127, 2-128, 2-129, 2-130, 2-132, 2-133, 2-134, 2-135, 2-136, 2-137, 2-138, 2-139, 2-140, 2-141, 2-142, 2-143, 2-144, 2-145, 2-146, 2-147, 2-150, 2-151, 2-152, 2-153, 2-154, 2-155, 2-156, 2-157, 2-158, 2-159, 2-160, 2-161, 2-162, 2-163, 2-164, 2-165, 2-166, 2-167, 2-168, 2-169, 2-170, 2-171, 2-172, 2-173, 2-174, 2-175, 2-176, 2-177, 2-178, 2-179, 2-180, 2-181, 2-182, 2-183, 2-184, 2-186, 2-187, 2-188, 2-189, 2-190, 2-191, 2-192, 2-193, 2-194, 2-195, 2-196, 2-197, 2-198, 2-199, 2-200, 2-201, 2-202, 2-203, 2-204, 2-205, 2-206, 2-207, 2-208, 2-209, 2-211, 2-212, 2-213, 2-214, 2-215, 2-216, 2-217, 2-218, 2-219, 2-222, 2-223, 2-227, 2-228, 2-231, 2-232, 2-233, 2-235, 2-238, 2-240, 2-244, 2-245, 2-246, 2-247, 2-248, 2-249, 2-250, 2-251, 2-252, 2-253, 2-254, 2-255, 2-256, 2-259, 2-260, 2-261, 2-262, 2-263, 2-264, 2-265, 2-267, 2-268, 2-269, 2-271, 2-272, 2-273, 2-276, 2-277, 2-287, 2-288, 2-289, 2-290, 2-297, 2-298, 2-299, 2-300, 2-301, 2-302, 2-303, 2-304, 2-305, 3-001, 3-002, 3-005, 3-006, 3-007, 3-009, 3-010, 3-011, 3-012, 3-013, 3-014, 3-015, 3-016, 3-017, 3-018, 3-020, 3-021, 3-022, 3-023, 3-024, 3-025, 3-026, 3-027, 3-028, 3-029, 3-030, 3-031, 3-032, 3-033, 3-034, 3-035, 3-036, 3-037, 3-038, 3-039, 3-040, 3-041, 3-042, 3-043, 3-044, 3-045, 3-046, 3-047, 3-048, 3-049, 3-050, 3-051, 3-052, 3-053, 3-055, 3-056, 3-057, 3-058, 3-059, 3-060, 3-061, 3-062, 3-063, 3-064, 3-065, 3-066, 3-067, 3-068, 3-069, 3-070, 3-071, 3-072, 3-073, 3-074, 3-075, 3-076, 3-077, 3-078, 3-079, 3-080, 3-081, 3-082, 3-083, 3-084, 3-085, 3-086, 3-087, 3-088, 3-089, 3-090, 3-091, 3-092, 3-093, 3-094, 3-095, 3-096, 3-097, 3-098, 3-100, 3-102, 3-103, 3-104, 3-105, 3-106, 3-107, 3-108, 3-110, 3-111, 3-112, 3-113, 3-114, 3-115, 3-116, 3-119, 3-121, 3-122, 3-123, 3-126, 3-127, 3-129, 3-130, 3-131, 3-132, 3-133, 3-134, 3-135, 3-136, 3-138, 3-139, 3-140, 3-141, 3-144, 3-146, 3-147, 3-152, 3-153, 3-154, 3-155, 3-156, 3-157, 3-158, 3-159, 3-160, 3-161, 3-162, 3-163, 3-164, 3-165, 3-166, 3-168, 3-169, 3-170, 3-171, 3-172, 3-173, 3-174, 3-175, 3-176, 3-177, 3-178, 3-179, 3-180, 3-182, 3-183, 3-184, 3-185, 3-186, 3-187, 3-188, 3-189, 3-190, 3-191, 3-192, 3-193, 3-194, 3-195, 3-196, 3-197, 3-198, 3-199, 3-200, 3-201, 3-202, 3-203, 3-204, 3-205, 3-206, 3-207, 3-208, 3-209, 3-210, 3-213, 3-214, 3-215, 3-216, 3-217, 3-219, 3-220, 3-221, 3-223, 3-224, 3-225, 3-226, 3-227, 3-228, 3-232, 3-233, 3-234, 3-235, 3-236, 3-237, 3-238, 3-239, 3-240, 3-241, 3-242, 3-243, 3-244, 3-245, 3-246, 3-249, 3-250, 3-251, 3-252, 3-253, 3-254, 3-255, 3-256, 3-257, 3-258, 3-259, 3-260, 3-261, 3-262, 3-263, 3-264, 3-265, 3-268, 3-269, 3-270, 3-271, 3-272, 3-273, 3-274, 3-275, 3-276, 3-277, 3-278, 3-279, 3-280, 3-281, 3-283, 3-284, 3-285, 3-286, 3-287, 3-288, 3-289, 3-290, 3-291, 3-292, 3-293, 3-294, 4-001, 5-001, 5-002, 5-003, 5-004, 5-005, 5-006, 5-007, 5-008, 5-009, 5-010, 5-011, 5-012, 5-013, 5-014, 5-015, 5-016, 6-001, 6-002, 6-003, 6-004, 6-005

### Test Example 2: Insecticidal test for green peach aphids

Wet absorbent cotton was laid on each glass dish having an inner diameter of 3 cm, a Chinese olive leaf cut into a circle having a diameter of 3 cm was placed thereon, and four wingless green peach aphids (*Myzus persicae*) imagoes were released. After a day, a 10% by mass emulsion of the presently invented compound prepared according to the above formulation example for an emulsion was diluted with water containing a spreader to prepare a chemical solution having a concentration of 500 ppm. The chemical solution prepared was sprinkled (2.5 mg/cm²) with a rotary tower sprinkler, and the dishes were capped and accommodated in a thermostatic room at 25°C. Six days after the accommodation, the number of dead green peach aphids individuals in the dishes were examined, and the death rate was calculated from the same calculation formula as in Test Example 1. The test was carried out in duplicate.

The result showed that the following compounds exhibited death rates of 90% or more.
The presently invented compound: 1-001, 1-004, 1-005, 1-006, 1-007, 1-008, 1-009, 1-010, 1-011, 1-012, 1-014, 1-015, 1-016, 1-017, 1-019, 1-020, 1-021, 1-023, 2-003, 2-005, 2-007, 2-010, 2-023, 2-032, 2-034, 2-035, 2-036, 2-040, 2-041, 2-042, 2-044, 2-045, 2-046, 2-047, 2-048, 2-049, 2-053, 2-054, 2-056, 2-057, 2-058, 2-062, 2-063, 2-064, 2-067, 2-068, 2-077, 2-083, 2-086, 2-087, 2-091, 2-097, 2-098, 2-099, 2-100, 2-104, 2-112, 2-115, 2-117, 2-120, 2-124, 2-125, 2-126, 2-137, 2-141, 2-146, 2-151, 2-155, 2-156, 2-157, 2-158, 2-159, 2-160, 2-161, 2-162, 2-163, 2-165, 2-167, 2-169, 2-170, 2-172, 2-173, 2-179, 2-180, 2-182, 2-183, 2-184, 2-186, 2-189, 2-192, 2-194, 2-196, 2-197, 2-198, 2-199, 2-200, 2-201, 2-202, 2-203, 2-208, 2-213, 2-214, 2-216, 2-217, 2-219, 2-242, 2-244, 2-245, 2-247, 2-250, 2-251, 2-253, 2-254, 2-255, 2-257, 2-264, 2-265, 2-266, 2-267, 2-268, 2-271, 2-273, 2-274, 2-278, 2-280, 2-281, 2-284, 2-285, 2-292, 2-298, 2-303, 3-002, 3-009, 3-011, 3-013, 3-014, 3-015, 3-017, 3-020, 3-022, 3-024, 3-027, 3-028, 3-033, 3-039, 3-042, 3-044, 3-049, 3-051, 3-053, 3-054, 3-055, 3-056, 3-058, 3-059, 3-060, 3-064, 3-070, 3-072, 3-074, 3-075, 3-080, 3-095, 3-096, 3-097, 3-098, 3-099, 3-100, 3-107, 3-109, 3-110, 3-111, 3-112, 3-117, 3-118, 3-119, 3-122, 3-123, 3-125, 3-126, 3-128, 3-129, 3-131, 3-132, 3-133, 3-134, 3-136, 3-137, 3-139, 3-142, 3-144, 3-148, 3-164, 3-166, 3-167, 3-170, 3-173, 3-174, 3-178, 3-179, 3-181, 3-183, 3-192, 3-194, 3-196, 3-200, 3-202, 3-204, 3-205, 3-206, 3-207, 3-208, 3-210, 3-213, 3-215, 3-217, 3-219, 3-222, 3-224, 3-225, 3-226, 3-228, 3-229, 3-230, 3-232, 3-236, 3-241, 3-246, 3-248, 3-249, 3-251, 3-252, 3-253, 3-254, 3-257, 3-265, 3-266, 3-268, 3-269, 3-270, 3-271, 3-272, 3-273, 3-274, 3-275, 3-276, 3-277, 3-278, 3-279, 3-280, 3-281, 3-291, 4-001, 5-002, 5-007, 5-009, 5-011, 5-012, 5-013, 5-015, 5-016, 6-001, 6-004

### Test Example 3: Insecticidal activity against western flower thrips

Wet filter paper was laid on each styrol cup having an inner diameter of 7 cm, and a common bean leaf cut into a 3 cm square was placed thereon, and inoculated with 20 western flower thrips (*Frankliniella occidentalis*) larvae. A 10% by mass emulsion of the presently invented compound prepared according to the above formulation example for an emulsion was diluted with water containing a spreader to prepare a chemical solution having a concentration of 500 ppm. For each styrol cup, 2.5 ml of the chemical solution prepared was sprinkled (2.5 mg/cm²) with a rotary tower sprinkler. After 2 days, the number of dead western flower thrips individuals in the styrol cups were examined, and the death rate was calculated from the same calculation formula as in Test Example 1. The test was carried out in duplicate.

The result showed that the following compounds exhibited death rates of 90% or more.
The presently invented compound: 1-001, 1-002, 1-003, 1-005, 1-007, 1-008, 1-009, 1-010, 1-011, 1-013, 1-014, 1-015, 1-016, 1-017, 1-019, 1-020, 1-022, 1-023, 1-024, 2-001, 2-005, 2-008, 2-009, 2-010, 2-011, 2-012, 2-013, 2-014, 2-016, 2-018, 2-019, 2-020, 2-021, 2-022, 2-024, 2-025, 2-027, 2-028, 2-029, 2-030, 2-031, 2-032, 2-033, 2-034, 2-035, 2-036, 2-037, 2-038, 2-039, 2-040, 2-041, 2-042, 2-044, 2-045, 2-047, 2-048, 2-049, 2-050, 2-053, 2-054, 2-055, 2-056, 2-057, 2-058, 2-059, 2-062, 2-063, 2-067, 2-068, 2-069, 2-071, 2-072, 2-073, 2-074, 2-076, 2-077, 2-078, 2-079, 2-080, 2-081, 2-082, 2-083, 2-084, 2-085, 2-086, 2-087, 2-088, 2-089, 2-090, 2-091, 2-092, 2-093, 2-094, 2-095, 2-096, 2-099, 2-100, 2-101, 2-102, 2-103, 2-104, 2-105, 2-106, 2-107, 2-108, 2-109, 2-110, 2-111, 2-112, 2-113, 2-114, 2-115, 2-116, 2-117, 2-118, 2-119, 2-120, 2-121, 2-122, 2-123, 2-124, 2-125, 2-126, 2-127, 2-128, 2-129, 2-130, 2-131, 2-132, 2-133, 2-134, 2-135, 2-136, 2-137, 2-138, 2-139, 2-140, 2-141, 2-142, 2-143, 2-144, 2-145, 2-146, 2-147, 2-150, 2-151, 2-152, 2-153, 2-154, 2-155, 2-156, 2-157, 2-158, 2-159, 2-160, 2-161, 2-162, 2-163, 2-164, 2-165, 2-166, 2-167, 2-168, 2-169, 2-170, 2-171, 2-172, 2-173, 2-174, 2-175, 2-176, 2-177, 2-178, 2-179, 2-180, 2-181, 2-182, 2-183, 2-184, 2-186, 2-187, 2-188, 2-189, 2-190, 2-191, 2-192, 2-193, 2-194, 2-195, 2-196, 2-198, 2-199, 2-200, 2-201, 2-202, 2-203, 2-204, 2-206, 2-207, 2-208, 2-209, 2-211, 2-212, 2-213, 2-214, 2-215, 2-216, 2-218, 2-219, 2-220, 2-221, 2-222, 2-223, 2-224, 2-225, 2-226, 2-227, 2-228, 2-229, 2-230, 2-231, 2-232, 2-233, 2-235, 2-238, 2-239, 2-240, 2-244, 2-245, 2-246, 2-247, 2-248, 2-249, 2-250, 2-251, 2-252, 2-253, 2-254, 2-255, 2-256, 2-257, 2-258, 2-259, 2-260, 2-261, 2-262, 2-263, 2-265, 2-269, 2-271, 2-272, 2-273, 2-276, 2-277, 2-279, 2-280, 2-281, 2-282, 2-287, 2-288, 2-296, 2-297, 2-298, 2-299, 2-300, 2-301, 2-302, 2-303, 2-304, 2-305, 3-001, 3-003, 3-005, 3-006, 3-007, 3-009, 3-010, 3-011, 3-012, 3-013, 3-014, 3-015, 3-016, 3-018, 3-020, 3-021, 3-022, 3-023, 3-024, 3-025, 3-026, 3-027, 3-028, 3-029, 3-030, 3-031, 3-032, 3-034, 3-035, 3-036, 3-037, 3-038, 3-039, 3-040, 3-041, 3-042, 3-043, 3-044, 3-045, 3-046, 3-047, 3-048, 3-049, 3-050, 3-051, 3-052, 3-054, 3-055, 3-056, 3-057, 3-058, 3-059, 3-060, 3-061, 3-062, 3-063, 3-064, 3-065, 3-066, 3-067, 3-068, 3-069, 3-070, 3-071, 3-072, 3-073, 3-074, 3-075, 3-076, 3-077, 3-078, 3-079, 3-080, 3-081, 3-082, 3-083, 3-084, 3-085, 3-086, 3-088, 3-089, 3-090, 3-091, 3-092, 3-095, 3-096, 3-097, 3-098, 3-100, 3-102, 3-103, 3-104, 3-105, 3-106, 3-107, 3-108, 3-109, 3-111, 3-113, 3-114, 3-115, 3-116, 3-120, 3-121, 3-122, 3-123, 3-124, 3-126, 3-127, 3-128, 3-129, 3-131, 3-132, 3-133, 3-134, 3-135, 3-138, 3-139, 3-140, 3-141, 3-144, 3-145, 3-146, 3-147, 3-154, 3-155, 3-156, 3-157, 3-159, 3-160, 3-161, 3-163, 3-164, 3-165, 3-169, 3-170, 3-171, 3-172, 3-173, 3-174, 3-175, 3-176, 3-177, 3-179, 3-180, 3-183, 3-184, 3-185, 3-186, 3-187, 3-188, 3-189, 3-190, 3-191, 3-192, 3-195, 3-197, 3-199, 3-200, 3-202, 3-204, 3-205, 3-206, 3-207, 3-208, 3-209, 3-210, 3-213, 3-214, 3-215, 3-216, 3-217, 3-219, 3-220, 3-221, 3-223, 3-224, 3-225, 3-226, 3-227, 3-228, 3-232, 3-233, 3-234, 3-235, 3-236, 3-238, 3-239, 3-240, 3-241, 3-242, 3-243, 3-244, 3-245, 3-246, 3-249, 3-250, 3-252, 3-253, 3-254, 3-255, 3-256, 3-257, 3-258, 3-259, 3-260, 3-261, 3-262, 3-263, 3-264, 3-265, 3-267, 3-268, 3-269, 3-270, 3-271, 3-272, 3-273, 3-274, 3-275, 3-277, 3-278, 3-279, 3-280, 3-281, 3-282, 3-283, 3-284, 3-285, 3-286, 3-287, 3-288, 3-289, 3-292, 3-294, 4-001, 5-001, 5-002, 5-003, 5-004, 5-005, 5-006, 5-007, 5-008, 5-009, 5-010, 5-011, 5-012, 5-013, 5-014, 5-015, 5-016, 6-001, 6-002, 6-003, 6-004

### Test Example 4: Insecticidal test for diamondback moths

A 10% by mass emulsion of the presently invented compound prepared according to the above formulation example for an emulsion was diluted with water containing a spreader to prepare a chemical solution having a concentration of 500 ppm. Chinese olive leaves were soaked in the chemical solution for approximately 10 seconds. After the completion of the soaking operation, the Chinese olive leaves treated with the chemical solution were air-dried, and then put in dishes. Into each dish, five third-instar diamondback moths (*Plutella xylostella*) larvae were released, and the dishes were capped and accommodated in a thermostatic room at 25°C. Six days after the accommodation, the number of dead diamondback moths individuals in the dishes were examined, and the death rate was calculated from the same calculation formula as in Test Example 1. The test was carried out in duplicate.

The result showed that the following compounds exhibited death rates of 90% or more.
The presently invented compound: 1-001, 1-002, 1-003, 1-004, 1-005, 1-006, 1-007, 1-008, 1-009, 1-010, 1-011, 1-013, 1-014, 1-015, 1-016, 1-017, 1-018, 1-019, 1-020, 1-021, 1-022, 1-023, 1-024, 2-001, 2-005, 2-008, 2-009, 2-010, 2-011, 2-012, 2-013, 2-014, 2-016, 2-017, 2-018, 2-019, 2-020, 2-021, 2-022, 2-024, 2-025, 2-027, 2-028, 2-029, 2-030, 2-031, 2-032, 2-033, 2-034, 2-035, 2-036, 2-038, 2-039, 2-040, 2-041, 2-042, 2-044, 2-045, 2-048, 2-049, 2-050, 2-055, 2-057, 2-058, 2-060, 2-061, 2-062, 2-063, 2-064, 2-066, 2-067, 2-068, 2-069, 2-072, 2-073, 2-076, 2-077, 2-078, 2-079, 2-080, 2-081, 2-082, 2-083, 2-084, 2-085, 2-086, 2-087, 2-088, 2-089, 2-090, 2-091, 2-092, 2-093, 2-094, 2-095, 2-096, 2-097, 2-098, 2-099, 2-100, 2-101, 2-102, 2-103, 2-104, 2-105, 2-106, 2-107, 2-108, 2-109, 2-110, 2-111, 2-112, 2-113, 2-114, 2-115, 2-117, 2-118, 2-119, 2-121, 2-122, 2-123, 2-124, 2-125, 2-126, 2-127, 2-128, 2-129, 2-130, 2-131, 2-132, 2-133, 2-134, 2-135, 2-136, 2-137, 2-138, 2-139, 2-140, 2-141, 2-142, 2-143, 2-144, 2-145, 2-146, 2-147, 2-148, 2-150, 2-151, 2-152, 2-153, 2-154, 2-155, 2-156, 2-157, 2-158, 2-159, 2-160, 2-161, 2-162, 2-163, 2-164, 2-166, 2-167, 2-168, 2-169, 2-170, 2-171, 2-172, 2-174, 2-175, 2-176, 2-177, 2-178, 2-179, 2-181, 2-182, 2-183, 2-184, 2-186, 2-187, 2-188, 2-189, 2-190, 2-191, 2-192, 2-193, 2-194, 2-195, 2-196, 2-197, 2-198, 2-199, 2-200, 2-201, 2-202, 2-203, 2-204, 2-205, 2-206, 2-207, 2-208, 2-209, 2-211, 2-212, 2-213, 2-214, 2-215, 2-216, 2-217, 2-218, 2-219, 2-220, 2-221, 2-222, 2-223, 2-224, 2-225, 2-226, 2-227, 2-228, 2-229, 2-231, 2-232, 2-233, 2-234, 2-235, 2-237, 2-240, 2-241, 2-242, 2-243, 2-244, 2-245, 2-246, 2-247, 2-248, 2-249, 2-250, 2-251, 2-252, 2-253, 2-254, 2-255, 2-256, 2-257, 2-259, 2-260, 2-261, 2-262, 2-263, 2-264, 2-265, 2-267, 2-268, 2-269, 2-271, 2-272, 2-273, 2-274, 2-276, 2-277, 2-278, 2-279, 2-280, 2-282, 2-283, 2-287, 2-288, 2-295, 2-297, 2-298, 2-299, 2-300, 2-301, 2-302, 2-303, 2-304, 2-305, 3-001, 3-002, 3-003, 3-004, 3-005, 3-006, 3-007, 3-009, 3-010, 3-011, 3-012, 3-013, 3-014, 3-015, 3-016, 3-017, 3-018, 3-019, 3-020, 3-021, 3-022, 3-023, 3-024, 3-025, 3-026, 3-027, 3-028, 3-029, 3-030, 3-031, 3-032, 3-033, 3-034, 3-035, 3-036, 3-037, 3-038, 3-039, 3-040, 3-041, 3-042, 3-043, 3-044, 3-046, 3-048, 3-049, 3-050, 3-051, 3-052, 3-053, 3-054, 3-055, 3-056, 3-057, 3-058, 3-059, 3-060, 3-061, 3-062, 3-063, 3-064, 3-065, 3-066, 3-067, 3-068, 3-069, 3-070, 3-071, 3-072, 3-073, 3-074, 3-075, 3-076, 3-077, 3-078, 3-079, 3-080, 3-081, 3-082, 3-083, 3-084, 3-085, 3-086, 3-088, 3-089, 3-090, 3-092, 3-093, 3-095, 3-096, 3-097, 3-098, 3-099, 3-100, 3-102, 3-103, 3-104, 3-105, 3-106, 3-107, 3-108, 3-109, 3-110, 3-113, 3-114, 3-115, 3-116, 3-119, 3-122, 3-123, 3-124, 3-125, 3-126, 3-127, 3-128, 3-129, 3-131, 3-132, 3-133, 3-134, 3-135, 3-136, 3-138, 3-139, 3-140, 3-141, 3-142, 3-143, 3-144, 3-145, 3-146, 3-147, 3-154, 3-155, 3-156, 3-157, 3-158, 3-159, 3-160, 3-161, 3-162, 3-163, 3-164, 3-165, 3-166, 3-169, 3-170, 3-172, 3-173, 3-174, 3-175, 3-176, 3-177, 3-178, 3-179, 3-180, 3-183, 3-184, 3-185, 3-186, 3-187, 3-188, 3-189, 3-190, 3-191, 3-192, 3-193, 3-194, 3-195, 3-196, 3-197, 3-198, 3-199, 3-200, 3-202, 3-203, 3-204, 3-205, 3-207, 3-208, 3-209, 3-210, 3-212, 3-213, 3-214, 3-215, 3-216, 3-217, 3-219, 3-220, 3-221, 3-222, 3-223, 3-224, 3-225, 3-226, 3-227, 3-228, 3-230, 3-232, 3-233, 3-234, 3-235, 3-236, 3-237, 3-238, 3-239, 3-240, 3-241, 3-243, 3-244, 3-246, 3-248, 3-249, 3-251, 3-252, 3-253, 3-254, 3-255, 3-256, 3-257, 3-258, 3-259, 3-260, 3-262, 3-263, 3-264, 3-265, 3-268, 3-269, 3-270, 3-271, 3-272, 3-273, 3-274, 3-275, 3-276, 3-277, 3-279, 3-280, 3-281, 3-282, 3-283, 3-284, 3-285, 3-286, 3-292, 4-001, 5-001, 5-002, 5-003, 5-004, 5-005, 5-006, 5-007, 5-008, 5-009, 5-010, 5-011, 5-012, 5-013, 5-014, 5-015, 5-016, 6-001, 6-002, 6-003, 6-004, 6-005

### Test Example 5: Effect test for kennel ticks

In 1 ml of acetone, 1 mg of the presently invented compound was dissolved to prepare a chemical solution having a concentration of 1000 ppm. The bottom surface and side surface of each glass container having an inner wall surface area of 25 cm² were coated with 250 µl of the chemical solution, and acetone was then volatilized to form a thin film of the compound on the inner wall of the glass container. Each of the glass containers used had an inner wall of 25 cm², and the amount of the applied chemical was 10 µg/cm².

Into each of the treated glass containers, five kennel ticks (*Rhipicephalus sanguineus*) protonymphs (mixed-sex) were released, and the glass containers were capped and accommodated in a thermostatic room at 25°C. Two days after the release, the number of dead tick individuals were examined, and the death rate was calculated from the same calculation formula as in Test Example 1.

The result showed that the following compounds exhibited death rates of 50% or more.
The presently invented compound: 1-006, 1-007, 2-120, 2-127, 2-128, 2-151, 2-153, 3-082, 3-100, 3-101, 3-114, 3-128, 3-129, 3-166, 3-186, 3-200, 3-208

### Test Example 6: Effect test for cat fleas

In 1 ml of acetone, 1 mg of the presently invented compound was dissolved to prepare a chemical solution having a concentration of 1000 ppm. The bottom surface and side surface of each glass container having an inner wall surface area of 25 cm² were coated with 250 µl of the chemical solution, and acetone was then volatilized to form a thin film of the compound on the inner wall of the glass container. Each of the glass containers used had an inner wall of 35 cm², and the amount of the applied chemical was 10 µg/cm².

Into each of the treated glass containers, five cat fleas (*Ctenocephalides felis*) imagoes (mixed-sex) were released, and the glass containers were capped and accommodated in a thermostatic room at 25°C. Three days after the release, the number of dead flea individuals were examined, and the death rate was calculated from the same calculation formula as in Test Example 1.

The result showed that the following compounds exhibited death rates of 50% or more.
The presently invented compound: 1-001, 1-002, 1-003, 1-004, 1-005, 1-006, 1-007, 1-008, 1-009, 1-011, 1-012, 1-014, 1-015, 1-016, 1-017, 1-018, 1-019, 1-020, 1-021, 1-023, 1-024, 2-001, 2-005, 2-008, 2-009, 2-010, 2-011, 2-012, 2-013, 2-014, 2-018, 2-019, 2-020, 2-021, 2-022, 2-024, 2-025, 2-027, 2-028, 2-029, 2-030, 2-031, 2-032, 2-033, 2-034, 2-035, 2-036, 2-037, 2-038, 2-039, 2-040, 2-041, 2-042, 2-044, 2-045, 2-047, 2-049, 2-058, 2-062, 2-063, 2-065, 2-067, 2-068, 2-069, 2-071, 2-072, 2-073, 2-076, 2-077, 2-078, 2-079, 2-080, 2-081, 2-082, 2-083, 2-084, 2-085, 2-086, 2-087, 2-088, 2-089, 2-090, 2-091, 2-092, 2-093, 2-094, 2-095, 2-096, 2-097, 2-098, 2-099, 2-100, 2-101, 2-102, 2-103, 2-104, 2-105, 2-107, 2-108, 2-109, 2-110, 2-111, 2-112, 2-113, 2-114, 2-115, 2-116, 2-117, 2-118, 2-119, 2-120, 2-121, 2-122, 2-123, 2-124, 2-125, 2-126, 2-127, 2-128, 2-129, 2-130, 2-131, 2-132, 2-133, 2-134, 2-135, 2-136, 2-137, 2-138, 2-139, 2-140, 2-141, 2-142, 2-144, 2-145, 2-146, 2-147, 2-148, 2-150, 2-151, 2-152, 2-153, 2-154, 2-155, 2-156, 2-157, 2-158, 2-159, 2-160, 2-161, 2-162, 2-163, 2-164, 2-165, 2-166, 2-167, 2-168, 2-169, 2-170, 2-171, 2-172, 2-173, 2-174, 2-175, 2-176, 2-177, 2-178, 2-179, 2-180, 2-181, 2-182, 2-183, 2-184, 2-186, 2-187, 2-188, 2-189, 2-190, 2-191, 2-192, 2-193, 2-194, 2-195, 2-196, 2-197, 2-198, 2-199, 2-200, 2-201, 2-202, 2-203, 2-204, 2-205, 2-206, 2-207, 2-208, 2-209, 2-211, 2-212, 2-213, 2-214, 2-215, 2-216, 2-217, 2-218, 2-219, 2-220, 2-221, 2-222, 2-223, 2-224, 2-225, 2-226, 2-227, 2-228, 2-229, 2-231, 2-232, 2-233, 2-235, 2-238, 2-240, 2-244, 2-245, 2-246, 2-247, 2-248, 2-249, 2-250, 2-251, 2-252, 2-253, 2-254, 2-255, 2-256, 2-257, 2-259, 2-260, 2-261, 2-263, 2-264, 2-267, 2-269, 2-270, 2-271, 2-272, 2-273, 2-274, 2-275, 2-276, 2-277, 2-278, 2-279, 2-280, 2-282, 2-283, 2-290, 2-297, 2-298, 2-299, 2-300, 2-301, 2-302, 2-303, 2-304, 2-305, 3-001, 3-003, 3-005, 3-006, 3-007, 3-009, 3-010, 3-011, 3-012, 3-013, 3-014, 3-016, 3-018, 3-019, 3-020, 3-021, 3-022, 3-023, 3-024, 3-025, 3-026, 3-027, 3-028, 3-029, 3-030, 3-031, 3-032, 3-033, 3-034, 3-035, 3-036, 3-037, 3-039, 3-040, 3-042, 3-043, 3-044, 3-045, 3-047, 3-048, 3-049, 3-050, 3-053, 3-055, 3-056, 3-057, 3-058, 3-059, 3-060, 3-061, 3-062, 3-063, 3-064, 3-065, 3-066, 3-068, 3-069, 3-071, 3-072, 3-073, 3-074, 3-075, 3-076, 3-077, 3-079, 3-080, 3-081, 3-082, 3-083, 3-084, 3-085, 3-086, 3-088, 3-089, 3-090, 3-091, 3-092, 3-093, 3-094, 3-095, 3-096, 3-097, 3-098, 3-100, 3-101, 3-102, 3-103, 3-105, 3-106, 3-107, 3-108, 3-109, 3-110, 3-113, 3-114, 3-115, 3-121, 3-122, 3-123, 3-127, 3-128, 3-129, 3-131, 3-132, 3-133, 3-135, 3-136, 3-138, 3-139, 3-140, 3-141, 3-144, 3-145, 3-146, 3-154, 3-155, 3-156, 3-157, 3-158, 3-159, 3-160, 3-161, 3-164, 3-166, 3-167, 3-168, 3-169, 3-170, 3-171, 3-172, 3-173, 3-174, 3-175, 3-176, 3-177, 3-178, 3-179, 3-180, 3-181, 3-182, 3-183, 3-184, 3-185, 3-186, 3-187, 3-188, 3-189, 3-190, 3-191, 3-192, 3-193, 3-194, 3-195, 3-196, 3-197, 3-199, 3-200, 3-203, 3-205, 3-207, 3-208, 3-209, 3-210, 3-212, 3-213, 3-214, 3-215, 3-216, 3-217, 3-219, 3-220, 3-221, 3-222, 3-223, 3-224, 3-225, 3-226, 3-227, 3-228, 3-232, 3-233, 3-234, 3-235, 3-236, 3-237, 3-238, 3-239, 3-243, 3-246, 3-248, 3-249, 3-254, 3-257, 3-263, 3-264, 3-265, 3-268, 3-269, 3-270, 3-271, 3-272, 3-273, 3-274, 3-275, 3-277, 3-278, 3-280, 3-281, 3-282, 3-283, 3-284, 3-285, 3-286, 3-287, 3-291, 3-292, 3-294, 4-001, 5-001, 5-002, 5-003, 5-004, 5-005, 5-006, 5-007, 5-008, 5-009, 5-010, 5-011, 5-012, 5-013, 5-014, 5-015, 5-016, 6-001, 6-002, 6-004, 6-005

### Test Example 7: Effect test for Trichostrongylus axei

Agar medium with 1% potato dextrose was aliquoted into 60-µl portions in a 96-well plate, and 30 µl of sterilized water containing *Trichostrongylus axei* eggs and a freeze-dried *Escherichia coli* OP50 strain (five eggs, 10 µg/10 µl *Escherichia coli*) was added to each well. From above, 10 µl of test chemical solution B of the presently invented compound was added to each well, and the plate was left to stand under dark conditions at 25°C. The number of unhatched eggs and the number of inactive larvae 4 days after the addition of the chemical were counted, and the efficacy (%) to the untreated plot was calculated from the following expression. Efficacy (%) = [(number of unhatched eggs in treated plot + number of inactive larvae) / (number of unhatched eggs in untreated plot + number of inactive larvae + number of active larvae)] × 100

The result showed that the following compounds among the tested compounds exhibited efficacies (%) of 50% or more.
The presently invented compound: 2-003, 2-035, 2-038, 2-056, 2-070, 2-088, 2-103, 2-115, 2-120, 2-126, 2-128, 2-129, 2-130, 2-131, 2-132, 2-133, 2-136, 2-138, 2-139, 2-142, 2-144, 2-145, 2-147, 2-153, 2-155, 2-156, 2-158, 2-160, 2-161, 2-164, 2-165, 2-166, 2-167, 2-169, 2-170, 2-171, 2-172, 2-173, 2-177, 2-179, 2-180, 2-181, 2-184, 2-188, 2-191, 2-192, 2-193, 2-194, 2-197, 2-201, 2-202, 2-203, 2-204, 2-206, 2-208, 2-211, 2-214, 2-218, 2-219, 2-220, 2-221, 2-225, 2-229, 2-232, 2-234, 2-235, 2-238, 2-239, 2-240, 2-244, 2-245, 2-246, 2-247, 2-248, 2-249, 2-250, 2-251, 2-252, 2-254, 2-255, 2-256, 2-262, 2-263, 2-264, 2-266, 2-270, 2-271, 2-272, 2-273, 2-274, 2-275, 2-277, 2-278, 2-279, 2-289, 2-290, 2-291, 2-292, 2-296, 2-297, 2-299, 2-303, 2-304, 3-011, 3-027, 3-031, 3-060, 3-061, 3-064, 3-065, 3-070, 3-071, 3-074, 3-076, 3-078, 3-082, 3-083, 3-085, 3-086, 3-087, 3-089, 3-097, 3-098, 3-100, 3-102, 3-107, 3-110, 3-114, 3-117, 3-121, 3-124, 3-125, 3-126, 3-127, 3-128, 3-129, 3-136, 3-137, 3-138, 3-139, 3-141, 3-143, 3-144, 3-145, 3-146, 3-148, 3-152, 3-154, 3-155, 3-157, 3-158, 3-161, 3-163, 3-164, 3-166, 3-169, 3-170, 3-171, 3-174, 3-175, 3-180, 3-183, 3-190, 3-192, 3-195, 3-199, 3-202, 3-209, 3-211, 3-216, 3-222, 3-226, 3-232, 3-233, 3-237, 3-239, 3-249, 3-250, 3-261, 3-263, 3-267, 3-268, 3-270, 3-271, 3-274, 3-275, 3-277, 3-279, 3-283, 3-285, 3-293, 5-001, 5-004, 5-005, 5-006, 5-007, 5-009, 5-010, 5-011, 5-012, 5-013, 5-015, 5-016, 6-001

### Industrial Applicability

The aryltetrahydropyridine compound of the present invention or a salt thereof has superior pesticidal activity, and is applicable as a pesticide.

## Claims

1. An aryltetrahydropyridine compound or a salt thereof, the aryltetrahydropyridine compound represented by a formula (1): 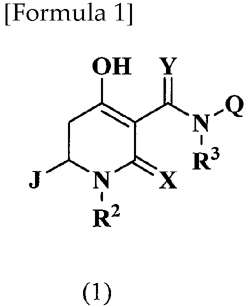 wherein
Q represents Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, Q-37, Q-38, Q-39, Q-40, Q-41, Q-42, Q-43, Q-44, Q-45, Q-46, Q-47, Q-48, Q-49, Q-50, Q-51, Q-52, Q-53, Q-54, Q-55 or Q-56, 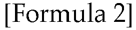 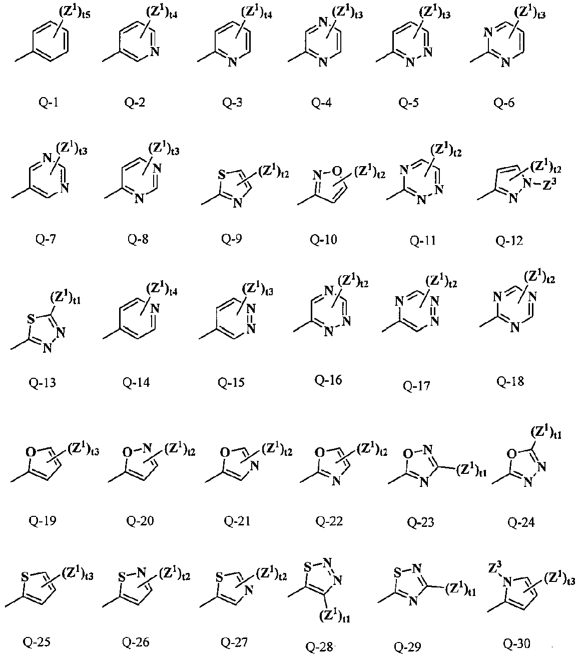 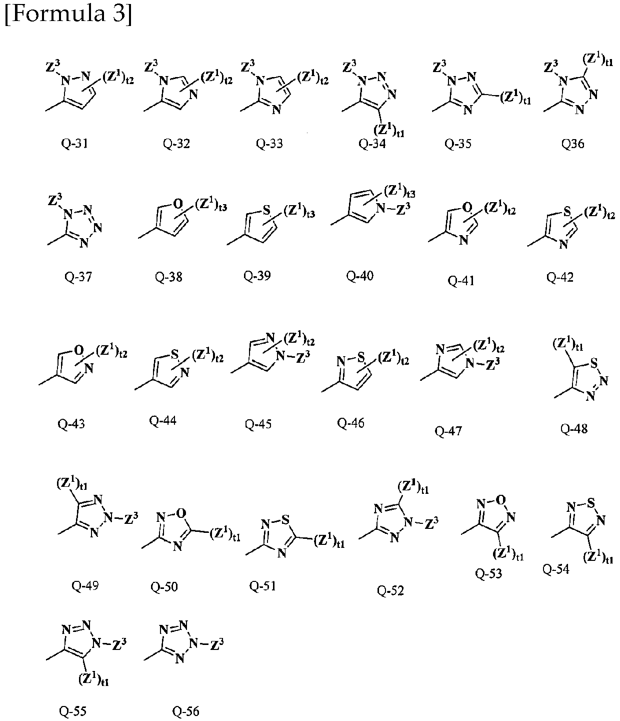
Z¹ represents a halogen atom, C₁-C₆ alkyl, C₂-C₆ alkenyl, C₂-C₆ alkynyl, halo (C₁-C₆) alkyl, halo (C₂-C₆) alkenyl, halo (C₂-C₆) alkynyl, C₁-C₆ alkoxy, halo (C₁-C₆) alkoxy, C₂-C₆ alkoxy (C₁-C₆) alkoxy, C₂-C₆ alkenyloxy, halo (C₂-C₆) alkenyloxy, C₂-C₆ alkynyloxy, halo (C₂-C₆) alkynyloxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, C₂-C₆ alkenylthio, C₂-C₆ alkenylsulfinyl, C₂-C₆ alkenylsulfonyl, halo (C₂-C₆) alkenylthio, halo (C₂-C₆) alkenylsulfinyl, halo (C₂-C₆) alkenylsulfonyl, C₂-C₆ alkynylthio, C₂-C₆ alkynylsulfinyl, C₂-C₆ alkynylsulfonyl, halo (C₂-C₆) alkynylthio, halo (C₂-C₆) alkynylsulfinyl, halo (C₂-C₆) alkynylsulfonyl, -NH₂, C₁-C₆ alkylamino, di (C₁-C₆) alkylamino, halo (C₁-C₆) alkylamino, dihalo (C₁-C₆) alkylamino, formyl, carboxy, C₁-C₆ alkylcarbonyl, halo (C₁-C₆) alkylcarbonyl, C₁-C₆ alkoxycarbonyl, halo (C₁-C₆) alkoxycarbonyl, C₁-C₆ alkylaminocarbonyl, halo (C₁-C₆) alkylaminocarbonyl, di (C₁-C₆) alkylaminocarbonyl, hydroxy, C₁-C₆ alkylcarbonyloxy, C₁-C₆ alkoxycarbonyloxy, C₁-C₆ alkylsulfonyloxy, halo (C₁-C₆) alkylsulfonyloxy, -SO₃H, C₁-C₆ alkylaminosulfonyl, di (C₁-C₆) alkylaminosulfonyl, -C(=NOR¹²)R¹³, cyano or nitro,
R¹² represents a hydrogen atom or C₁-C₆ alkyl,
R¹³ represents a hydrogen atom or C₁-C₆ alkyl,
Z³ represents a hydrogen atom or C₁-C₆ alkyl,
J represents J-1 or J-2, 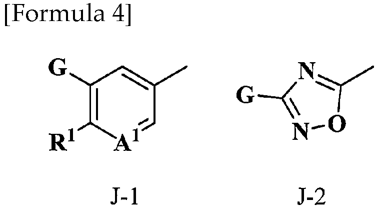
A¹ represents a nitrogen atom or CH,
G represents G-1, G-2, G-3, G-4, G-5, G-6, G-7, G-8, G-9, G-10, G-11, G-12, G-13, G-14, G-15, G-16, G-17, G-18, G-19, G-20, G-21, G-22, G-23, G-24, G-25, G-26, G-27, G-28, G-29, G-30, G-31, G-32, G-33, G-34, G-35, G-36, G-37, G-38, G-39, G-40, G-41, G-42, G-43, G-44, G-45, G-46, G-47, G-48, G-49, G-50, G-51, G-52, G-53, G-54, G-55, G-56, G-57, G-58, G-59, G-60 or G-61, 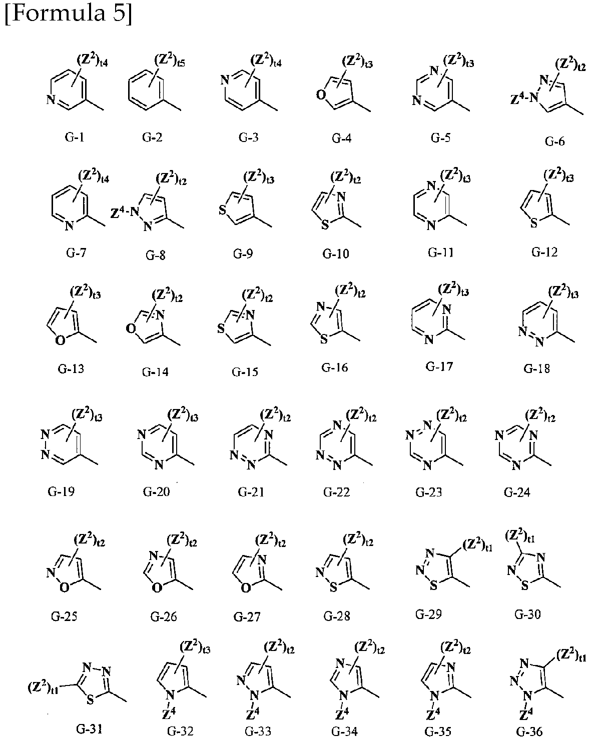 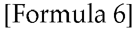 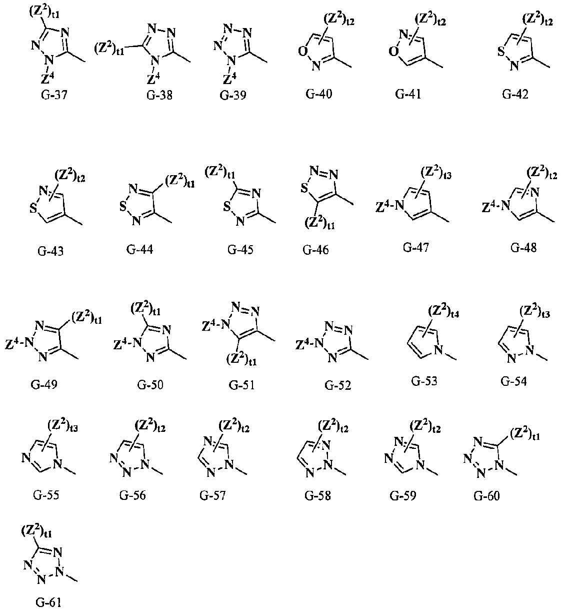
Z² represents a halogen atom, C₁-C₆ alkyl, C₂-C₆ alkenyl, C₂-C₆ alkynyl, halo (C₁-C₆) alkyl, halo (C₂-C₆) alkenyl, halo (C₂-C₆) alkynyl, C₁-C₆ alkoxy, halo (C₁-C₆) alkoxy, C₁-C₆ alkoxy (C₁-C₆) alkoxy, C₂-C₆ alkenyloxy, halo (C₂-C₆) alkenyloxy, C₂-C₆ alkynyloxy, halo (C₂-C₆) alkynyloxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, C₂-C₆ alkenylthio, C₂-C₆ alkenylsulfinyl, C₂-C₆ alkenylsulfonyl, halo (C₂-C₆) alkenylthio, halo (C₂-C₆) alkenylsulfinyl, halo (C₂-C₆) alkenylsulfonyl, C₂-C₆ alkynylthio, C₂-C₆ alkynylsulfinyl, C₂-C₆ alkynylsulfonyl, halo (C₂-C₆) alkynylthio, halo (C₂-C₆) alkynylsulfinyl, halo (C₂-C₆) alkynylsulfonyl, -NH₂, C₁-C₆ alkylamino, di (C₁-C₆) alkylamino, halo (C₁-C₆) alkylamino, dihalo (C₁-C₆) alkylamino, formyl, carboxy, C₁-C₆ alkylcarbonyl, halo (C₁-C₆) alkylcarbonyl, C₁-C₆ alkoxycarbonyl, halo (C₁-C₆) alkoxycarbonyl, C₁-C₆ alkylaminocarbonyl, halo (C₁-C₆) alkylaminocarbonyl, di (C₁-C₆) alkylaminocarbonyl, hydroxy, C₁-C₆ alkylcarbonyloxy, C₁-C₆ alkoxycarbonyloxy, C₁-C₆ alkylsulfonyloxy, halo (C₁-C₆) alkylsulfonyloxy, -SO₃H, C₁-C₆ alkylaminosulfonyl, di (C₁-C₆) alkylaminosulfonyl, -C(=NOR¹⁴)R¹⁵, nitro or cyano,
R¹⁴ represents a hydrogen atom or C₁-C₆ alkyl,
R¹⁵ represents a hydrogen atom or C₁-C₆ alkyl,
Z⁴ represents C₁-C₆ alkyl,
R¹ represents a hydrogen atom, a halogen atom, halo (C₁-C₆) alkyl, C₁-C₆ alkyl or cyano,
R² represents a hydrogen atom, C₁-C₆ alkyl, halo (C₁-C₆) alkyl, C₂-C₆ alkenyl, C₂-C₆ alkynyl, C₃-C₆ cycloalkyl, -C(O)R⁴, -C(O)OR⁵, D-1, D-2 or D-3, 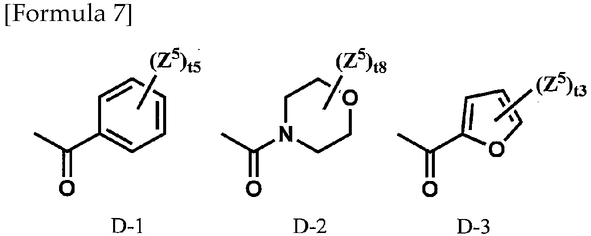
R⁴ represents C₁-C₆ alkyl, C₂-C₆ alkenyl, C₂-C₆ alkynyl, C₃-C₆ cycloalkyl, C₁-C₆ alkylcarbonyl, di (C₁-C₆) alkylamino or (C₁-C₆) alkyl substituted with R⁶,
R⁶ represents a halogen atom, C₁-C₆ alkoxy, cyano, nitro, phenyl or phenyl substituted with R⁸,
Z⁵ represents a halogen atom, C₁-C₆ alkyl, C₁-C₆ alkoxy, cyano or nitro,
R⁸ represents a halogen atom, C₁-C₆ alkyl, C₁-C₆ alkoxy, cyano or nitro,
R⁵ represents C₁-C₆ alkyl, C₂-C₆ alkenyl, C₂-C₆ alkynyl, C₃-C₆ cycloalkyl, (C₁-C₆) alkyl substituted with R⁹, phenyl or phenyl substituted with R¹⁰,
R⁹ represents a halogen atom, C₁-C₆ alkoxy, cyano, nitro, phenyl or phenyl substituted with R¹¹,
R¹⁰ represents a halogen atom, C₁-C₆ alkyl, C₁-C₆ alkoxy, cyano or nitro,
R¹¹ represents a halogen atom, C₁-C₆ alkyl, C₁-C₆ alkoxy, cyano or nitro,
R³ represents a hydrogen atom, C₁-C₆ alkyl, halo (C₁-C₆) alkyl, C₂-C₆ alkenyl, C₂-C₆ alkynyl, C₃-C₆ cycloalkyl or C₁-C₆ alkylcarbonyl,
X represents an oxygen atom or a sulfur atom,
Y represents an oxygen atom or a sulfur atom,
t1 represents an integer of 0 or 1,
t2 represents an integer of 0, 1 or 2,
t3 represents an integer of 0, 1, 2 or 3,
t4 represents an integer of 0, 1, 2, 3 or 4,
t5 represents an integer of 0, 1, 2, 3, 4 or 5 and
t8 represents an integer of 0, 1, 2, 3 or 4.

2. The aryltetrahydropyridine compound according to claim 1 or a salt thereof, wherein
A¹ represents a nitrogen atom.

3. The aryltetrahydropyridine compound according to any one of claims 1 to 2 or a salt thereof, wherein
Z¹ represents a halogen atom, C₁-C₆ alkyl, C₂-C₆ alkenyl, C₂-C₆ alkynyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, -NH₂, C₁-C₆ alkylamino, di (C₁-C₆) alkylamino, carboxy, C₁-C₆ alkylcarbonyl, C₁-C₆ alkoxycarbonyl, C₁-C₆ alkylaminocarbonyl, di (C₁-C₆) alkylaminocarbonyl, hydroxy, C₁-C₆ alkylcarbonyloxy, C₁-C₆ alkylsulfonyloxy, cyano or nitro,
Z² represents a halogen atom, C₁-C₆ alkyl, C₂-C₆ alkenyl, C₂-C₆ alkynyl, halo (C₁-C₆) alkyl, halo (C₂-C₆) alkenyl, halo (C₂-C₆) alkynyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, halo (C₁-C₆) alkylsulfinyl, halo (C₁-C₆) alkylsulfonyl, -NH₂, C₁-C₆ alkylamino, di (C₁-C₆) alkylamino, carboxy, C₁-C₆ alkylcarbonyl, C₁-C₆ alkoxycarbonyl, C₁-C₆ alkylaminocarbonyl, di (C₁-C₆) alkylaminocarbonyl, hydroxy, C₁-C₆ alkylcarbonyloxy, C₁-C₆ alkylsulfonyloxy, -C(=NOR¹⁴)R¹⁵, nitro or cyano,
Z⁵ represents a halogen atom, C₁-C₆ alkyl or C₁-C₆ alkoxy,
R¹ represents a hydrogen atom, a halogen atom or halo (C₁-C₆) alkyl,
R² represents a hydrogen atom, -C(O)R⁴, -C(O)OR⁵, D-1, D-2 or D-3,
R³ represents a hydrogen atom,
Q represents Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-25, Q-26, Q-27, Q-29, Q-30, Q-31, Q-32, Q-33, Q-38, Q-39, Q-40, Q-41, Q-42, Q-43, Q-44, Q-45, Q-46, Q-47, Q-50, Q-51, Q-52 or Q-54, and
G represents G-1, G-2, G-3, G-4, G-5, G-6, G-7, G-8, G-9, G-10, G-11, G-12, G-13, G-14, G-15, G-16, G-17, G-18, G-19, G-20, G-21, G-22, G-23, G-24, G-25, G-26, G-27, G-28, G-29, G-30, G-31, G-32, G-33, G-34, G-35, G-37, G-38, G-40, G-41, G-42, G-43, G-45, G-47, G-48, G-50, G-53, G-54, G-55 or G-57.

4. The aryltetrahydropyridine compound according to any one of claims 1 to 3 or a salt thereof, wherein
J represents J-1, and
A¹ represents a nitrogen atom.

5. The aryltetrahydropyridine compound according to any one of claims 1 to 3 or a salt thereof, wherein
J represents J-2.

6. The aryltetrahydropyridine compound according to any one of claims 1 to 5 or a salt thereof, wherein
G represents G-1, G-3, G-4, G-5, G-7, G-9, G-10, G-11, G-12, G-13, G-14, G-15, G-16, G-17, G-18, G-19, G-20, G-21, G-22, G-23, G-24, G-25, G-26, G-27, G-28, G-29, G-30, G-31, G-32, G-34, G-35, G-37, G-38, G-40, G-41, G-42, G-43, G-45, G-47, G-48, G-50, G-53, G-55 or G-57.

7. The aryltetrahydropyridine compound according to claim 1 or a salt thereof, wherein
Q represents Q-1, Q-2, Q-3, Q-4, Q-5, Q-6, Q-7, Q-8, Q-9, Q-10, Q-11, Q-12, Q-13, Q-14, Q-20, Q-22, Q-29, Q-31, Q-45 or Q-52,
Z¹ represents a halogen atom, C₁-C₆ alkyl, C₂-C₆ alkenyl, C₂-C₆ alkynyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, carboxy, C₁-C₆ alkylcarbonyl, C₁-C₆ alkoxycarbonyl, C₁-C₆ alkylaminocarbonyl, hydroxy, cyano or nitro,
G represents G-1, G-2, G-3, G-4, G-5, G-6, G-7, G-8, G-9, G-10, G-11, G-12, G-13, G-14, G-15, G-16, G-17, G-18, G-19, G-20, G-27, G-28 or G-40,
Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, di (C₁-C₆) alkylamino, C₁-C₆ alkylcarbonyl, C₁-C₆ alkoxycarbonyl, hydroxy, -C(=NOR¹⁴)R¹⁵, nitro or cyano,
R¹⁴ represents C₁-C₆ alkyl,
R¹ represents a hydrogen atom, a halogen atom or halo (C₁-C₆) alkyl,
R² represents a hydrogen atom, -C(O)R⁴, -C(O)OR⁵, D-1, D-2 or D-3,
R⁴ represents C₁-C₆ alkyl, C₃-C₆ cycloalkyl, C₁-C₆ alkylcarbonyl or (C₁-C₆) alkyl substituted with R⁶,
R⁶ represents a halogen atom or C₁-C₆ alkoxy,
Z⁵ represents C₁-C₆ alkyl,
R⁵ represents C₁-C₆ alkyl or C₂-C₆ alkenyl,
R³ represents a hydrogen atom, and
X represents an oxygen atom.

8. The aryltetrahydropyridine compound according to claim 7 or a salt thereof, wherein
G represents G-1, G-3, G-4, G-5, G-7, G-9, G-10, G-11, G-12, G-13, G-14, G-15, G-16, G-17, G-18, G-19, G-20, G-27, G-28 or G-40.

9. The aryltetrahydropyridine compound according to claim 8 or a salt thereof, wherein
Q represents Q-1, Q-2, Q-3, Q-4, Q-5, Q-6, Q-7, Q-8, Q-9, Q-10, Q-12, Q-13, Q-14, Q-20, Q-22, Q-29 or Q-45,
Z¹ represents a halogen atom, C₁-C₆ alkyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, C₁-C₆ alkoxycarbonyl, cyano or nitro,
Z³ represents C₁-C₆ alkyl, and
Z² represents a halogen atom, C₁-C₆ alkyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, hydroxy, nitro or cyano.

10. The aryltetrahydropyridine compound according to claim 9 or a salt thereof, wherein
J represents J-1,
A¹ represents a nitrogen atom,
Q represents Q-1, Q-2, Q-3, Q-4, Q-5, Q-6, Q-7, Q-8, Q-9, Q-12, Q-13, Q-14 or Q-22,
G represents G-1, G-3, G-4 or G-5,
Z² represents a halogen atom, C₁-C₆ alkyl, halo (C₁-C₆) alkyl, C₁-C₆ alkoxy, halo (C₁-C₆) alkoxy or nitro,
R² represents a hydrogen atom or -C(O)OR⁵,
R⁵ represents C₁-C₆ alkyl, and
Y represents an oxygen atom.

11. The aryltetrahydropyridine compound according to claim 10 or a salt thereof, wherein
Q represents Q-1, Q-2, Q-3, Q-4, Q-6, Q-9 or Q-12,
Z¹ represents a halogen atom, C₁-C₆ alkyl, halo (C₁-C₆) alkyl, cyano or nitro,
G represents G-1 or G-3,
Z² represents a halogen atom, C₁-C₆ alkyl or halo (C₁-C₆) alkyl,
R¹ represents a halogen atom or halo (C₁-C₆) alkyl, and
R² represents a hydrogen atom.

12. The aryltetrahydropyridine compound according to claim 9 or a salt thereof, wherein
J represents J-2,
Q represents Q-1, Q-2, Q-3, Q-4, Q-6, Q-9, Q-10, Q-12, Q-20, Q-29 or Q-45,
Z¹ represents a halogen atom, C₁-C₆ alkyl, halo (C₁-C₆) alkyl, C₁-C₆ alkoxy, C₁-C₆ alkoxycarbonyl, cyano or nitro,
G represents G-1, G-3, G-7, G-9, G-10, G-11, G-12, G-13, G-14, G-15, G-16, G-17, G-18, G-19, G-20, G-27, G-28 or G-40, and
Z² represents a halogen atom, C₁-C₆ alkyl, halo (C₁-C₆) alkyl, C₁-C₆ alkoxy, C₁-C₆ alkylthio, C₁-C₆ alkylsulfinyl, C₁-C₆ alkylsulfonyl or hydroxy.

13. The aryltetrahydropyridine compound according to claim 12 or a salt thereof, wherein
Q represents Q-1, Q-2, Q-3, Q-4, Q-6, Q-9 or Q-12,
Z¹ represents a halogen atom, C₁-C₆ alkyl, halo (C₁-C₆) alkyl, cyano or nitro,
G represents G-1, G-3 or G-7,
Z² represents a halogen atom, C₁-C₆ alkyl or halo (C₁-C₆) alkyl, and
R² represents a hydrogen atom.

14. A production intermediate for the aryltetrahydropyridine compound according to any one of claims 1 to 13 or a salt thereof, the production intermediate represented by a formula (1-1): 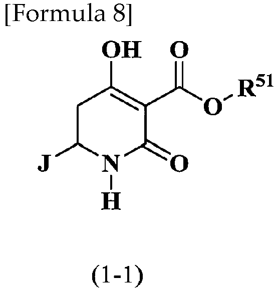 wherein J represents J-1 or J-2, 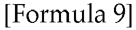 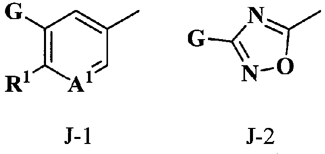
A¹ represents a nitrogen atom,
G represents G-1, G-2, G-3, G-4, G-5, G-6, G-7, G-8, G-9, G-10, G-11, G-12, G-13, G-14, G-15, G-16, G-17, G-18, G-19, G-20, G-27, G-28 or G-40, 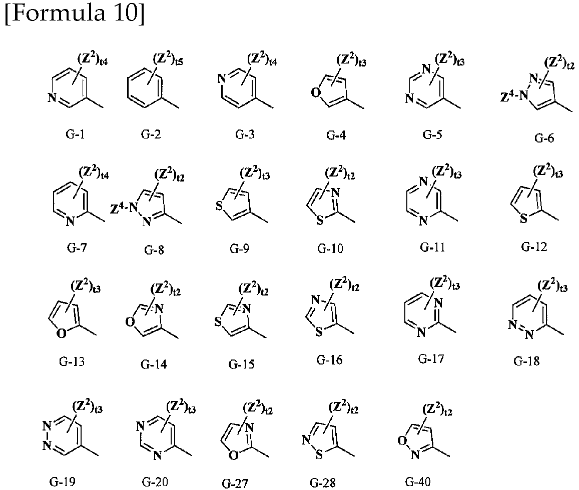
Z² represents a halogen atom, C₁-C₆ alkyl, C₂-C₆ alkenyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, di (C₁-C₆) alkylamino, C₁-C₆ alkylcarbonyl, C₁-C₆ alkoxyarbonyl, hydroxy, -C(=NOR¹⁴)R¹⁵, nitro or cyano,
R¹⁴ represents C₁-C₆ alkyl,
R¹⁵ represents a hydrogen atom or C₁-C₆ alkyl,
Z⁴ represents C₁-C₆ alkyl,
R¹ represents a hydrogen atom, a halogen atom or halo (C₁-C₆) alkyl,
R⁵¹ represents C₁-C₆ alkyl,
t2 represents an integer of 0, 1 or 2,
t3 represents an integer of 0, 1, 2 or 3,
t4 represents an integer of 0, 1, 2, 3 or 4, and
t5 represents an integer of 0, 1, 2, 3, 4 or 5.

15. The production intermediate according to claim 14 for the aryltetrahydropyridine compound or a salt thereof, wherein
J represents J-1,
G represents G-1, G-2 or G-3,
Z² represents a halogen atom or C₁-C₆ alkyl, and
R¹ represents a halogen atom or halo (C₁-C₆) alkyl.

16. A production intermediate for the aryltetrahydropyridine compound according to any one of claims 1 to 13 or a salt thereof, the production intermediate represented by a formula (1-2): 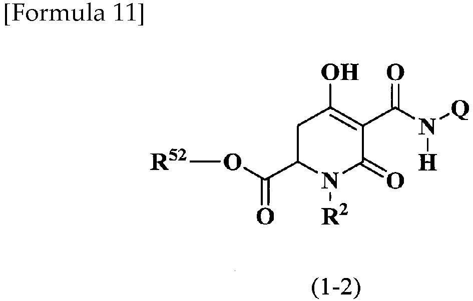 wherein R⁵² represents a hydrogen atom or C₁-C₆ alkyl,
R² represents a hydrogen atom, -C(O)R⁴ or -C(O)OR⁵,
R⁴ represents C₁-C₆ alkyl,
R⁵ represents C₁-C₆ alkyl,
Q represents Q-1, Q-2, Q-3, Q-4, Q-5, Q-6, Q-7, Q-8, Q-9, Q-10, Q-11, Q-12, Q-13, Q-14, Q-20, Q-22, Q-29, Q-31, Q-45 or Q-52, 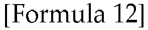 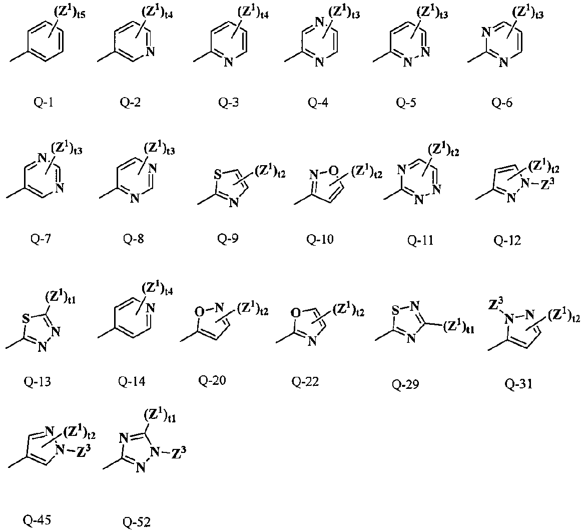
Z¹ represents a halogen atom, C₁-C₆ alkyl, C₂-C₆ alkenyl, C₂-C₆ alkynyl, halo (C₁-C₆) alkyl, C₁-C₆ alkoxy, halo (C₁-C₆) alkoxy, C₁-C₆ alkylthio, C₁-C₆ alkylsulfinyl, C₁-C₆ alkylsulfonyl, halo (C₁-C₆) alkylthio, carboxy, C₁-C₆ alkylcarbonyl, C₁-C₆ alkoxycarbonyl, C₁-C₆ alkylaminocarbonyl, hydroxy, cyano or nitro,
Z³ represents a hydrogen atom or C₁-C₆ alkyl,
t1 represents an integer of 0 or 1,
t2 represents an integer of 0, 1 or 2,
t3 represents an integer of 0, 1, 2 or 3,
t4 represents an integer of 0, 1, 2, 3 or 4, and
t5 represents an integer of 0, 1, 2, 3, 4 or 5.

17. The production intermediate according to claim 16 for the aryltetrahydropyridine compound or a salt thereof, wherein
Q represents Q-1, Q-2, Q-3, Q-4, Q-6 or Q-9,
Z¹ represents a halogen atom, C₁-C₆ alkyl or cyano, and
R² represents a hydrogen atom or -C(O)OR⁵.

18. A pesticide comprising one or two or more selected from the aryltetrahydropyridine compound according to any one of claims 1 to 13 and salts thereof as an active ingredient.

19. An agrochemical comprising one or two or more selected from the aryltetrahydropyridine compound according to any one of claims 1 to 13 and salts thereof as an active ingredient.

20. A control agent for an internal or external parasite in a mammal or a bird, the control agent comprising one or two or more selected from the aryltetrahydropyridine compound according to any one of claims 1 to 13 and salts thereof as an active ingredient.

21. The control agent according to claim 20, wherein the external parasite is a species belonging to Siphonaptera or a species belonging to Acari.

22. An insecticide or acaricide comprising one or two or more selected from the aryltetrahydropyridine compound according to any one of claims 1 to 13 and salts thereof as an active ingredient.

23. An agent for seed treatment, the agent comprising one or two or more selected from the aryltetrahydropyridine compound according to any one of claims 1 to 13 and salts thereof as an active ingredient.

24. The agent for seed treatment according to claim 23, wherein the seed treatment is performed by soaking.

25. An agent for soil treatment, the agent comprising one or two or more selected from the aryltetrahydropyridine compound according to any one of claims 1 to 13 and salts thereof as an active ingredient.

26. The agent for soil treatment according to claim 25, wherein the soil treatment is performed by soil drenching.
